# EUROPÄISCHE PATENTSCHRIFT

(11) **EP 1 307 547 B1**
(45) Veröffentlichungstag und Bekanntmachung des Hinweises auf die Patenterteilung: **26.10.2005**
(21) Anmeldenummer: 01967209.6
(22) Anmeldetag: 19.07.2001
(51) Int. Cl.: C12N 9/28

(54) **NEUES AMYLOLYTISCHES ENZYM AUS BACILLUS SP. A 7-7 (DSM 12368) SOWIE WASCH- UND REINIGUNGSMITTEL MIT DIESEM NEUEN AMYLOLYTISCHEN ENZYM**
NOVEL AMYLOLYTIC ENZYME EXTRACTED FROM BACILLUS SP. A 7-7 (DSM 12368) AND WASHING AND CLEANING AGENTS CONTAINING THIS NOVEL AMYLOLYTIC ENZYME
NOUVELLE ENZYME AMYLOLYTIQUE ISSUE DE BACILLUS SP. A 7-7 (DSM 12368) ET LESSIVE ET AGENT DE NETTOYAGE CONTENANT CETTE ENZYME AMYLOLYTIQUE

(30) Priorität: 28.07.2000 DE 10036752; 28.07.2000 DE 10036753
(43) Veröffentlichungstag der Anmeldung: 07.05.2003
(73) Patentinhaber: Henkel Kommanditgesellschaft auf Aktien, 40589 Düsseldorf (DE)
(72) Erfinder: KOTTWITZ, Beatrix, 40593 Düsseldorf (DE); BREVES, Roland, 40882 Ratingen (DE); MAURER, Karl-Heinz, 40699 Erkrath (DE); POLANYI, Laura, 50859 Köln (DE); HELLEBRANDT, Angela, 50735 Köln (DE); SCHMIDT, Irmgard, 42697 Solingen (DE); STEHR, Regina, 40597 Düsseldorf (DE); WEBER, Angrit, 51467 Bergisch-Gladbach (DE)
(86) Internationale Anmeldenummer: PCT/EP2001/008359
(87) Internationale Veröffentlichungsnummer: WO 2002/010356

(56) Entgegenhaltungen:
- WO-A-00/22103
- WO-A-00/60060
- WO-A-01/66712
- WO-A-01/88107
- WO-A-01/96537

## Beschreibung

Die vorliegende Anmeldung betrifft ein neues amylolytisches Enzym aus dem Mikroorganismus *Bacillus sp.* A 7-7 (DSM 12368) und hinreichend ähnliche Proteine mit amylolytischer Funktion, Verfahren zu deren Herstellung sowie diverse Einsatzmöglichkeiten für diese Proteine. Über die ausgeführten Einsatzmöglichkeiten hinaus können sie für andere, vor allem technische Zwecke weiterentwickelt werden. Die vorliegende Anmeldung betrifft insbesondere Wasch- und Reinigungsmittel mit derartigen amylolytischen Proteinen, Verfahren zur Reinigung von Textilien oder harten Oberflächen unter Beteiligung solcher amylolytischen Proteine oder entsprechender Mittel, sowie deren Verwendung zur Reinigung von Textilien oder harten Oberflächen.

α-Amylasen (E.C. 3.2.1.1) hydrolysieren im Polymerinneren gelegene α-1,4-glycosidische Bindungen von Stärke und stärkeähnlichen Polymeren, wie beispielsweise Amylose, Amylopektin oder Glykogen, unter Bildung von Dextrinen und β-1,6-verzweigten Oligosacchariden. Sie gehören zu den wichtigsten industriell genutzten Enzymen überhaupt. Dies aus zwei Gründen: Zum einen werden sie zumeist wie viele substratabbauende Enzyme von Mikroorganismen in das umgebende Medium abgegeben, so daß sie durch Fermentation und Aufreinigung aus dem Kulturmedium mit vergleichsweise geringem Aufwand in industriellem Maßstab gewonnen werden können. Zum anderen werden Amylasen für ein breites Anwendungsspektrum benötigt.

Eine wichtige technische Verwendung von α-Amylase ist die Herstellung von Glucosesirup. Andere Verwendungen sind beispielsweise die als aktive Komponenten in Wasch- und Reinigungsmitteln, zur Behandlung von Rohmaterialien in der Textilherstellung, zur Herstellung von Klebstoffen oder zur Herstellung von zuckerhaltigen Lebensmitteln oder Lebensmittelbestandteilen.

Enzyme wie Proteasen, Amylasen, Lipasen oder Cellulasen werden seit Jahrzehnten als aktive Komponenten in Wasch- und Reinigungsmitteln eingesetzt. Ihr jeweiliger Beitrag zur Wasch-, beziehungsweise Reinigungsleistung des betreffenden Mittels ist im Fall von Protease die Fähigkeit zum Abbau proteinhaltiger Verunreinigungen, im Fall von Amylase der Abbau von stärkehaltigen Verunreinigungen und im Fall von Lipase die fettspaltende Aktivität. Cellulasen werden insbesondere wegen ihres Beitrags zur Sekundärwaschleistung eines Waschmittels und wegen ihrer Faserwirkung auf Textilien bevorzugt in Waschmitteln eingesetzt. Die jeweiligen Hydrolyseprodukte werden von den übrigen Wasch- oder Reinigungsmittel-Bestandteilen angegriffen, gelöst, emulgiert oder suspendiert oder aufgrund ihrer höheren Löslichkeit mit der Waschflotte ausgeschwemmt, so daß es zu Synergieeffekten zwischen den Enzymen und den übrigen Bestandteilen kommt.

Eine in Wasch- und Reinigungsmitteln häufig eingesetzte α-Amylase ist die aus *Bacillus licheniformis*. Das entsprechende Produkt der Fa. Novo Nordisk A/S, Bagsværd, Dänemark, beispielsweise, trägt den Handelsnamen Termamyl®; von der Fa. Genencor Int., Rochester, New York, USA, heißt es Purastar®. Das aus *B. subtilis*, beziehungsweise *B. amyloliquefaciens* gewonnene und in der US-Anmeldung US 1 227 374 offenbarte Homolog wird von Novo Nordisk A/S unter dem Namen BAN® vertrieben.

Dieses Amylase-Molekül, beziehungsweise dessen nahen Verwandte, sind in zahlreichen Erfindungen weiterentwickelt worden, denen die Aufgabe zugrunde gelegen hat, mithilfe diverser molekularbiologischer Modifikationen ihre enzymatischen Eigenschaften auf spezifische Anwendungen hin zu optimieren. Solche Optimierungen können beispielsweise die Substratspezifitäten, die Stabilität des Enzyms unter verschiedenen Reaktionsbedingungen oder die enzymatische Aktivität selbst betreffen. Beispielhaft für solche Optimierungen hinsichtlich spezifischer Anwendungen seien folgende Anmeldungen genannt: EP 0410498 für das Schlichten von Textilien und WO 96/02633 zur Stärkeverflüssigung.

α-Amylasen werden vor allem aber auch hinsichtlich ihrer Verwendung in Wasch- und Reinigungsmitteln weiterentwickelt. Als Beispiele dafür seien nur folgende Anmeldungen genannt: Die Amylasen der Anmeldung WO 99/02702 sind bei höheren Temperaturen stabiler als das Ausgangsmolekül. Die Enzyme der Anmeldung WO 99/23211 sind bei hohen pH-Werten, in Gegenwart von Calcium-lonen und bei höheren Temperaturen stabil. Die α-Amylasen der Anmeldung WO 97/43424 zeigen ein geändertes Calciumionen-Bindungsverhalten und damit geänderte enzymatische Eigenschaften. Das Mutagenese-Verfahren der Anmeldung WO 99/20768 führt zu α-Amylase-Varianten, die in Gegenwart von Reinigungsmittelbestandteilen besonders stabil sind. Praktisch immer wirkt sich bei derartigen Modifikationen eine Änderung einzelner enzymatischer Eigenschaften auch auf andere Eigenschaften und auf die Waschleistung des betreffenden Enzyms aus. Ein Beispiel für ein auf solche Weise erhaltenes, inzwischen kommerziell vermarktetes Optimierungsprodukt ist Duramyl® (WO 94/02597) mit verringerter Oxidationsempfindlichkeit (Fa. Novo Nordisk A/S, Bagsværd, Dänemark; *SÖFW-Journal* 123, (1997), S. 723-731).

Da Entwicklungen, die lediglich in Optimierungen von nur wenigen bekannten Ausgangsenzymen bestehen, möglicherweise in den erzielbaren Ergebnissen beschränkt sind, findet parallel dazu eine intensive Suche nach vergleichbaren Enzymen aus anderen natürlichen Quellen statt. Identifiziert wurden stärkespaltende Enzyme beispielsweise aus *Pimelobacter, Pseudomonas* und *Thermus* für die Lebensmittelherstellung, Kosmetik und Pharmaka (EP 0 636693), ebensolche aus *Rhizobium, Arthrobacter, Brevibacterium* und *Micrococcus* (EP 0 628 630), aus *Pyrococcus* (WO 94/19454) und *Sulfolobus* zur Stärkeverflüssigung bei hohen Temperaturen, beziehungsweise stark sauren Reaktionsbedingungen (EP 0 727 485 und WO 96/02633). Für den Einsatz bei alkalischen pH-Werten sind Amylasen aus *Bacillus sp.* (WO 95/26397 und WO 97/00324) gefunden worden. Wegen ihrer geringen Empfindlichkeit gegenüber Detergenzien eignen sich andere Amylasen aus verschiedenen *Bacilli* (EP 0 670 367) zur Verwendung in Wasch- oder Reinigungsmitteln.

Enzyme aus neu erschlossenen Organismen eignen sich aufgrund ihrer Herkunft möglicherweise besser als die wenigen etablierten Enzyme, um auf spezifische Anwendungen hin weiterentwickelt zu werden. Ein Beispiel dafür ist die Amylase aus *Thermoalcalibacter* (WO 98/13481), deren natürliche Aktivität weitgehend unempfindlich gegenüber Calcium-Ionen ist, so daß sie von vornherein gute Voraussetzungen für die Verwendung in Waschmitteln mitbringt.

Weitere Optimierungen der aus natürlichen Quellen isolierten Enzyme für das jeweilige Anwendungsgebiet können beispielsweise über molekularbiologische Methoden (etwa gemäß US 5171673 oder WO 99/20768) oder über chemische Modifikationen vorgenommen werden (DE 4013142). In der Patentanmeldung WO 99/43793, beispielsweise, wird eine Weiterentwicklung der bekannten Novamyl®-α-Amylase beschrieben. Darin werden Sequenzähnlichkeiten zwischen Novamyl® und bekannten Cyclodextringlucanotransferasen (CGTasen) ausgenutzt, um mithilfe molekularbiologischer Techniken eine Schar verwandter Moleküle zu konstruieren. Bei diesen handelt es sich um α-Amylasen mit zusätzlichen CGTase-spezifischen Consensus-Sequenzen (Boxen) und Funktionen oder, umgekehrt, um CGTasen mit zusätzlichen für α-Amylasen typischen Bereichen und Funktionen oder um Chimären beider Moleküle. Der Sinn dieser Entwicklung besteht darin, Novamyl® für diese Anwendungen zu optimieren.

In der Anmeldung WO 99/57250 wird eine weitere Methode offenbart, wie Waschmittelenzyme, beispielsweise Lipasen, Cellulasen, Proteasen, Amylasen oder auch CGTasen, in ihrer Waschleistung gesteigert werden können. Das dort beschriebene Prinzip besteht darin, daß die betreffenden Enzyme über einen nicht-Aminosäure-Linker an Cellulose-Bindungsdomänen (CBD) bakteriellen Ursprungs kovalent gebunden werden. Diese sorgen dafür, daß das Enzym verstärkt auf der Oberfläche des Textils wirksam wird. Mit der Schrift WO 99/57252 werden in dieses Konzept andere mögliche Linker miteinbezogen, und mit der Schrift WO 99/57254 andere Enzyme, wie beispielsweise Glykosyl-Transferasen oder Acyl-Transferasen, die entweder unter Bildung eines chimären Proteins oder über die in WO 99/57252 erwähnten Linker an die CBD gebunden werden.

Jede für Wasch- und Reinigungsmittel verwendete Amylase besitzt ein individuelles Leistungsprofil, was sich darin zeigt, daß manche Anschmutzungen besser von dem einen und andere besser von dem anderen Enzym beseitigt werden. Dies belegt zusätzlich die Notwendigkeit dafür, den Stand der Technik um weitere amylolytische Enzyme zu bereichem, die ebenfalls individuelle Wirkungsspektren aufweisen. Diese Notwendigkeit ergibt sich auch aus den sich ändernden Gewohnheiten und Ansprüchen der Verbraucher, nach denen, beispielsweise, ein zunehmender Bedarf nach Waschmitteln für die Reinigung bei niedrigen und mittleren Temperaturen besteht.

Darüberhinaus können neue Enzyme, wie sie aus bislang für diesen Zweck noch nicht erschlossenen Organismen erhalten werden können, im Sinne eines "Protein Engineering" als Ausgangspunkt für weitere gentechnische Modifizierungen dienen. Deren Ziel ist die Herbeiführung von Eigenschaften, die die bisher bekannten Enzyme oder die von diesen abgeleiteten Waschmittelenzyme nicht aufweisen oder nicht aufweisen können.

Andererseits erscheinen aber gerade solche natürlichen Enzyme als besonders geeignete Kandidaten für derartige Optimierungen, die von vornherein im Zusammenspiel mit üblichen Wasch- oder Reinigungsmittelbestandteilen eine gewisse Wasch-, beziehungsweise Reinigungsleistung aufweisen.

Trotz all dieser Entwicklungen besteht aber unverändert die Aufgabe, neben den wenigen natürlichen amylolytischen Enzymen, die unverändert oder in Gestalt von Weiterentwicklungen tatsächlich industriell genutzt werden, weitere aufzufinden, die *a priori* ein breites Anwendungsspektrum aufweisen und als Ausgangspunkt für spezifische Weiterentwicklungen dienen können.

Der vorliegenden Erfindung hat somit die Aufgabe zugrunde gelegen, eine natürliche, bislang noch nicht beschriebene α-Amylase zu identifizieren, welche selbst für technische Einsatzmöglichkeiten, insbesondere in Wasch- oder Reinigungsmitteln als geeignet erscheint oder als Grundlage für anwendungsspezifische Weiterentwicklungen dienen kann.

Eine Teilaufgabe bestand darin, die für eine derartige α-Amylase codierende Nukleinsäure zu erhalten, da diese sowohl für die biotechnologische Herstellung als auch für die Weiterentwicklung dieser Enzyme unerläßlich ist.

Eine weitere Teilaufgabe bestand darin, einen Organismus aufzufinden, welcher die betreffende α-Amylase natürlicherweise produziert.

Eine weitere Teilaufgabe bestand darin, die biotechnologische Produktion der gefundenen α-Amylase zu ermöglichen.

Eine weitere Teilaufgabe bestand darin, Wasch- oder Reinigungsmittel bereitzustellen, deren Wasch-, beziehungsweise Reinigungsleistung durch die gefundene α-Amylase verbessert wird, das heißt deren Wasch-, beziehungsweise Reinigungsleistung wenigstens zum Teil auf das erfindungsgemäße amylolytische Protein zurückgeführt werden kann.

Weitere Teilaspekte liegen in der Bereitstellung entsprechender Wasch- oder Reinigungsverfahren und dem Aufzeigen entsprechender Verwendungsmöglichkeiten.

Eine weitere Teilaufgabe bestand darin, weitere technische Einsatzmöglichkeiten für eine α-Amylase zu definieren, die in erster Linie für den Einsatz in Wasch- und Reinigungsmitteln als geeignet erscheint.

Die Lösung der Aufgabe und damit den ersten Erfindungsgegenstand stellen amylolytische Proteine dar, deren Aminosäuresequenz zu der in SEQ ID NO.2 angegebenen Aminosäuresequenz mindestens zu 96 %, bevorzugt mindestens zu 98 %, besonders bevorzugt zu 100 % identisch ist, insbesondere über den Teilbereich, der den Aminosäuren 32 bis 516 gemäß der SEQ ID NO. 2 entspricht.

Hierzu gehören solche amylolytischen Proteine, die sich von einer Nukleotidsequenz ableiten, die zu der in SEQ ID NO. 1 angegebenen Nukleotidsequenz mindestens zu 85 %, bevorzugt mindestens zu 90 % und besonders bevorzugt zu 100 % identisch ist, insbesondere über den Teilbereich, der den Aminosäuren 32 bis 516 gemäß der SEQ ID NO. 2 entspricht. Hierzu gehören auch solche proteolytischen Enzyme, die zu diesen hinreichende Ähnlichkeit aufweisen oder mit an sich bekannten Methoden abgeleitet werden können. Bevorzugte Vertreter lassen sich natürlicherweise aus Mikroorganismen, besonders gram-positiven Bakterien der Gattung Bacillus, insbesondere der Spezies *Bacillus sp. A 7-7,* und hierunter insbesondere um *Bacillus sp. A 7-7* (DSM 12368) isolieren.

Den zweiten Erfindungsgegenstand bilden für amylolytische Proteine codierende Nukleinsäuren, deren Nukleotidsequenz zu der in SEQ ID NO. 1 angegebenen Nukleotidsequenz mindestens zu 85 %, bevorzugt mindestens zu 90 % und besonders bevorzugt zu 100 % identisch ist, insbesondere über den Teilbereich, der den Aminosäuren 32 bis 516 gemäß der SEQ ID NO.2 entspricht. Hierzu gehören entsprechend bevorzugt die Nukleinsäuren, die für die jeweiligen Proteine des ersten Erfindungsgegenstands codieren.

Den dritten Erfindungsgegenstand bilden die natürlichen Organismen, die ein Protein oder Derivat des ersten Erfindungsgegenstands bilden oder für diese codierende Nukleinsäuren enthalten. Eine besonders bevorzugte Ausführungsform hiervon ist der Stamm *Bacillus sp.* A 7-7, der unter der Bezeichnung DSM (12368) hinterlegt worden ist.

Den vierten Erfindungsgegenstand bilden Vektoren mit den Nukleinsäuren des zweiten Erfindungsgegenstands, mit derartigen Vektoren transformierte Wirtszellen und alle biotechnologischen Verfahren zur Herstellung eines Proteins oder Derivats nach dem ersten Erfindungsgegenstand.

Den fünften Erfindungsgegenstand bilden Wasch- oder Reinigungsmittel, die dadurch gekennzeichnet sind, daß sie ein Protein oder Derivat nach dem ersten Erfindungsgegenstand enthalten. Hierzu gehören vorzugsweise solche, die das amylolytische Protein oder Derivat in Anteilen von 0,000001 Gewichts-Prozent bis 5 Gew.-%, insbesondere 0,00001 bis 3 Gew.-% enthalten, die weitere Enzyme enthalten, die in an sich bekannten Darreichungsformen vorliegen oder in denen die amylolytische Aktivität eine Funktion für die Freisetzung der Inhaltsstoffe des Mittels erfüllt oder selbst kontrolliert wird.

Den sechsten Erfindungsgegenstand bilden Verfahren zur Reinigung von Textilien oder von harten Oberflächen, die dadurch gekennzeichnet, daß in wenigstens einem der Verfahrensschritte ein amylolytisches Protein oder Derivat den ersten Erfindungsgegenstands aktiv wird. Vorzugsweise werden hierfür Mittel nach dem fünften Erfindungsgegenstand eingesetzt; und vorzugsweise kommt das amylolytische Protein oder Derivat in dem betreffenden Verfahrensschritt in einer Menge von 0,01 mg bis 200 mg pro Anwendung, vorzugsweise von 0,02 mg bis 100 mg pro Anwendung zum Einsatz.

Den siebten Erfindungsgegenstand bilden entsprechende Verwendungsmöglichkeiten der Proteine oder Derivate des ersten Erfindungsgegenstands oder der Mittel des fünften Erfindungsgegenstands zur Reinigung von Textilien oder von harten Oberflächen oder zur Freisetzung der Inhaltsstoffe entsprechender Mittel; vorzugsweise pro Anwendung in einer Geschirrspülmaschine oder einer Waschmaschine, in einer Menge von 0,01 mg bis 200 mg, bevorzugt 0,02 mg bis 100 mg des amylolytischen Proteins oder Derivats.

Den achten Erfindungsgegenstand bilden weitere technische Einsatzmöglichkeiten für die gefundenen α-Amylasen. Hierzu gehören Verfahren zur Stärkeverflüssigung, insbesondere zur Ethanolproduktion, temporäre Klebeverfahren und diverse Verwendungsmöglichkeiten, insbesondere zur Behandlung von Rohmaterialien oder Zwischenprodukten in der Textilherstellung, insbesondere zum Entschlichten von Baumwolle, zur Herstellung von linearen und/oder kurzkettigen Oligosacchariden, zur Hydrolyse von Cyclodextrinen, zur Freisetzung von niedermolekularen Verbindungen aus Polysaccharidträgern oder Cyclodextrinen, zur Herstellung von Lebensmitteln und/oder Lebensmittelbestandteilen, zur Herstellung von Tierfutter und/oder Tierfutterbestandteilen und zur Auflösung stärkehaltiger Klebeverbindungen.

Unter einem Protein ist im Sinne der vorliegenden Anmeldung ein aus den natürlichen Aminosäuren zusammengesetztes, weitgehend linear aufgebautes, zur Ausübung seiner Funktion zumeist dreidimensionale Struktur annehmendes Polymer zu verstehen. In der vorliegenden Anmeldung werden die 19 proteinogenen, natürlich vorkommenden L-Aminosäuren mit den international gebräuchlichen 1- und 3-Buchstaben-Codes bezeichnet.

Unter einem Enzym ist im Sinne der vorliegenden Anmeldung ein Protein zu verstehen, das eine bestimmte biochemische Funktion ausübt. Unter amylolytischen Proteinen oder Enzymen mit amylolytischer Funktion sind solche zu verstehen, die α-1,4-glykosidische Bindungen von Polysacchariden hydrolysieren, insbesondere solche, die im Inneren der Polysaccharide liegen. Sie werden deshalb auch als α-1,4-Amylasen (E.C. 3.2.1.1) bezeichnet.

Zahlreiche Proteine werden als sogenannte Präproteine, also zusammen mit einem Signalpeptid gebildet. Darunter ist dann der N-terminale Teil des Proteins zu verstehen, dessen Funktion zumeist darin besteht, die Ausschleusung des gebildeten Proteins aus der produzierenden Zelle in das Periplasma oder das umgebende Medium und/oder dessen korrekte Faltung zu gewährleisten. Anschließend wird das Signalpeptid unter natürlichen Bedigungen durch eine Signalpeptidase vom übrigen Protein abgespalten, so daß dieses seine eigentliche katalytische Aktivität ohne den zunächst vorhandenen N-Terminus ausübt. Die native α-Amylase aus *Bacillus sp*. A 7-7 (DSM 12368), beispielsweise, ist, wie in SEQ ID NO. 2 gezeigt ist, 516 Aminosäuren lang. Wie in SEQ ID NO. 1 dargestellt ist, umfaßt das Signalpeptid dieses Enzyms 31 Aminosäuren, so daß sich für das mature Enzym eine Länge von 485 Aminosäuren ergibt.

Für technische Anwendungen sind aufgrund ihrer enzymatischen Aktivität die maturen Peptide, das heißt die nach ihrer Herstellung prozessierten Enzyme gegenüber den Präproteinen bevorzugt.

Pro-Proteine sind inaktive Vorstufen von Proteinen. Deren Vorläufer mit Signalsequenz werden als Prä-Pro-Proteine bezeichnet.

Unter Nukleinsäuren sind im Sinne der vorliegenden Anmeldung die natürlicherweise aus Nukleotiden aufgebauten als Informationsträger dienenden Moleküle zu verstehen, die für die lineare Aminosäureabfolge in Proteinen oder Enzymen codieren. Sie können als Einzelstrang, als ein zu diesem Einzelstrang komplementärer Einzelstrang oder als Doppelstrang vorliegen. Als der natürlicherweise dauerhaftere Informationsträger ist die Nukleinsäure DNA für molekularbiologische Arbeiten bevorzugt. Demgegenüber wird für die Realisierung der Erfindung in natürlicher Umgebung, wie beispielsweise in einer exprimierenden Zelle, eine RNA gebildet, weshalb erfindungswesentliche RNA-Moleküle ebenfalls Ausführungsformen der vorliegenden Erfindung darstellen.

Bei DNA sind die Sequenzen beider komplementärer Stränge in jeweils allen drei möglichen Leserastem zu berücksichtigen. Ferner ist zu berücksichtigen, daß verschiedene Codon-Triplets für dieselben Aminosäuren codieren können, so daß eine bestimmte Aminosäure-Abfolge von mehreren unterschiedlichen und möglicherweise nur geringe Identität aufweisenden Nukleotidsequenzen abgeleitet werden kann (Degeneriertheit des genetischen Codes). Außerdem weisen verschiedene Organismen Unterschiede im Gebrauch dieser Codons auf. Aus diesen Gründen müssen sowohl - Aminosäuresequenzen als auch Nukleobdsequenzen in die Betrachtung des Schutzbereichs einbezogen werden und angegebene Nukleotidsequenzen sind jeweils nur als eine beispielhafte Codierung für eine bestimmte Aminosäurefolge anzusehen.

Die einem Protein entsprechende Informationseinheit wird auch im Sinne der vorliegenden Anmeldung als Gen bezeichnet.

Einem Fachmann ist es über heutzutage allgemein bekannte Methoden, wie beispielsweise die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen die entsprechenden Nukleinsäuren bis hin zu vollständigen Genen herzustellen. Derartige Methoden sind beispielsweise aus dem "Lexikon der Biochemie", Spektrum Akademischer Verlag, Berlin, 1999, Band 1, S. 267-271 und Band 2, S. 227-229, bekannt.

Änderungen der Nukleotidsequenz, wie sie beispielsweise durch an sich bekannte molekularbiologische Methoden herbeigeführt werden können, werden als Mutationen bezeichnet. Je nach Art der Änderung kennt man beispielsweise Deletions-, Insertions- oder Substitutionsmutationen oder solche, bei denen verschiedene Gene oder Teile von Genen miteinander fusioniert (shuffling) werden; dies sind Genmutationen. Die zugehörigen Organismen werden als Mutanten bezeichnet. Die von mutierten Nukleinsäuren abgeleiteten Proteine werden als Varianten bezeichnet. So führen beispielsweise Deletions-, Insertions- Substitutionsmutationen oder Fusionen zu deletions-, insertions- substitutionsmutierten oder Fusionsgenen und auf Proteinebene zu entsprechenden Deletions-, Insertions- oder Substitutionsvarianten, beziehungsweise Fusionsproteinen.

Unter Fragmenten werden alle Proteine oder Peptide verstanden, die kleiner sind als natürliche Proteine oder solche, die vollständig translatierten Genen entsprechen, und beispielsweise auch synthetisch erhalten werden können. Aufgrund ihrer Aminosäuresequenzen können sie den betreffenden vollständigen Proteinen zugeordnet werden. Sie können beispielsweise gleiche Strukturen annehmen oder proteolytische Aktivitäten oder Teilaktivitäten ausüben, wie beispielsweise die Komplexierung eines Substrats. Fragmente und Deletionsvarianten von Ausgangsproteinen sind prinzipiell gleichartig; während Fragmente eher kleinere Bruchstücke darstellen, fehlen den Deletionsmutanten eher nur kurze Bereiche, und damit nur einzelne Teilfunktionen.

Unter chimären oder hybriden Proteinen sind im Sinne der vorliegenden Anmeldung solche Proteine zu verstehen, die aus Elementen zusammengesetzt sind, die natürlicherweise von verschiedenen Polypeptidketten aus demselben Organismus oder aus verschiedenen Organismen stammen. Dieses Vorgehen wird auch Shuffling oder Fusionsmutagenese genannt. Der Sinn einer solchen Fusion kann beispielsweise darin bestehen, mithilfe des heranfusionierten Proteinteils eine bestimmte enzymatische Funktion herbeizuführen oder zu modifizieren.

Unter durch Insertionsmutation erhaltene Proteinen sind solche Varianten zu verstehen, die über an sich bekannte Methoden durch Einfügen eines Nukleinsäure-, beziehungsweise Proteinfragments in die Ausgangssequenzen erhalten worden sind. Sie sind ihrer prinzipiellen Gleichartigkeit wegen den chimären Proteinen zuzuordnen. Sie unterscheiden sich von jenen lediglich im Größenverhältnis des unveränderten Proteinteils zur Größe des gesamten Proteins. In solchen insertionsmutierten Proteinen ist der Anteil an Fremdprotein geringer als in chimären Proteinen.

Inversionsmutagenese, also eine partielle Sequenzumkehrung, kann als Sonderform sowohl der Deletion, als auch der Insertion angesehen werden. Dasselbe gilt für eine von der ursprünglichen Aminosäureabfolge abweichende Neugruppierung verschiedener Molekülteile. Sie kann sowohl als Deletionsvariante, als Insertionsvariante, als auch als Shuffling-Variante des ursprünglichen Proteins angesehen werden.

Unter Derivaten werden im Sinne der vorliegenden Anmeldung solche Proteine verstanden, deren reine Aminosäurekette chemisch modifiziert worden ist. Solche Derivatisierungen können beispielsweise biologisch im Zusammenhang mit der Proteinbiosynthese durch den Wirtsorganismus erfolgen. Hierfür können molekularbiologische Methoden eingesetzt werden. Sie können aber auch chemisch durchgeführt werden, etwa durch die chemische Umwandlung einer Seitenkette einer Aminosäure oder durch kovalente Bindung einer anderen Verbindung an das Protein. Bei solch einer Verbindung kann es sich beispielsweise um andere Proteine handeln, die beispielsweise über bifunktionelle chemische Verbindungen an erfindungsgemäße Proteine gebunden werden. Ebenso ist unter Derivatisierung die kovalente Bindung an einen makromolekularen Träger zu verstehen.

Im Sinne der vorliegenden Erfindung werden alle Enzyme, Proteine, Fragmente und Derivate, sofern sie nicht explizit als solche angesprochen zu werden brauchen, unter dem Oberbegriff Proteine zusammengefaßt.

Unter Vektoren werden im Sinne der vorliegenden Erfindung aus Nukleinsäuren bestehende Elemente verstanden, die als kennzeichnenden Nukleinsäurebereich ein interessierendes Gen enthalten. Sie vermögen dieses in einer Spezies oder einer Zellinie über mehrere Generationen oder Zellteilungen hinweg als vom übrigen Genom unabhängig replizierendes, stabiles genetisches Element zu etablieren. Vektoren sind insbesondere bei der Verwendung in Bakterien spezielle Plasmide, also zirkulare genetische Elemente. Man unterscheidet in der Gentechnik einerseits zwischen solchen Vektoren, die der Lagerung und somit gewissermaßen auch der gentechnischen Arbeit dienen, den sogenannten Klonierungsvektoren, und andererseits denen, die die Funktion erfülllen, das interessierende Gen in der Wirtszelle zu realisieren, das heißt, die Expression des betreffenden Proteins zu ermöglichen. Diese Vektoren werden als Expressionsvektoren bezeichnet.

Durch Vergleich mit bekannten Enzymen, die beispielsweise in allgemein zugänglichen Datenbanken hinterlegt sind, lassen sich aus der Aminosäure- oder Nukleotid-Sequenz charakteristische Molekülteile wie beispielsweise Strukturelemente oder die enzymatische Aktivität eines betrachteten Enzyms folgern. Solch ein Vergleich geschieht dadurch, daß ähnliche Abfolgen in den Nukleotid- oder Aminosäuresequenzen der betrachteten Proteine einander zugeordnet werden. Dies nennt man Homologisierung. Eine tabellarische Zuordnung der betreffenden Positionen wird als Alignment bezeichnet. Bei der Analyse von Nukleotidsequenzen sind wiederum beide komplementären Stränge und jeweils allen drei möglichen Leserastern zu berücksichtigen; ebenso die Degeneriertheit des genetischen Codes und die organismenspezifische Codon-Usage. Inzwischen werden Alignments über Computerprogramme erstellt, wie beispielsweise durch die Algorithmen FASTA oder BLAST; dieses Vorgehen wird beispielsweise von D. J. Lipman und W. R. Pearson (1985) in *Science,* Band 227, S. 1435-1441 beschrieben. Eine Zusammenstellung aller in den verglichenen Sequenzen übereinstimmenden Positionen wird als Consensus-Sequenz bezeichnet.

Solch ein Vergleich erlaubt auch eine Aussage über die Ähnlichkeit oder Homologie der verglichenen Sequenzen zueinander. Diese wird in Prozent Identität, das heißt dem Anteil der identischen Nukleotide oder Aminosäurereste an denselben Positionen widergegeben. Ein weiter gefaßter Homologiebegriff bezieht die konservierten Aminosäure-Austausche in diesen Wert mit ein. Es ist dann von Prozent Ähnlichkeit die Rede: Solche Aussagen können über ganze Proteine oder Gene oder nur über einzelne Bereiche getroffen werden.

Homologe Bereiche von verschiedenen Proteinen sind zumeist solche mit gleichen Strukturelementen und/oder Funktionen, die sich durch Übereinstimmungen in der primären Aminosäuresequenz erkennen lassen. Sie geht bis zu völligen Identitäten in kleinsten Bereichen, sogenannten Boxen, die nur wenige Aminosäuren umfassen und meist für die Gesamtaktivität essentielle Funktionen ausüben. Unter den Funktionen der homologen Bereiche sind kleinste Teilfunktionen der vom gesamten Protein ausgeübten Funktion zu verstehen, wie beispielsweise die Ausbildung einzelner Wasserstoffbrückenbindungen zur Komplexierung eines Substrats oder Übergangskomplexes.

Die enzymatische Aktivität kann durch andere Bereiche des Proteins, die nicht an der eigentlichen Reaktion beteiligt sind, qualitativ oder quantitativ modifiziert werden. Dies betrifft beispielsweise die Enzymstabilität, die Aktivität, die Reaktionsbedingungen oder die Substratspezifität.

Unter dem Begriff eines erfindungsgemäßen amylolytischen Proteins ist somit nicht allein eines mit der reinen, die Hydrolyse von α-1,4-glycosidischen Bindungen durchführenden Funktion zu verstehen, die auf die wenigen Aminosäurereste eines vermutlichen katalytisch aktiven Zentrums zurückzuführen sind. Er umfaßt darüberhinaus alle die Hydrolyse einer α-1,4-glycosidischen Bindung unterstützende Funktionen. Solche können beispielsweise von einzelnen Peptiden als auch von einem oder mehreren einzelnen Teilen eines Proteins durch Einwirken auf die eigentlich katalytisch aktiven Bereiche erreicht werden. Der Begriff der amylolytischen Funktion umfaßt auch allein solche modifizierenden Funktionen. Denn einerseits ist nicht genau bekannt, welche Aminosäurereste des erfindungsgemäßen Proteins tatsächlich die Hydrolyse katalysieren, und andererseits können nicht von vomherein bestimmte Einzelfunktionen definitiv von der Beteiligung an der Katalyse ausgenommen werden. Zu den Hilfsfunktionen oder Teilaktivitäten gehören beispielsweise die Bindung eines Substrats, eines Zwischen- oder Endprodukts, die Aktivierung oder die Inhibierung oder Vermittlung eines regulierenden Einflusses auf die hydrolytische Aktivität. Dabei kann es sich beispielsweise auch um die Ausbildung eines Strukturelements handeln, das fern vom aktiven Zentrum liegt, oder um ein Signalpeptid, dessen Funktion die Ausschleusung des gebildeten Proteins aus der Zelle und/oder dessen korrekte Faltung betrifft und ohne das *in vivo* in der Regel kein funktionsfähiges Enzym gebildet wird. Allerdings muß sich insgesamt eine Hydrolyse von α-1,4-glycosidischen Bindungen von Stärke oder stärkeähnlichen Polymeren ergeben.

Unter der Leistung eines Enzyms wird dessen Wirksamkeit im jeweils betrachteten technischen Bereich verstanden. Diese basiert auf der eigentlichen enzymatischen Aktivität, hängt darüberhinaus aber von weiteren, für den jeweiligen Prozeß relevanten Fatoren ab. Dazu gehören beispielsweise Stabilität, Substratbindung, Wechselwirkung mit dem das Substrat tragenden Material oder Wechselwirkungen mit anderen Inhaltsstoffen, insbesondere Synergien. So wird beispielsweise bei der Untersuchung, ob sich ein Enzym für den Einsatz in Wasch- oder Reinigungsmitteln eignet, dessen Beitrag zur Wasch- oder Reinigungsleistung eines mit weiteren Bestandteilen formulierten Mittels betrachtet. Für verschiedene technische Anwendungen kann ein Enzym über an sich bekannte molekularbiologische Techniken, insbesondere die oben genannten weiterentwickelt und optimiert werden.

Gemäß dem Budapester Vertrag über die internationale Anerkennung der Hinterlegung von Mikroorganismen vom 28. April 1977 ist für die vorliegende Anmeldung folgender Mikroorganismus bei der Deutschen Sammlung von Mikroorganismen und Zellkulturen GmbH in Braunschweig (DSMZ) hinterlegt worden: *Bacillus sp. A 7-7*. Er trägt dort die Registrierungsnummer DSM 12368 (DSM 98-587). Die maßgeblichen Angaben über die Merkmale dieses biologischen Materials, wie sie von der DSMZ bei der Hinterlegung bestimmt worden sind, werden in folgender Tabelle 1 zusammengestellt.

**Tabelle 1:**

| Mikrobiologische Eigenschaften des *Bacillus sp. A 7-7* (DSM 12368) (Bestimmt von der DSMZ am 9.10.1998) | |
|---|---|
| **Eigenschaft** | **Ergebnis** |
| Zellform | Stäbchen |
| Breite [µm] | 3,0 - 4,5 |
| Länge [µm] | 0,8 - 1,0 |
| Sporen | positiv/oval |
| Sporangium | angeschwollen |
| Oxidase | positiv |
| Katalase | positiv |
| Anaerobes Wachstum | positiv |
| VP Reaktion | negativ |
| pH in VP-Medium | 9,1 |
| Wachstum bei 40 °C | positiv/schwach |
| Wachstum bei 50 °C | negativ |
| Wachstum in | |
| Medium pH 7,0 | negativ |
| NaCl 2 % | positiv |
| NaCl 5 % | positiv |
| NaCl 7 % | positiv |
| NaCl 10 % | positiv |
| NaCl 12 % | negativ |
| NaCl 16 % | negativ |
| Lysozym-Medium | positiv |
| Säure aus | |
| D-Glucose | negativ |
| L-Arabinose | negativ |
| D-Xylose | negativ |
| D-Mannit | positiv |
| D-Fructose | positiv |
| Hydrolyse von | |
| Stärke | positiv |
| Gelatine | positiv |
| Casein | positiv |
| Tyrosin | schwach |
| Tween 80 | positiv |
| Tween 60 | positiv |
| Tween 40 | positiv |
| Tween 20 | negativ |
| Lecithinase | positiv |
| Pullulan | positiv |
| Hydrolyse von Hippurat | positiv |
| Esculin | positiv |
| Verwertung von | |
| Citrat | positiv |
| Propionat | positiv |
| NO₂ aus NO₃ | positiv |
| Indolreaktion | negativ |
| Phenylalanindesaminase | negativ |
| ERGEBNIS | *Bacillus sp.* (RNA-Gruppe VI, alcaliphil) |
| Bemerkung | Die physiologischen Testergebnisse deuten auf die Spezies *B. alcalophilus* oder *B. horikoshii*, können jedoch keine der genannten Spezies eindeutig identifizieren. Der Stamm zeigte 2 Kolonieformen, die jedoch mit Hilfe der Fettsäureanalyse als Varianten einer Spezies bestimmt wurden. Die partielle Sequenzierung der 16S rDNA ergab eine Übereinstimmung von 94,8% zu *B. alcalophilus*. Es handelt sich wahrscheinlich bei Stamm A 7-7 um den Vertreter einer neuen Spezies. |

Wie über diese Charakterisierung hinaus nun überraschenderweise festgestellt werden konnte, weist das von diesem Stamm produzierte amylolytische Enzym Eigenschaften auf, die es für den Einsatz in einer Vielzahl von technischen Prozessen prädestinieren. Zudem verfügt der Stamm über Eigenschaften, die die Kultivierbarkeit günstig gestalten.

Das erfindungsgemäße amylolytische Enzym des Stammes *Bacillus sp*. A 7-7 (DSM 12368) läßt sich, wie in Beispiel 2 im einzelnen dargelegt ist, folgendermaßen biochemisch charakterisieren: Es weist als reifes Protein in der denaturierenden SDS-Polyacrylgelelektrophorese ein apparentes Molekulargewicht von 58 kD auf, während sich aus der 516 Aminosäuren umfassenden Proteinsequenz (SEQ ID NO. 2) ein Molekulargewicht von circa 59 kD ableiten läßt, beziehungsweise nach Abspaltung des 31 Aminosäuren umfassenden Signalpeptids eines von 55,5 kD. Gemäß isoelektrischer Fokussierung liegt der isoelektrische Punkt des reifen Proteins bei 6,0. Es weist stärkespaltende Aktivität auf. Es ist bei Inkubation für 10 min bei pH 10 bis 50°C stabil.

Bei 60°C werden noch 50 % Restaktivität gefunden. Das Enzym ist bei Inkubation, über 10 min bei 40 °C weitgehend stabil zwischen pH-Werten von 5 und 12, die beste Stabilität wird bei pH 9 beobachtet. In Gegenwart von 0,1 % SDS, nach 15minütiger Inkubation bei pH 10 und 50°C weist das Enzym noch 98 % Restaktivität auf. In Gegenwart von zusätzlich 10 HPE/ml an Proteaseaktivität und nach 15minütiger Inkubation bei pH 10 und 50°C weist das Enzym noch 74 % Restaktivität auf.

Mit der vorliegenden Erfindung wird also ein natürlich vorkommendes Enzym zur Verfügung gestellt, welches aufgrund seiner Sequenzhomologien zu den bislang bekannten Enzymen und aufgrund seiner enyzmatischen Aktivität als α-Amylase angesehen werden muß. Es kann prinzipiell für alle Verwendungen eingesetzt werden, die einer amylolytischen Funktion bedürfen. Es eignet sich besonders für solche Verwendungen, die bei alkalischen pH-Werten und mittleren Temperaturbereichen ablaufen, insbesondere bei pH-Werten oberhalb von 9 und/oder Temperaturen oberhalb von 40°C. Das Anwendungsspektrum wird erweitert durch die vergleichsweise hohe Stabilität des Enzyms gegenüber Detergenzien und Proteasen. Es erscheint somit als besonders geeignet für den Einsatz in Wasch- oder Reinigungsmitteln.

Die Nukleotidsequenz dieses Enzyms wird im Sequenzprotokoll unter der Bezeichnung SEQ ID NO. 1 angegeben. Sie steht somit beispielsweise für Weiterentwicklungen über an sich bekannte molekularbiologische Methoden zur Verfügung. Die Aminosäuresequenz dieses Enzyms wird im Sequenzprotokoll unter der Bezeichnung SEQ ID NO. 2 angegeben.

Vergleichbare amylolytische Proteine stellen ebenfalls Ausführungsformen der vorliegenden Erfindung dar und werden insoweit beansprucht, wie sie Protein- oder DNA-Sequenzen aufweisen, die innerhalb des Ähnlichkeitsbereichs zu den in in SEQ ID NO. 1 und/oder SEQ ID NO. 2 angegebenen Sequenzen liegen. Dieser Ähnlichkeitsbereich umfaßt alle Proteine, deren Aminosäuresequenz zu der in SEQ ID NO. 2 angegebenen Aminosäuresequenz mindestens zu 96 %, zu 96,5 %, zu 97 %, zu 97,5 %, zu 98 %, zu 98,5 %, zu 99 %, zu 99,5 % oder zu 100 % identisch ist. Der Ähnlichkeitsbereich umfaßt auch alle Proteine, deren Nukleotidsequenz zu der in SEQ ID NO. 1 angegebenen Nukleotidsequenz mindestens zu 87,5 %, zu 90 %, zu 92,5 %, zu 95 %, zu 96 %, zu 97 %, zu 98 %, zu 99 % oder zu 100 % identisch ist. Dies gilt insbesondere für jene Teilbereiche des Proteins, die die Aminosäuren 32 bis 516 betreffen.

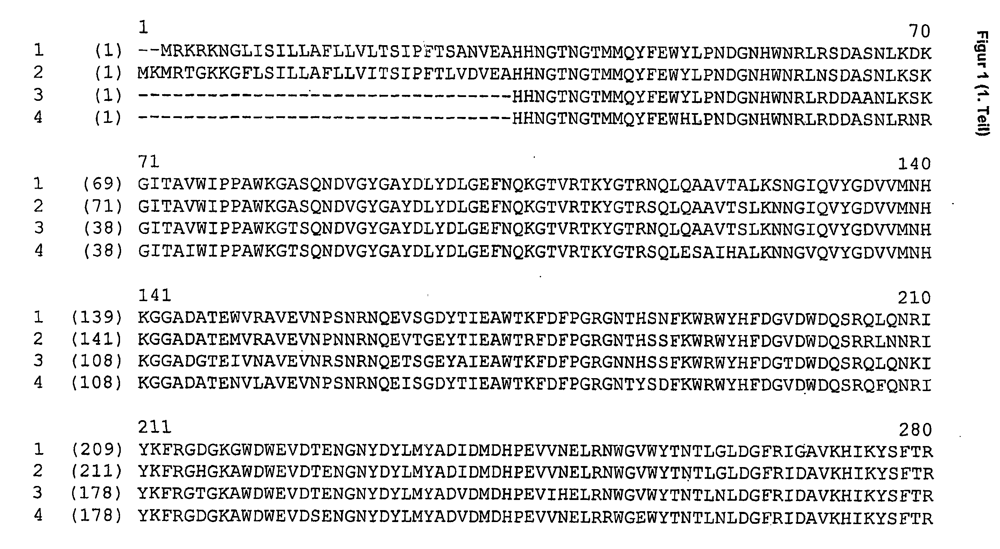

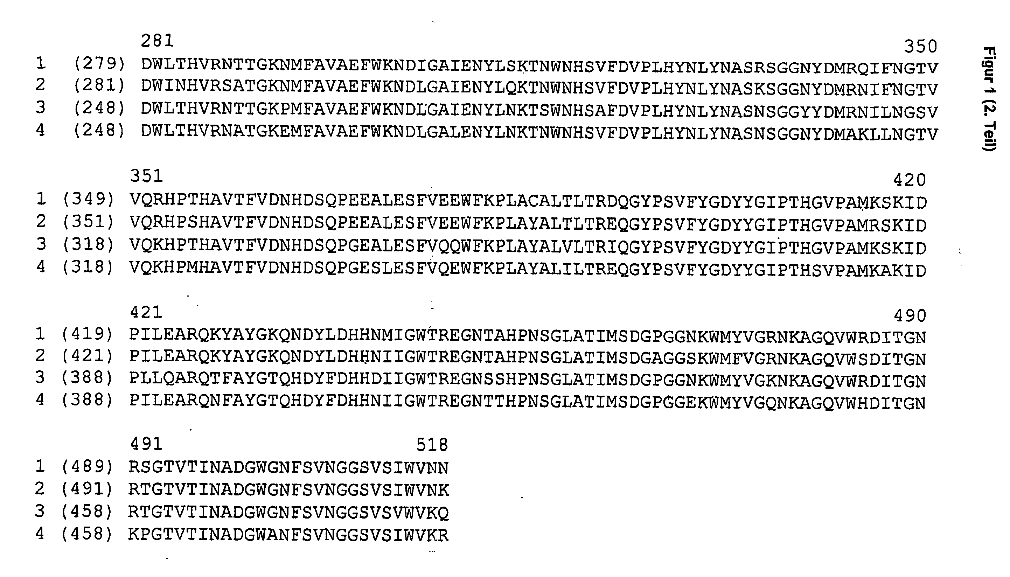

Bei dem nächstähnlichen, bis zum 17. 3. 2000 bekannten Protein handelt es sich um die α-Amylase aus *Bacillus alcalophilus*, mit der Bezeichnung P 19571 in der Datenbank Swiss-Prot (Geneva Bioinformatics (GeneBio) S.A., Genf, Schweiz; http://www.genebio.com/sprot.html). Dieses weist zum erfindungsgemäßen amylolytischen Enzym aus *Bacillus sp. A 7-7* (DSM 12368)eine Sequenz-Homologie von 93,4% Identität auf Proteinebene auf. Das erfindungsgemäße Protein ist über die homologen Bereiche eindeutig als α-Amylase charakterisiert. Repräsentative verwandte Proteine werden im Alignment der Figur 1 vorgestellt.

Aufgrund dieses Alignments können für erfindungsgemäße Proteine weitgehend dieselben Sekundär- und Tertiärstrukturen angenommen werden wie für die zur Homologisierung herangezogenen Proteine. Deren Strukturelemente können in allgemein zugänglichen Datenbanken wie beispielsweise am EMBL-European Bioinformatics Institute (EBI) in Cambridge, Großbritannien (http://www.ebi.ac.uk), Swiss-Prot oder GenBank (National Center for Biotechnology Information NCBI, National Institutes of Health, Bethesda, MD, USA) abgerufen werden. Sollten sich hiervon abweichende Strukturen ergeben oder sich herausstellen, daß verschiedene Faltungsvarianten mit variierenden amylolytischen Eigenschaften existieren, was beispielsweise die optimalen Reaktionsbedingungen oder die Substratspezifität angent, so werden diese alle in den Schutzbereich der vorliegenden Erfindung einbezogen. Denn zum einen kann die Faltung von den Herstellungsbedingungen abhängen, beispielsweise bei Anwesenheit oder Fehlen des Leader-Peptids. Zum anderen können sich diese Varianten für jeweils verschiedene Einsatzmöglichkeiten, beispielsweise für die quantitative Stärkeverflüssigung, für die Hydrolyse von Cyclodextrinen oder für den Einsatz in Wasch- oder Reinigungsmitteln als besonders geeignet herausstellen.

Besonderes Interesse gilt der Teilsequenz, die den Aminosäuren 32 bis 516 aus der in SEQ ID NO. 2 angegebenen Sequenz entspricht Denn die ersten 31 Aminosäuren stellen, wie aus der Aminosäuresequenz geschlossen werden kann, ein Signalpeptid dar, welches vermutlich bei Produktion in entsprechenden Mikroorganismen die Ausschleusung des Proteins aus dem Zellinneren in das die Zellen umgebende Medium einleitet. Dieses Signalpeptid wird *in vivo* nach der Ausschleusung abgespalten, so daß die eigentliche amylolytische Aktivität vom übrigen Teil des Proteins ausgeübt wird.

Für die eigentliche amylolytische Funktion sind die Aminosäuren 1 bis 31 somit wahrscheinlich von geringer Bedeutung, wohl aber für die Produktion, und zwar insbesondere für die gewünschte Faltung. Deshalb können sie nicht aus dem Schutzbereich der vorliegenden Erfindung ausgeschlossen werden.

Sollte sich herausstellen, daß bei der Herstellung, beispielsweise durch den einen oder anderen Bakterienstamm, Abweichungen in der Länge des Signalpeptids und/oder des maturen Proteins ergeben, so gelten die auf die Positionen 1 bis 31, beziehungsweise 32 bis 516 gemäß SEQ ID NO.2 bezogenen Ansprüche entsprechend für die abweichenden Varianten. So wäre es beispielsweise möglich, daß die Translation des Proteins aus *Bacillus sp.* A 7-7 (DSM 12368) bereits sechs Nukleotide vor der in SEQ ID NO. 1 angebenen Nukleotidsequenz einsetzt. Vor diesem als wahrscheinlich angenommenen Anfang liegen die sechs Nukleotide ATG ACG. Diese könnten als Met-Thr translatiert werden, wodurch das Signalpeptid N-terminal um zwei Aminosäuren länger wird und sich eine gewisse Ähnlichkeit zu der in Figur 1 unter Nummer 2 angegebenen Amylase aus *Bacillus sp.* #707 ergibt. Ebenso sind auch Variationen bei der Abspaltung des Signalpeptids möglich.

Unter den innerhalb des oben angegebenen Ähnlichkeitsbereich liegenden Varianten sind insbesondere solche bevorzugt, die hinsichtlich der vorgesehenen Einsatzmöglichkeiten optimierte Eigenschaften aufweisen. Solche sind, wie einleitend dargestellt, nach an sich bekannten, vorzugsweise molekularbiologischen Methoden herstellbar. Beispielsweise wäre es auch möglich, in Anwendung der Lehre aus der Anmeldung WO 94/18314 Methionin-, Tryptophan-, Cystein- und/oder Tyrsosinreste erfindungsgemäßer Proteine zu deletieren und/oder gegen weniger leicht oxidierbare Aminosäurereste zu substitutieren. Dadurch können die Oxidationsstabilität, das pH-Aktivitätsprofil und/oder die Thermostabilität verbessert werden. Weiterentwicklungen über gezielte Punktmutagenese können sich beispielsweise auch an den Anmeldungen WO 99/09183 und WO 99/23211 orientieren.

Unter erfindungsgemäßen Fragmenten werden alle Proteine oder Peptide verstanden, die kleiner sind als jene Proteine, die denen von SEQ ID NO. 1 oder SEQ ID NO. 2 entsprechen, zu ihnen in den entsprechenden Teilsequenzen aber hinreichend homolog sind. Sofern sie eine amylolytische oder wenigstens eine die Hydrolyse einer α-1,4-glycosidischen Bindung unterstützende Funktion ausüben, werden sie als amylolytisch aktive Fragmente angesehen und stellen Ausführungsformen der vorliegenden Erfindung dar. Das betrifft beispielsweise solche Fragmente, die zur Komplexierung eines Substrats oder zur Ausbildung eines für die Hydrolyse erforderlichen Strukturelements beitragen. Bei den Fragmenten kann es sich beispielsweise um einzelne Domänen handeln oder um Bruchstücke, die nicht mit den Domänen übereinstimmen. Solche Fragmente können kostengünstiger herzustellen sein, bestimmte eventuell nachteilige Charakteristika des Ausgangsmoleküls nicht mehr besitzen, wie möglicherweise einen aktivitätssenkenden Regulationsmechanismus, oder ein günstigeres Aktivitätsprofil entfalten. Derartige Proteinfragmente können auch nicht-biosynthetisch, sondem beispielsweise chemisch hergestellt werden. Die chemische Synthese kann beispielsweise dann vorteilhaft sein, wenn im Anschluß an die Synthese chemische Modifikationen vorgenommen werden sollen.

Den Fragmenten sind wegen ihrer prinzipiellen Gleichartigkeit auch durch Deletionsmutation erhältliche Proteine zuzuordnen. Solche können biochemisch weitgehend mit den Ausgangsmotekülen übereinstimmen oder einzelne Funktionen gerade nicht mehr aufweisen. Dies erscheint beispielsweise bei der Deletion inhibierender Bereiche besonders sinnvoll. Im Ergebnis können mit den Deletionen sowohl eine Spezialisierung als auch eine Erweiterung des Anwendungsbereichs des Proteins einhergehen. Sofern dadurch eine im weitesten Sinne amylolytische Funktion aufrechterhalten, modifiziert, spezifiziert oder auch erst erreicht wird, handelt es sich bei den durch Deletionsvarianten wie bei den Fragmenten um erfindungsgemäße Proteine; einzige zusätzliche Voraussetzung dafür ist, daß sie über die noch vorhandene homologe Teilsequenz hinweg innerhalb des oben bereits angegeben Ähnlichkeitsbereichs zu den Sequenzen SEQ ID NO. 1 und SEQ ID NO. 2 liegen.

So ist es beispielsweise in Anlehnung an WO 99/57250 möglich, ein erfindungsgemäßes Protein oder Teile davon über peptidische oder nicht-peptidische Linker mit Bindungsdomänen aus anderen Proteinen zu versehen und dadurch die Hydrolyse des Substrats effektiver zu gestalten. Solche Konstrukte liegen dann innerhalb des Schutzbereichs der vorliegenden Erfindung, wenn sie amylolytische Aktivitäten ausüben und die Teile des Konstrukts, die diese Funktion ausüben, zu den angegebenen erfindungsgemäßen Sequenzen hinreichend ähnlich sind. Ebenso können auch erfindungsgemäße amylolytische Proteine beispielsweise auch mit Proteasen verknüpft werden, um eine Doppelfunktion auszuüben.

Als natürlicherweise gebildete Fragmente, beziehungsweise deletionsmutierte Proteine kann man auch die von Präproteinen durch Abspaltung der N-terminalen Aminosäuren erhältlichen Proteine und Signalpeptide ansehen. Solch ein Spaltungsmechanismus kann auch dazu verwendet werden, um mithilfe bestimmter Sequenzbereiche, die von Signalpeptidasen erkannt werden, in rekombinanten Proteinen spezifische Spaltstellen vorzugeben. Somit können *in vitro* Aktivierung und/oder Deaktivierung erfindungsgemäßer Proteine vorgenommen werden. Auf jedes dieser Proteine erstreckt sich der Schutzbereich der vorliegenden Erfindung, sofern es innerhalb des beanspruchten Schutzbereichs liegt und es eine amylolytische Aktivität vermittelt.

Unter erfindungsgemäßen chimären oder hybriden Proteinen sind solche Proteine zu verstehen, die aus Elementen zusammengesetzt sind, die natürlicherweise von verschiedenen Polypeptidketten stammen. Dieses Vorgehen wird auch Shuffling-oder Fusionsmutagenese genannt. Es handelt sich dann um erfindungsgemäße chimäre Proteine, wenn die durch die Fusion erhaltenen Proteine eine im weitesten Sinne amylolytische Aktivität aufweisen. Diese kann von einem Molekülteil ausgeübt oder modifiziert werden, das sich von einem erfindungsgemäßen Protein herleitet und innerhalb des beanspruchten Ähnlichkeitsbereichs liegt. Der Sinn einer solchen Fusion kann beispielsweise darin bestehen, mithilfe des heranfusionierten erfindungsgemäßen Proteinteils eine amylolytische oder die Hydrolyse von α-1,4-glycosidischen Bindungen unterstützende Funktion herbeizuführen oder zu modifizieren. Es ist dabei im Sinne der vorliegenden Erfindung unwesentlich, ob solch ein chimäres Protein aus einer einzelnen Polypeptidkette oder mehreren Untereinheiten besteht, auf welche sich unterschiedliche Funktionen verteilen können. Zur Realisierung der letztgenannten Alternative ist es beispielsweise möglich, posttranslational oder erst nach einem Aufreinigungsschritt durch eine gezielte proteolytische Spaltung eine einzelne chimäre Polypeptidkette in mehrere zu zerlegen. Zum Gegenstand der Erfindung gehören auch solche chimären Proteine, die aufgrund ihrer Konstruktion über ihre gesamte Aminosäure- und oder Nukleotidsequenz hinweg eine gegebenenfalls geringere Identität aufweisen als für den erfindungsgemäßen Ähnlichkeitsbereich oben definiert, die ihm aber in mindestens einer der durch Fusion eingebrachten Bereiche zugerechnet werden können und in diesem Teil dieselben Funktionen wie in einer Amylase ausüben, die über ihre ganze Länge in den genannten Homologiebereich fällt.

Unter erfindungsgemäßen, durch Insertionsmutation erhältlichen Proteinen sind Varianten von den über ihre volle Sequenzlänge in die bezeichneten Schutzbereiche der Sequenzen SEQ ID NO. 1 oder SEQ ID NO 2 fallenden Proteine zu verstehen, die durch Einfügen eines Nukleinsäure-, beziehungsweise Proteinfragments in die betreffenden Sequenzen erhalten worden sind. Der Sinn von Insertionsmutagenese kann wie der der Hybridbildung darin bestehen, einzelne oder Eigenschaften erfindungsgemäßer Proteine mit denen anderer Proteine zu kombinieren. Es handelt sich dann um erfindungsgemäße, durch Insertionsmutation erhaltene Proteine oder chimäre Proteine, wenn die über ihre Homologie auf die Sequenzen SEQ ID NO.1 oder SEQ ID NO. 2 zurückzuführenden Bereiche entsprechende Homologiewerte aufweisen und das Protein aufgrund dieser Bereiche eine im weitesten Sinne amylolytische Funktion besitzt.

Auch durch Inversionsmutagenese erhaltene Proteine und solche mit einer von der ursprünglichen Aminosäureabfolge abweichende Neugruppierung verschiedener Molekülteile sind somit in den Schutzbereich der vorliegenden Erfindung eingeschlossen. Dasselbe gilt für. Sie kann sowohl als Deletionsvariante, als Insertionsvariante, als auch als Shuffling-Variante des ursprünglichen Proteins angesehen werden.

Unter erfindungsgemäßen, amylolytisch aktiven Derivaten werden solche amylolytischen Proteine verstanden, die modifiziert worden sind, beispielsweise im Zusammenhang mit der Proteinbiosynthese über Prozessierung durch den Wirtsorganismus oder chemisch, etwa durch die Umwandlung einer Seitenkette einer Aminosäure oder durch kovalente Bindung einer anderen Verbindung an das Protein. Bei solch einer Verbindung kann es sich beispielsweise um andere Proteine handeln, die beispielsweise über bifunktionelle chemische Verbindungen an erfindungsgemäße Proteine gebunden werden. Derartige Modifikationen können beispielsweise die Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt. Dies kann beispielsweise für den Zeitraum der Lagerung sinnvoll sein. Eine weitere Ausführungsform sind solche Derivate, die durch kovalente Bindung an einen makromolekularen Träger, wie beispielsweise Polythylenglykol oder ein Polysaccharid erhalten worden sind.

Weitere Lösungen der erfindungsgemäßen Aufgabe sind amylolytische Proteine oder Derivate, die wenigstens eine antigene Determinante mit einem der oben genannten Proteine oder Derivate gemeinsam haben.

Denn entscheidend für die Ausübung enzymatischer Aktivitäten ist nicht allein die pure Aminosäureabfolge eines Proteins, sondern auch dessen Sekundärstrukturelemente und dessen dreidimensionale Faltung. So können in ihrer Primärstruktur deutlich voneinander abweichende Domänen räumlich weitgehend übereinstimmende Strukturen ausbilden und somit gleiches enzymatisches Verhalten ermöglichen. Solche Gemeinsamkeiten in der Sekundärstruktur werden üblicherweise als übereinstimmende antigene Determinanten von Antiseren oder reinen oder monoklonalen Antikörpern erkannt. Ober immunchemische Kreuzreaktionen können somit einander strukturell ähnliche Proteine oder Derivate detektiert und zugeordnet werden.

Deshalb werden in den Schutzbereich der vorliegenden Erfindung gerade auch solche Proteine oder Derivate einbezogen, die amylolytische Aktivität aufweisen und möglicherweise nicht über ihre Homologiewerte in der Primärstruktur, wohl aber über ihre immunchemische Verwandtschaft den oben definierten erfindungsgemäßen Proteinen oder Derivaten zugeordnet werden können.

Erfindungsgemäße Proteine, die aus natürlichen Quellen stammen, sind bevorzugte Ausführungsformen der vorliegenden Erfindung, insbesondere wenn sie aus Mikroorganismen wie einzellige Pilze oder Bakterien stammen. Denn diese lassen sich zumeist einfacher handhaben als vielzellige Organismen oder die von Vielzellem abgeleiteten Zellkulturen. Diese stellen für spezielle Ausführungsformen sinnvolle Optionen dar.

Besonders bevorzugt sind erfindungsgemäße Proteine oder Derivate aus gram-positiven Bakterien, weil diese keine äußere Membran besitzen und sekretierte Proteine somit unmittelbar ins umgebende Medium abgeben.

Ganz besonders bevorzugt sind erfindungsgemäße Proteine oder Derivate aus gram-positiven Bakterien der Gattung Bacillus, weil diese als Produktionsorganismen mit einer besonders hohen Produktionsleistung in technischen Prozessen etabliert sind.

Unter den erfindungsgemäßen Proteine oder Derivate aus Bacillus-Species, wiederum, sind die aus alkaliphilen Bacilli bevorzugt, darunter insbesondere aus *Bacillus sp.* A 7-7, und ganz besonders aus dem Stamm *Bacillus sp.* A 7-7 (DSM 12368). Denn aus diesem wurde die Ausführungsform des erfindungsgemäßen Enzyms, deren zugehörige Sequenzen im Sequenzprotokoll angegeben sind und dessen enzymatische Charakteristika in den Beispielen beschrieben sind, ursprünglich erhalten.

Aus produktionstechnischen Gründen sind jeweils solche Stämme bevorzugt, die das gebildete amylolytische Protein in das sie umgebende Medium abgeben.

Es ist möglich, daß natürlich vorkommende Produzenten zwar ein erfindungsgemäßes amylolytisches Enzym herstellen können, dieses aber unter den zunächst ermittelten Bedingungen nur in geringem Maße exprimieren und/oder in das umgebende Medium abgeben. Sie unterfallen dennoch solange dem Schutzbereich der vorliegenden Erfindung, wie die Möglichkeit besteht, geeignete Umweltbedingungen oder niedermolekulare oder sonstige Faktoren experimentell zu ermitteln, unter deren Einwirken sie zu einer Produktion des erfindungsgemäßen Proteins angeregt werden können, die eine wirtschaftliche Nutzung sinnvoll erscheinen läßt. Solch ein Regulationsmechanismus kann für die biotechnologische Produktion gezielt eingesetzt werden, zum Beispiel zur Regulation der verantwortlichen Promotoren.

Je nach Gewinnung, Aufarbeitung oder Präparation eines Proteins kann es mit diversen anderen Stoffen vergesellschaftet sein, insbesondere wenn es aus natürlichen Produzenten dieses Proteins gewonnen worden ist. Es kann dann, aber auch unabhängig davon, mit bestimmten anderen Stoffen gezielt versetzt worden sein, beispielsweise zur Erhöhung seiner Lagerstabilität. Unter dem Begriff des erfindungsgemäßen Proteins sind deshalb zusätzlich auch alle Präparationen des eigentlichen erfindungswesentlichen Proteins zu verstehen. Das ist auch unabhängig davon, ob es in einer bestimmten Präparation tatsächlich diese enzymatische Aktivität entfaltet oder nicht. Denn es kann gewünscht sein, daß es bei der Lagerung keine oder nur geringe Aktivität besitzt, und erst zum Zeitpunkt der Verwendung seine amylolytische Funktion entfaltet. Dies kann beispielsweise vom Faltungszustand des Proteins abhängig sein oder aus der reversiblen Bindung eines oder mehrer Begleitstoffe aus der Präparation oder aus einem sonstigen Kontrollmechanismus resultieren.

Erfindungsgemäße Proteine können besonders während der Lagerung durch Stabilisatoren beispielsweise vor Denaturierung, Zerfall oder Inaktivierung, etwa durch physikalische Einflüsse, Oxidation oder proteolytische Spaltung geschützt werden. Bei Proteinen, die aus Mikroorganismen erhalten werden, ist eine Inhibierung der Proteolyse besonders kritisch, weil die meisten Mikroorganismen verschiedene Proteasen als Verdauungsenzyme in die umgebenden Medien sekretieren. Diese können die interessierenden Proteine während der nachfolgenden Aufreinigungstufen erheblich schädigen.

Eine Gruppe von Stabilisatoren sind reversible Proteaseinhibitoren, wie beispielsweise Benzamidin-Hydrochlorid und Leupeptin, Borax, Borsäuren, Boronsäuren, deren Salze oder Ester, Peptidaldehyde oder rein peptidische Inhibitoren wie Ovomucoid oder spezifische Subtilisin-Inhibitoren. Weitere geläufige Enzymstabilisatoren sind Aminoalkohole wie Mono-, Di-, Triethanol- und -propanolamin, aliphatische Carbonsäuren bis zu C₁₂, Dicarbonsäuren, niedere aliphatische Alkohole, v.a. aber Polyole, wie beispielsweise Glycerin, Ethylenglykol, Propylenglykol oder Sorbit. Ebenso werden Calciumsalze verwendet, wie beispielsweise Calciumacetat oder Calcium-Formiat, Magnesiumsalze, verschiedenste Polymere wie beispielsweise Lignin, Cellulose-Ether, Polyamide oder wasserlösliche Vinyl-Copolymere, um die Enzym-Präparation v.a. gegenüber physikalischen Einflüssen oder pH-Wert-Schwankungen zu stabilisieren. Reduktionsmittel und Antioxidantien, wie beispielsweise Natrium-Sulfit oder reduzierende Zucker, erhöhen die Stabilität der Proteine gegenüber oxidativem Zerfall.

Auch in der Ausführungsform entsprechender Nukleinsäuren wird die vorliegende Erfindung verwirklicht, sofern die betreffenden Nukleinsäuren für ein im weitesten Sinne amylolytisches Protein codieren und zu der unter SEQ ID NO. 1 angegebenen Sequenz eine hinreichende, oben definierte Ähnlichkeit aufweisen, insbesondere solche Nukleinsäuren, die für ein Protein codieren, das dem Teilbereich der Aminosäuren von 32 bis 516 der in SEQ ID NO. 1 angegebenen Aminosäuresequenz entspricht.

Besonders bevorzugte Ausführungsformen stellen jene Nukleinsäuren dar, die für eines der oben ausgeführten erfindungsgemäßen amylolytischen Proteine codieren. Dies schließt auch jene Varianten ein, die nicht über ihre ganze Sequenzlänge hinweg in den nach SEQ ID NO.1 definierten Ähnlichkeitsbereich fallen, dies aber in einzelnen Bereichen tun. Dazu gehören beispielsweise die Nukleotidsequenzen, die, wie oben ausgeführt, durch Insertions- oder Deletionsmutation erhalten worden sind, chimäre Proteine oder Proteinfragmente. Aber auch sogenannte Antisense-Konstrukte, beispielsweise über einzelne Teilabschnitte, stellen Ausführungsformen der vorliegenden Erfindung dar, weil sie zur Regulation der amylolytischen Aktivität eingesetzt werden können.

Nukleinsäuren bilden den Ausgangspunkt für molekularbiologische Untersuchungen und Weiterentwicklungen. Solche Methoden sind beispielsweise in dem Handbuch von Fritsch, Sambrook und Maniatis "Molecular cloning: a laboratory manual", *Cold Spring Harbour Laboratory Press,* New York, 1989 beschrieben. Auf dem Gen, insbesondere auf dem klonierten Gen beruhen auch alle im Stand der Technik unter dem Begriff Protein Engineering zusammengefaßten gentechnischen und Protein-biochemischen Methoden. Mit solchen können erfindungsgemäße Proteine in Hinblick auf verschiedene Verwendungen weiter optimiert werden, beispielsweise durch Punktmutagenese oder durch Fusion mit Sequenzen aus anderen Genen.

Zu den über an sich bekannte molekularbiologische Methoden erhältlichen, erfindungsgemäßen Varianten eines Proteins gehören insbesondere solche mit einzelnen gezielten Aminosäureaustauschen oder randomisierten Punktmutationen, Deletionen einzelner Aminosäuren oder von Teilsequenzen, Fusionen mit anderen Fragmenten oder anderen Enzymen, Insertionen oder Inversionen, also partielle Sequenzumkehrungen. Derartige Mutationen oder Modifikationen können bevorzugte Ausführungsformen für spezifische Anwendungen darstellen. Solch eine Mutagenese kann zielgerichtet oder über zufallsgemäße Methoden durchgeführt werden. Dies kann beispielsweise mit einem anschließenden auf die Aktivität gerichteten Erkennungs- und/oder Auswahlverfahren (Screening und Selektion) an den klonierten Genen kombiniert werden. Die durch Mutantion erhaltenen Gene unterliegen dem Schutzbereich der hier beschriebenen Erfindung, wenn sie für im weitesten Sinne amylolytische Proteine codieren und in dem oben definierten Ähnlichkeitsbereich liegen; letzteres zumindest in den homologen und funktionsrelevanten Bereichen.

Eine weitere Lösung der erfindungsgemäßen Aufgabe und damit einen eigenen Erfindungsgegenstand stellen die Organismen dar, die ein erfindungsgemäßes Protein oder Derivat natürlicherweise bilden oder Nukleinsäuren enthalten, die für ein erfindungsgemäßes Protein oder Derivat codieren. Denn deren Auffinden ermöglicht das Umsetzen des Erfindungsgedankens. Derartige Organismen sind in Anwendung allgemein bekannter Techniken, beispielsweise durch Isolieren von Stämmen aus einem natürlichen Habitat oder durch Screening von Genbanken erhältlich. Die in SEQ ID NO. 1 angegebene Nukleotidsequenz kann hierbei beispielsweise als Sonde zum Screening eingesetzt werden oder als Vorlage für die Konstruktion entsprechender PCR-Primer dienen. Analog dazu können kurzkettige oder vollständige Peptide mit Aminosäuresequenzen nach SEQ ID NO. 2 zur Bildung entsprechender Antiseren verwendet werden, mit deren Hilfe entsprechende Organismen, beziehungsweise die von ihnen freigesetzten Proteine identifiziert werden können.

Entsprechend der oben gemachten Ausführungen sind vor allem aufgrund der Kultivierbarkeit Mikroorganismen, vorzugsweise Bakterien, hierunter gram-positive Bakterien, hierunter solcher der Gattung Bacillus, insbesondere *Bacillus sp.* A 7-7 und ganz besonders um *Bacillus sp.* A 7-7 DSM (12368) bevorzugt.

Einem eigenen Erfindungsgegenstand werden Vektoren zugerechnet, die einen der Nukleinsäurebereiche des zweiten Erfindungsgegenstands enthalten.

Denn um mit Nukleinsäuren umzugehen, wird die DNA geeigneterweise in einen Vektor kloniert. Dazu gehören beispielsweise solche, die sich von bakteriellen Plasmiden, von Viren oder von Bacteriophagen ableiten, oder überwiegend synthetische Vektoren oder Plasmide mit Elementen verschiedenster Herkunft. Mit den weiteren jeweils vorhandenen genetischen Elementen vermögen Vektoren, sich in den betreffenden Wirtszellen über mehrere Generationen hinweg als stabile Einheiten zu etablieren. Es ist dabei im Sinne der Erfindung unerheblich, ob sie sich extrachomosomal als eigene Einheiten etablieren oder in ein Chromosom integrieren. Welches der zahlreichen aus dem Stand der Technik bekannten Systeme gewählt wird, hängt vom Einzelfall ab. Ausschlaggebend können beispielsweise die erreichbare Kopienzahl, die zur Verfügung stehenden Selektionssysteme, darunter vor allem Antibiotikaresistenzen, oder die Kultivierbarkeit der zur Aufnahme der Vektoren befähigten Wirtszellen sein.

Die Vektoren bilden geeignete Ausgangspunkte für molekularbiologische und biochemische Untersuchungen des betreffenden Gens oder zugehörigen Proteins und für erfindungsgemäße Weiterentwicklungen und letztlich für die Amplifikation und Produktion erfindungsgemäßer Proteine. Sie stellen insofern Ausführungsformen der vorliegenden Erfindung dar, als die Sequenzen der enthaltenen erfindungsgemäßen Nukleihsäurebereiche innerhalb des oben näher bezeichneten Homologiebereichs liegen.

Bevorzugte Ausführungsformen der vorliegenden Erfindung sind Klonierungsvektoren. Diese eignen sich neben der Lagerung, der biologischen Amplifikation oder der Selektion des interessierenden Gens für die Charakterisierung des betreffenden Gens, etwa über das Erstellen einer Restriktionskarte oder die Sequenzierung. Klonierungsvektoren sind auch deshalb bevorzugte Ausführungsformen der vorliegenden Erfindung, weil sie eine transportierbare und lagerfähige Form der beanspruchten DNA darstellen. Sie sind auch bevorzugte Ausgangspunkte für molekularbiologische Techniken, die nicht an Zellen gebunden sind, wie beispielsweise die Polymerasekettenreaktion.

Expressionsvektoren besitzen Teilsequenzen, die sie dazu befähigen, in den für die Produktion von Proteinen optimierten Wirtsorganismen zu replizieren und dort das enthaltene Gen zur Expression zu bringen. Bevorzugte Ausführungsformen sind Expressionsvektoren, die selbst die zur Expression notwendigen genetischen Elemente tragen. Die Expression wird beispielsweise von Promotoren beeinflußt, welche die Transkription des Gens regulieren. So kann die Expression durch den natürlichen, ursprünglich vor diesem Gen lokalisierten Promotor erfolgen, aber auch nach gentechnischer Fusion sowohl durch einen auf dem Expressionsvektor bereitgestellten Promotor der Wirtszelle als auch durch einen modifizierten oder einen völlig anderen Promotor eines anderen Organismus.

Bevorzugte Ausführungsformen sind solche Expressionsvektoren, die über Änderungen der Kulturbedingungen oder Zugabe von bestimmten Verbindungen, wie beispielsweise die Zelldichte oder spezielle Faktoren, regulierbar sind. Expressionsvektoren ermöglichen, daß das zugehörige Protein heterolog, also in einem anderen Organismus als dem, aus dem es natürlicherweise gewonnen werden kann, produziert wird. Auch eine homologe Proteingewinnung aus einem das Gen natürlicherweise exprimierenden Wirtsorganismus über einen passenden Vektor liegt innerhalb des Schutzbereichs der vorliegenden Erfindung. Diese kann den Vorteil aufweisen, daß natürliche, mit der Translation in einem Zusammenhang stehende Modifikationsreaktionen an dem entstehenden Protein genauso durchgeführt werden, wie sie auch natürlicherweise ablaufen würden.

Ausführungsformen der vorliegenden Erfindung können auch zellfreie Expressionssysteme sein, bei denen die Proteinbiosynthese *in vitro* nachvollzogen wird. Derartige Expressionssysteme sind im Stand der Technik ebenfalls etabliert.

Eine weitere Ausführungsform dieses Erfindungsgegenstands stellen Zellen dar, die einen der oben definierten Vektoren, insbesondere einen Klonierungs- oder einen Expressionsvektor enthalten. Denn insbesondere im Zuge molekularbiologischer Arbeiten, wie sie beispielsweise für die Mutagenese, die Sequenzierung oder Lagerung der Vektoren nötig sind, erfolgt deren Transformation in entsprechende Zellen. Hierfür können, abhängig von der Methode, beispielsweise gram-positive, insbesondere aber auch gram-negative Bakterien geeignet sein.

Eine weitere Ausführungsform sind Wirtszellen, die ein Protein oder Derivat des ersten Erfindungsgegenstands exprimieren oder zu deren Expression angeregt werden können, vorzugsweise unter Einsatz eines oben definierten Expressionsvektors.

Denn die bevorzugte *In-vivo*-Synthese eines erfindungsgemäßen amylolytischen Enzyms erfordert den Transfer des zugehörigen Gens in eine Wirtszelle. Als Wirtszellen eignen sich prinzipiell alle Organismen, das heißt Prokaryonten, Eukaryonten oder Cyanophyta. Bevorzugt sind solche Wirtszellen, die sich genetisch gut handhaben lassen, was beispielsweise die Transformation mit dem Expressionsvektor und dessen stabile Etablierung angeht, beispielsweise einzellige Pilze oder Bakterien. Zudem zeichnen sich bevorzugte Wirtszellen durch eine gute mikrobiologische und biotechnologische Handhabbarkeit aus. Das betrifft beispielsweise leichte Kultivierbarkeit, hohe Wachstumsraten, geringe Anforderungen an Fermentationsmedien und gute Produktions- und Sekretionsraten für Fremdproteine. Häufig müssen aus der Fülle an verschiedenen nach dem Stand der Technik zur Verfügung stehenden Systeme die optimalen Expressionssysteme für den Einzelfall experimentell ermitteln werden.

Jedes erfindungsgemäße Protein kann auf diese Weise aus einer Vielzahl von Wirtsorganismen gewonnen werden.

Bevorzugte Ausführungsformen stellen solche Wirtszellen dar, die aufgrund genetischer Regulationselemente, die beispielsweise auf dem Expressionsvektor zur Verfügung gestellt werden, aber auch von vornherein in diesen Zellen vorhanden sein können, in ihrer Aktivität regulierbar sind. Beispielsweise durch kontrollierte Zugabe - von chemischen Verbindungen, die als Aktivatoren dienen, durch Änderung der Kultivierungsbedingungen oder bei Erreichen einer bestimmten Zelldichte können diese zur Expression angeregt werden. Dies ermöglicht eine sehr wirtschaftliche Produktion der interessierenden Proteine.

Eine Variante dieses Versuchsprinzips stellen Expressionssysteme dar, bei denen zusätzliche Gene, beispielsweise solche, die auf anderen Vektoren zur Verfügung gestellt werden, die Produktion erfindungsgemäßer Proteine beeinflussen. Hierbei kann es sich um modifizierende Genprodukte handeln oder um solche, die mit dem erfindungsgemäßen Protein gemeinsam aufgereinigt werden sollen, etwa um dessen amylolytische Funktion zu beeinflussen. Dabei kann es sich beispielsweise um andere Proteine oder Enzyme, um Inhibitoren oder um solche Elemente handeln, die die Wechselwirkung mit verschiedenen Substraten beinflussen.

Bevorzugte Wirtszellen sind prokaryontische oder bakterielle Zellen. Bakterien zeichnen sich gegenüber Eukaryonten in der Regel durch kürzere Generationszeiten und geringere Ansprüche an die Kultivierungsbedingungen aus. Dadurch können kostengünstige Verfahren zur Gewinnung erfindungsgemäßer Proteine etabliert werden.

Insbesondere bevorzugt sind Wirtszellen, insbesondere Bakterien, die das gebildete Protein oder Derivat ins umgebende Medium sekretieren, so daß die exprimierten erfindungsgemäßen Proteine direkt aufgereinigt werden können.

Eine Ausführungsform der vorliegenden Erfindung nutzt *Bacillus sp.* A 7-7 (DSM 12368) selbst, um erfindungsgemäße Proteine homolog zu exprimieren. Dies kann beispielsweise über einen eingebrachten Vektor erfolgen, der das bereits endogen vorhandene Gen, oder erfindungsgemäße Abwandlungen desselben, in diese Zellen einbringt, beispielsweise in einer vielfachen Kopienzahl. Dies kann dann besonders vorteilhaft sein, wenn das Protein im Anschluß an seine Synthese Modifikationen erfahren soll, die geeigneterweise von den betreffenden Zellen selbst durchgeführt werden.

Demgegenüber ist jedoch die heterologe Expression bevorzugt. Grampositive Bakterien, wie beispielsweise Actinomycten oder Bacilli, besitzen keine äußere Membran, so daß sie sekretierte Proteine unmittelbar in das sie umgebende Medium abgeben. Zu den für die heterologe Expression bevorzugten Bakterien gehören somit solche der Gattung Bacillus, insbesondere solche der Species *Bacillus licheniformis, Bacillus amyloliquefaciens, Bacillus subtilis* oder *Bacillus alcalophilus*.

Auch gram-negative Bakterien können für die heterologe Expression genutzt werden. Bei ihnen werden eine Vielzahl von Proteinen in den periplasmatischen Raum sekretiert, also in das Kompartiment zwischen den beiden die Zellen einschließenden Membranen. Dies kann für spezielle Anwendungen vorteilhaft sein. Hierzu gehören beispielsweise solche der Gattungen Klebsiella oder Escherichia, bevorzugt der Spezies *Escherichia coli*, besonders bevorzugt der Stämme *E. coli* JM 109, *E. coli* DH 100B oder *E. coli* DH 12S.

Auch eukaryontische Zellen können sich zur Produktion erfindungsgemäßer amylolytischer Proteine eignen. Beispiele dafür sind Hefen wie *Saccharomyces* oder *Kruyveromyces*. Dies kann beispielsweise dann besonders vorteilhaft sein, wenn die Proteine im Zusammenhang mit ihrer Synthese spezifische Modifikationen erfahren sollen, die derartige Systeme ermöglichen. Dazu gehören beispielsweise die Bindung niedermolekularer Verbindungen wie Membrananker oder Oligosaccaride.

Alle bereits oben ausgeführten Elemente können zu Verfahren kombiniert werden, um erfindungsgemäße Proteine herzustellen. Es ist dabei für jedes erfindungsgemäße Protein eine Vielzahl von Kombinationsmöglichkeiten an Verfahrensschritten denkbar. Sie alle verwirklichen die der vorliegenden Erfindung zugrundeliegende Idee, nämlich Vertreter eines über die amylolytische Funktion und gleichzeitig die hohe Homologie zu den in den Sequenzprotokollen angegebenen Sequenzen definierten Proteintyps mithilfe der zugehörigen genetischen Information quantitativ herzustellen. Das optimale Verfahren muß für jeden konkreten Einzelfall experimentell ermittelt werden.

Prinzipiell wird dabei folgendermaßen vorgegangen: Erfindungsgemäße Nukleinsäuren, also solche die innerhalb des oben definierten Ähnlichkeitsbereiches zu der Seqzuenz von SEQ ID NO. 1 liegen, werden geeigneterweise in Form der DNA in einen geeigneten Expressionsvektor ligiert. Dieser wird in die Wirtszelle transformiert, beispielsweise in Zellen eines leicht zu kultivierenden Bakterienstammes, der die Proteine, deren Gene unter der Kontrolle entsprechender genetischer Elemente stehen, in das umgebende Nährmedium ausschleust; regulierende Elemente dafür können beispielsweise vom Expressionsvektor zur Verfügung gestellt werden. Aus dem umgebenden Medium kann das erfindungsgemäße Protein über mehrere Aufreinigungsschritte, wie beispielsweise Fällungen oder Chromatographien, aufgreinigt werden. Ein Fachmann ist in der Lage, ein System, welches im Labormaßstab experimentell optimiert worden ist, auf einen großtechnischen Produktionsmaßstab zu übertragen.

Im folgenden werden die wichtigsten technischen Verwendungsmöglichkeiten für erfindungsgemäße Proteine ausgeführt. Zahlreiche in der Technik etablierte Anwendungsmöglichkeiten für amylolytische Enzyme werden in Handbüchem, wie beispielsweise dem Buch "Industrial enyzmes and their applications" von H. Uhlig, Wiley-Verlag, New York, 1998, ausgeführt. Folgende Zusammenstellung ist nicht als abschließende Aufzählung zu verstehen, sondem stellt eine Auswahl aus allen theoretisch denkbaren Verwendungsmöglichkeiten dar. Sollte sich herausstellen, daß einzelne erfindungsgemäße Proteine für zusätzliche, hier nicht ausdrücklich beanspruchte Anwendungsmöglichkeiten geeignet sind, so werden diese hiermit in den Schutzbereich der vorliegenden Erfindung eingeschlossen.

Ein wichtiges Einsatzgebiet für amylolytische Enzyme ist ihre Verwendung als aktive Komponenten in Wasch- oder Reinigungsmitteln zur Reinigung von Textilien oder von festen Oberflächen. In diesen Anwendungen dient die amylolytische Aktivität dazu, kohlenhydrathaltige, insbesondere stärkeähnliche Verunreinigungen hydrolytisch aufzulösen und/oder vom Untergrund abzulösen. Dabei können sie allein, in geeigneten Medien oder auch in Wasch- oder Reinigungsmitteln zur Anwendung gebracht werden. Diese Mittel zeichnen sich dadurch aus, daß die amylolytischen Enzyme und die übrigen Komponenten synergistisch die Beseitigung der Verunreinigungen bewirken, beispielsweise indem die Hydrolyseprodukte der amylolytischen Proteine durch andere Bestandteile der Mittel wie etwa Tenside solubilisiert werden. Ein erfindungsgemäßes Protein kann sowohl in Mitteln für Großverbraucher oder technische Anwender als auch in Produkten für den Privatverbraucher Anwendung finden.

Einen eigenen Erfindungsgegenstand stellen somit alle Waschmittel oder Reinigungsmittel dar, die dadurch gekennzeichnet sind, daß sie ein erfindungsgemäßes amylolytisches Protein oder Derivat enthalten.

Damit sind alle denkbaren Reinigungsmittelarten gemeint, sowohl Konzentrate, als auch unverdünnt anzuwendende Mittel; zum Einsatz im kommerziellen Maßstab, in der Waschmaschine oder bei der Hand-Wäsche, beziehungsweise -Reinigung. Dazu gehören beispielsweise Waschmittel für Textilien, Teppiche, oder Naturfasern, für die nach der vorliegenden Erfindung die Bezeichnung Waschmittel verwendet wird. Dazu gehören beispielsweise auch Geschirrspülmittel für Geschirrspülmaschinen oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder; für solche wird nach der vorliegenden Erfindung die Bezeichnung Reinigungsmittel verwendet. Jegliche Reinigungsmittelart stellt eine Ausführungsform der vorliegenden Erfindung dar, sofern sie um ein erfindungsgemäßes Protein bereichert ist.

Ausführungsformen der vorliegenden Erfindung umfassen alle nach den Stand der Technik etablierten und/oder alle zweckmäßigen Darreichungsformen der erfindungsgemäßen Mittel. Dazu zählen beispielsweise feste, pulverförmige, flüssige, gelförmige oder pastöse Mittel, gegebenenfalls auch aus mehreren Phasen, komprimiert oder nicht komprimiert; ferner gehören beispielsweise dazu: Extrudate, Granulate, Tabletten oder Pouches, sowohl in Großgebinden als auch portionsweise abgepackt.

Neben einem erfindungswesentlichen Enzym enthält das erfindungsgemäße Mittel gegebenenfalls weitere Inhaltsstoffe wie Tenside, zum Beispiel nichtionische, anionische und/oder amphotere Tenside, und/oder Bleichmittel, und/oder Builder, sowie gegebenenfalls weitere übliche Inhaltsstoffe.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, indenen der Alkoholrest linear oder bevorzugt in 2-Stellung methylverzweigt sein kann, beziehungsweise lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂₋₁₄-Alkohole mit 3 EO oder 4 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 5 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester.

Eine weitere Klasse von nichtionischen Tensiden, die vorteilhafterweise eingesetzt werden können, sind die Alkylpolyglycoside (APG). Einsetzbare Alkypolyglycoside genügen der allgemeinen Formel RO(G)_{z}, in der R für einen linearen oder verzweigten, insbesondere in 2-Stellung methylverzweigten, gesättigten oder ungesättigten, aliphatischen Rest mit 8 bis 22, vorzugsweise 12 bis 18 C-Atomen bedeutet und G das Symbol ist, das für eine Glykoseeinheit mit 5 oder 6 C-Atomen, vorzugsweise für Glucose, steht. Der Glycosylierungsgrad z liegt dabei zwischen 1,0 und 4,0, vorzugsweise zwischen 1,0 und 2,0 und insbesondere zwischen 1,1 und 1,4. Bevorzugt eingesetzt werden lineare Alkylpolyglucoside, also Alkylpolyglycoside, in denen der Polyglycosylrest ein Glucoserest und der Alkylrest ein n-Alkylrest ist.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Der Anteil dieser nichtionischen Tenside liegt vorzugsweise nicht über dem der ethoxylierten Fettalkohole, insbesondere bei nicht mehr als der Hälfte davon.

Weitere geeignete Tenside sind Polyhydroxyfettsäureamide der Formel (II), 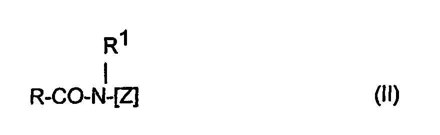 in der RCO für einen aliphatischen Acylrest mit 6 bis 22 Kohlenstoffatomen, R¹ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.

Zur Gruppe der Polyhydroxyfettsäureamide gehören auch Verbindungen der Formel (III), 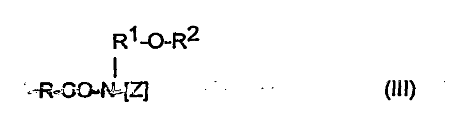 in der R für einen linearen oder verzweigten Alkyl- oder Alkenylrest mit 7 bis 12 Kohlenstoffatomen, R¹ für einen linearen, verzweigten oder cyclischen Alkylrest oder einen Arylrest mit 2 bis 8 Kohlenstoffatomen und R² für einen linearen, verzweigten oder cyclischen Alkylrest oder einen Arylrest oder einen Oxy-Alkylrest mit 1 bis 8 Kohlenstoffatomen steht, wobei C₁₋₄-Alkyl- oder Phenylreste bevorzugt sind und [Z] für einen linearen Polyhydroxyalkylrest steht, dessen Alkylkette mit mindestens zwei Hydroxylgruppen substituiert ist, oder alkoxylierte, vorzugsweise ethoxylierte oder propoxylierte Derivate dieses Restes.

[Z] wird vorzugsweise durch reduktive Aminierung eines reduzierenden Zuckers erhalten, beispielsweise Glucose, Fructose, Maltose, Lactose, Galactose, Mannose oder Xylose. Die N-Alkoxy- oder N-Aryloxy-substituierten Verbindungen können beispielsweise durch Umsetzung mit Fettsäuremethylestern in Gegenwart eines Alkoxids als Katalysator in die gewünschten Polyhydroxyfettsäureamide überführt werden.

Als anionische Tenside werden beispielsweise solche vom Typ der Sulfonate und Sulfate eingesetzt. Als Tenside vom Sulfonat-Typ kommen dabei vorzugsweise C₉₋₁₃-Alkylbenzolsulfonate, Olefinsulfonate, das heißt Gemische aus Alken- und Hydroxyalkansulfonaten sowie Disulfonaten, wie man sie beispielsweise aus C₁₂₋₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus C₁₂₋₁₈-Alkanen beispielsweise durch Sulfochlorierung oder Sulfoxidation mit anschließender Hydrolyse beziehungsweise Neutralisation gewonnen werden. Ebenso sind auch die Ester von α-Sulfofettsäuren (Estersulfonate), zum Beispiel die α-sulfonierten Methylester der hydrierten Kokos-, Palmkern- oder Talgfettsäuren geeignet.

Weitere geeignete Aniontenside sind sulfierte Fettsäureglycerinester. Unter Fettsäureglycerinestem sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C₁₂-C₁₆-Alkylsulfate und C₁₂-C₁₅-Alkylsulfate sowie C₁₄-C₁₅-Alkylsulfate bevorzugt. Auch 2,3-Alkylsulfate sind geeignete Aniontenside.

Auch die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO, sind geeignet. Sie werden in Reinigungsmitteln aufgrund ihres hohen Schaumverhaltens nur in relativ geringen Mengen, beispielsweise in Mengen bis 5 Gew.-%, üblicherweise von 1 bis 5 Gew.-%, eingesetzt.

Weitere geeignete Aniontenside sind auch die Salze der Alkylsulfobemsteinsäure, die auch als Sulfosuccinate oder als Sulfobemsteinsäureester bezeichnet werden und die Monoester und/oder Diester der Sulfobemsteinsäure mit Alkoholen, vorzugsweise Fettalkoholen und insbesondere ethoxylierten Fettalkoholen darstellen. Bevorzugte Sulfosuccinate enthalten C₈₋₁₈-Fettalkoholreste oder Mischungen aus diesen. Insbesondere bevorzugte Sulfosuccinate enthalten einen Fettalkoholrest, der sich von ethoxylierten Fettalkoholen ableitet, die für sich betrachtet nichtionische Tenside darstellen (Beschreibung siehe unten). Dabei sind wiederum Sulfosuccinate, deren Fettalkohol-Reste sich von ethoxylierten Fettalkoholen mit eingeengter Homologenverteilung ableiten, besonders bevorzugt. Ebenso ist es auch möglich, Alk(en)ylbemsteinsäure mit vorzugsweise 8 bis 18 Kohlenstoffatomen in der Alk(en)ylkette oder deren Salze einzusetzen.

Als weitere anionische Tenside kommen insbesondere Seifen in Betracht. Geeignet sind gesättigte Fettsäureseifen, wie die Salze der Laurinsäure, Myristinsäure, Palmitinsäure, Stearinsäure, hydrierte Erucasäure und Behensäure sowie insbesondere aus natürlichen Fettsäuren, zum Beispiel Kokos-, Palmkern- oder Talgfettsäuren, abgeleitete Seifengemische.

Die anionischen Tenside einschließlich der Seifen können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze vor.

Die Tenside können in den erfindungsgemäßen Reinigungs- oder Waschmitteln insgesamt in einer Menge von vorzugsweise 5 Gew.-% bis 50 Gew.-%, insbesondere von 8 Gew.-% bis 30 Gew.-%, bezogen auf das fertige Mittel, enthalten sein.

Erfindungsgemäße Mittel können Bleichmittel enthalten. Unter den als Bleichmittel dienenden, in Wasser H₂O₂ liefemden Verbindungen haben das Natriumpercarbonat, das Natriumperborattetrahydrat und das Natriumperboratmonohydrat besondere Bedeutung. Weitere brauchbare Bleichmittel sind beispielsweise Peroxopyrophosphate, Citratperhydrate sowie H₂O₂ liefernde persaure Salze oder Persäuren, wie Persulfate beziehungsweise Perschwefelsäure. Brauchbar ist auch das Hamstoffperoxohydrat Percarbamid, das durch die Formel H₂N-CO-NH₂·H₂O₂ beschrieben werden kann. Insbesondere beim Einsatz der Mittel für das Reinigen harter Oberflächen, zum Beispiel beim maschinellen Geschirrspülen, können sie gewünschtenfalls auch Bleichmittel aus der Gruppe der organischen Bleichmittel enthalten, obwohl deren Einsatz prinzipiell auch bei Mitteln für die Textilwäsche möglich ist. Typische organische Bleichmittel sind die Diacylperoxide, wie zum Beispiel Dibenzoylperoxid. Weitere typische organische Bleichmittel sind die Peroxysäuren, wobei als Beispiele besonders die Alkylperoxysäuren und die Arylperoxysäuren genannt werden. Bevorzugte Vertreter sind die Peroxybenzoesäure und ihre ringsubstituierten Derivate, wie Alkylperoxybenzoesäuren, aber auch Peroxy-α-Naphthoesäure und Magnesium-monoperphthalat, die aliphatischen oder substituiert aliphatischen Peroxysäuren, wie Peroxylaurinsäure, Reroxystearinsäure, ε-Phthalimidoperoxycapronsäure (Phthalimidoperoxyhexansäure, PAP), o-Carboxybenzamidoperoxycapronsäure, N-Nonenylamidoperadipinsäure und N-Nonenylamidopersuccinate, und aliphatische und araliphatische Peroxydicarbonsäuren, wie 1,12-Diperoxycarbonsäure, 1,9-Diperoxyazelainsäure, Diperoxysebacinsäure, Diperoxybrassylsäure, die Diperoxyphthalsäuren, 2-Decyldiperoxybutan-1,4-disäure, N,N-Terephthaloyl-di(6-aminopercapronsäure) können eingesetzt werden.

Der Gehalt der Mittel an Bleichmittel kann 1 bis 40 Gew.-% und insbesondere 10 bis 20 Gew.-%, betragen, wobei vorteilhafterweise Perboratmonohydrat oder Percarbonat eingesetzt wird. Eine synergistische Verwendung von Amylase mit Percarbonat oder von Amylase mit Percarbonsäure wird mit den Anmeldungen WO 99/63036, beziehungsweise WO 99/63037 offenbart.

Um beim Waschen bei Temperaturen von 60°C und darunter, und insbesondere bei der Wäschevorbehandlung eine verbesserte Bleichwirkung zu erreichen, können die Mittel auch Bleichaktivatoren enthalten. Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glycolurile, insbesondere 1,3,4,6-Tetraacetylglycoluril (TAGU), N-Acylimide, insbesondere N-Nonanoylsuccinimid (NOSI), acylierte Phenolsulfonate, insbesondere *n*-Nonanoyl- oder Isononanoyloxybenzolsulfonat (*n*- beziehungsweise iso-NOBS), acylierte Hydroxycarbonsäuren, wie Triethyl-O-acetylcitrat (TEOC), Carbonsäureanhydride, insbesondere Phthalsäureanhydrid, Isatosäureanhydrid und/oder Bemsteinsäureanhydrid, Carbonsäureamide, wie N-Methyldiacetamid, Glycolid, acylierte mehrwertige Alkohole, insbesondere Triacetin, Ethylenglycoldiacetat, Isopropenylacetat, 2,5-Diacetoxy-2,5-dihydrofuran und die aus den deutschen Patentanmeldungen DE 196 16 693 und DE 196 16 767 bekannten Enolester sowie acetyliertes Sorbitol und Mannitol beziehungsweise'deren in der europäischen Patentanmeldung EP 0 525239 beschriebene Mischungen (SORMAN), acylierte Zuckerderivate, insbesondere Pentaacetylglucose (PAG), Pentaacetylfructose, Tetraacetylxylose und Octaacetyllactose sowie acetyliertes, gegebenenfalls N-alkyliertes Glucamin beziehungsweise Gluconolacton, Triazol beziehungsweise Triazolderivate und/oder teilchenförmige Caprolactame und/oder Caprolactamderivate, bevorzugt N-acylierte Lactame, beispielsweise N-Benzoylcaprolactam und N-Acetylcaprolactam, die aus den internationalen Patentanmeldungen WO 94/27970, WO 94/28102, WO 94/28103, WO 95/00626, WO 95/14759 und WO 95/17498 bekannt sind. Die aus der deutschen Patentanmeldung DE 196 16 769 bekannten hydrophil substituierten Acylacetale und die in der deutschen Patentanmeldung DE 196 16 770 sowie der internationalen Patentanmeldung WO 95/14075 beschriebenen Acyllactame werden ebenfalls bevorzugt eingesetzt. Auch die aus der deutschen Patentanmeldung DE 4443 177 bekannten Kombinationen konventioneller Bleichaktivatoren können eingesetzt werden. Ebenso können Nitrilderivate wie Cyanopyridine, Nitrilquats, zum Beispiel N-Alkyammoniumacetonitrile, und/oder Cyanamidderivate eingesetzt werden. Bevorzugte Bleichaktivatoren sind Natrium-4-(octanoyloxy)-benzolsulfonat, *n*-Nonanoyl- oder Isononanoyloxybenzolsulfonat (*n*- beziehungsweise iso-NOBS), Undecenoyloxybenzolsulfonat (UDOBS), Natriumdodecanoyloxybenzolsulfonat (DOBS), Decanoyloxybenzoesäure (DOBA, OBC 10) und/oder Dodecanoyloxybenzolsulfonat (OBS 12), sowie N-Methylmorpholinum-acetonitril (MMA). Derartige Bleichaktivatoren können im üblichen Mengenbereich von 0,01 bis 20 Gew.-%, vorzugsweise in Mengen von 0,1 bis 15 Gew.-%, insbesondere 1 Gew.-% bis 10 Gew.-%, bezogen auf die gesamte Zusammensetzung, enthalten sein.

Zusätzlich zu den konventionellen Bleichaktivatoren oder an deren Stelle können auch sogenannte Bleichkatalysatoren enthalten sein. Bei diesen Stoffen handelt es sich um bleichverstärkende Übergangsmetallsalze beziehungsweise Übergangsmetallkomplexe wie beispielsweise Mn-, Fe-, Co-, Ru - oder Mo-Salenkomplexe oder -carbonylkomplexe. Auch Mn-, Fe-, Co-, Ru-, Mo-, Ti-, V- und Cu-Komplexe mit N-haltigen Tripod-Liganden sowie Co-, Fe-, Cu- und Ru-Aminkomplexe sind als Bleichkatalysatoren geeignet, wobei solche Verbindungen bevorzugt eingesetzt werden, die in der DE 197 09 284 A1 beschrieben sind. Gemäß WO 99/63038 vermögen auch Acetonitril-Derivate und gemäß WO 99/63041 bleichaktivierende Übergangsmetallkomplexverbindungen in Kombination mit Amylasen eine bleichaktivierende Wirkung zu entfalten.

Erfindungsgemäße Mittel enthalten in der Regel einen oder mehrere Builder, insbesondere Zeolithe, Silikate, Carbonate, organische Cobuilder und - wo keine ökologischen Gründe gegen ihren Einsatz sprechen - auch die Phosphate. Letztere sind insbesondere in Reinigungsmitteln für das maschinelle Geschirrspülen bevorzugt einzusetzende Gerüststoffe.

Zu nennen sind hier kristalline, schichtförmige Natriumsilicate der allgemeinen Formel NaMSiₓO₂ₓ₊₁·yH₂O, wobei M Natrium oder Wasserstoff bedeutet, x eine Zahl von 1,6 bis 4, vorzugsweise 1,9 bis 4,0 und y eine Zahl von 0 bis 20 ist und bevorzugte Werte für x 2, 3 oder 4 sind. Derartige kristalline Schichtsilicate werden beispielsweise in der europäischen Patentanmeldung EP 0164 514 beschrieben. Bevorzugte kristalline Schichtsilicate der angegebenen Formel sind solche, in denen M für Natrium steht und x die Werte 2 oder 3 annimmt. Insbesondere sind sowohl β- als auch δ-Natriumdisilicate Na₂Si₂O₅·yH₂O bevorzugt. Im Handel befinden sich derartige Verbindungen beispielsweise unter der Bezeichnung SKS® (Fa. Clariant). So handelt es sich bei SKS-6® vorwiegend um ein δ-Natriumdisilicat mit der Formel Na₂Si₂O₅·yH₂O, bei SKS-7® vorwiegend um das β-Natriumdisilicat. Durch Reaktion mit Säuren (zum Beispiel Citronensäure oder Kohlensäure) entsteht aus dem δ-Natriumdisilicat Kanemit NaHSi₂O₅·yH₂O, im Handel unter den Bezeichnungen SKS-9® beziehungsweise SKS-10® (Fa. Clariant). Von Vorteil kann es auch sein, chemische Modifikationen dieser Schichtsilicate einzusetzen. So kann beispielsweise die Alkalität der Schichtsilicate geeignet beeinflußt werden. Mit Phosphat beziehungsweise mit Carbonat dotierte Schichtsilicate weisen im Vergleich zu dem δ-Natriumdisilicat veränderte Kristallmorphologien auf, lösen sich schneller und zeigen im Vergleich zu δ-Natriumdisilicat ein erhöhtes Calciumbindevermögen. So sind Schichtsilicate der allgemeinen Summenformel x Na₂O • y SiO₂ • z P$\frac{\text{-}}{\text{2}}$O₅, in der das Verhältnis x zu y einer Zahl 0,35 bis 0,6, das Verhältnis x zu z einer Zahl von 1,75 bis 1200 und das Verhältnis y zu z einer Zahl von 4 bis 2800 entsprechen, in der Patentanmeldung DE 19601 063 beschrieben. Die Löslichkeit der Schichtsilicate kann auch erhöht werden, indem besonders feinteilige Schichtsilicate eingesetzt werden. Auch Compounds aus den kristallinen Schichtsilicaten mit anderen Inhaltsstoffen können eingesetzt werden. Dabei sind insbesondere Compounds mit Cellulosederivaten, die Vorteile in der desintegrierenden Wirkung aufweisen und insbesondere in Waschmitteltabletten eingesetzt werden, sowie Compounds mit Polycarboxylaten, zum Beispiel Citronensäure, beziehungsweise polymeren Polycarboxylaten, zum Beispiel Copolymeren der Acrylsäure, zu nennen.

Einsetzbar sind auch amorphe Natriumsilikate mit einem Modul Na₂O : SiO₂ von 1:2 bis 1:3,3, vorzugsweise von 1:2 bis 1:2,8 und insbesondere von 1:2 bis 1:2,6, welche löseverzögert sind und Sekundärwascheigenschaften aufweisen. Die Löseverzögerung gegenüber herkömmlichen amorphen Natriumsilikaten kann dabei auf verschiedene Weise, beispielsweise durch Oberflächenbehandlung, Compoundierung, Kompaktierung/Verdichtung oder durch Übertrocknung hervorgerufen worden sein. Im Rahmen dieser Erfindung wird unter dem Begriff "amorph" auch "röntgenamorph" verstanden. Dies heißt, daß die Silikate bei Röntgenbeugungsexperimenten keine scharfen Röntgenreflexe liefern, wie sie für kristalline Substanzen typisch sind, sondern allenfalls ein oder mehrere Maxima der gestreuten Röntgenstrahlung, die eine Breite von mehreren Gradeinheiten des Beugungswinkels aufweisen. Es kann jedoch sehr wohl sogar zu besonders guten Buildereigenschaften führen, wenn die Silikatpartikel bei Elektronenbeugungsexperimenten verwaschene oder sogar scharfe Beugungsmaxima liefem. Dies ist so zu interpretieren, daß die Produkte mikrokristalline Bereiche der Größe 10 bis einige Hundert nm aufweisen, wobei Werte bis max. 50 nm und insbesondere bis max. 20 nm bevorzugt sind. Insbesondere bevorzugt sind verdichtete/kompaktierte amorphe Silikate, compoundierte amorphe Silikate und übertrocknete röntgenamorphe Silikate.

Ein gegebenenfalls einsetzbarer, feinkristalliner, synthetischer und gebundenes Wasser enthaltender Zeolith ist vorzugsweise Zeolith A und/oder P. Als Zeolith P wird Zeolith MAP® (Handelsprodukt der Firma Crosfield) besonders bevorzugt. Geeignet sind jedoch auch Zeolith X sowie Mischungen aus A, X und/oder P. Kommerziell erhältlich und im Rahmen der vorliegenden Erfindung bevorzugt einsetzbar ist beispielsweise auch ein Co-Kristallisat aus Zeolith X und Zeolith A (ca. 80 Gew.-% Zeolith X), das von der Firma CONDEA Augusta S.p.A. unter dem Markennamen VEGOBOND AX® vertrieben wird und durch die Formel

nNa₂O · (1-n)K₂O · Al₂O₃ · (2 - 2,5)SiO₂ · (3,5 - 5,5) H₂O

beschrieben werden kann. Geeignete Zeolithe weisen eine mittlere Teilchengröße von weniger als 10 µm (Volumenverteilung; Meßmethode: Coulter Counter) auf und enthalten vorzugsweise 18 bis 22 Gew.-%, insbesondere 20 bis 22 Gew.-% an gebundenem Wasser.

Selbstverständlich ist auch ein Einsatz der allgemein bekannten Phosphate als Buildersubstanzen möglich, sofern ein derartiger Einsatz nicht aus ökologischen Gründen vermieden werden sollte. Unter der Vielzahl der kommerziell erhältlichen Phosphate haben die Alkalimetallphosphate unter besonderer Bevorzugung von Pentanatrium- beziehungsweise Pentakaliumtriphosphat (Natrium- beziehungsweise Kaliumtripolyphosphat) in der Wasch- und Reinigungsmittel-Industrie die größte Bedeutung.

Alkalimetallphosphate ist dabei die summarische Bezeichnung für die Alkalimetall-(insbesondere Natrium- und Kalium-) -Salze der verschiedenen Phosphorsäuren, bei denen man Metaphosphorsäuren (HPO₃)ₙ und Orthophosphorsäure H₃PO₄ neben höhermolekularen Vertretern unterscheiden kann. Die Phosphate vereinen dabei mehrere Vorteile in sich: Sie wirken als Alkaliträger, verhindern Kalkbeläge auf Maschinenteilen beziehungsweise Kalkinkrustationen in Geweben und tragen überdies zur Reinigungsleistung bei.

Natriumdihydrogenphosphat, NaH₂PO₄, existiert als Dihydrat (Dichte 1,91 gcm⁻³, Schmelzpunkt 60°) und als Monohydrat (Dichte 2,04 gcm⁻³). Beide Salze sind weiße, in Wasser sehr leicht lösliche Pulver, die beim Erhitzen das Kristallwasser verlieren und bei 200°C in das schwach saure Diphosphat (Dinatriumhydrogendiphosphat, Na₂H₂P₂O₇), bei höherer Temperatur in Natiumtrimetaphosphat (Na₃P₃O₉) und Maddrellsches Salz (siehe unten), übergehen. NaH₂PO₄ reagiert sauer, es entsteht, wenn Phosphorsäure mit Natronlauge auf einen pH-Wert von 4,5 eingestellt und die Maische versprüht wird. Kaliumdihydrogenphosphat (primäres oder einbasiges Kaliumphosphat, Kaliumbiphosphat, KDP), KH₂PO₄, ist ein weißes Salz der Dichte 2,33 gcm⁻³, hat einen Schmelzpunkt 253° [Zersetzung unter Bildung von Kaliumpolyphosphat (KPO₃)ₓ] und ist leicht löslich in Wasser.

Dinatriumhydrogenphosphat (sekundäres Natriumphosphat), Na₂HPO₄, ist ein farbloses, sehr leicht wasserlösliches kristallines Salz. Es existiert wasserfrei und mit 2 Mol. (Dichte 2,066 gcm⁻³, Wasserverlust bei 95°), 7 Mol. (Dichte 1,68 gcm⁻³, Schmelzpunkt 48° unter Verlust von 5 H₂O) und 12 Mol. Wasser (Dichte 1,52 gcm⁻³, Schmelzpunkt 35° unter Verlust von 5 H₂O), wird bei 100° wasserfrei und geht bei stärkerem Erhitzen in das Diphosphat Na₄P₂O₇ über. Dinatriumhydrogenphosphat wird durch Neutralisation von Phosphorsäure mit Sodalösung unter Verwendung von Phenolphthalein als Indikator hergestellt. Dikaliumhydrogenphosphat (sekundäres od. zweibasiges Kaliumphosphat), K₂HPO₄, ist ein amorphes, weißes Salz, das in Wasser leicht löslich ist.

Trinatriumphosphat, tertiäres Natriumphosphat, Na₃PO₄, sind farblose Kristalle, die als Dodecahydrat eine Dichte von 1,62 gcm⁻³ und einen Schmelzpunkt von 73-76°C (Zersetzung), als Decahydrat (entsprechend 19-20% P₂O₅) einen Schmelzpunkt von 100°C und in wasserfreier Form (entsprechend 39-40% P₂O₅) eine Dichte von 2,536 gcm⁻³ aufweisen. Trinatriumphosphat ist in Wasser unter alkalischer Reaktion leicht löslich und wird durch Eindampfen einer Lösung aus genau 1 Mol Dinatriumphosphat und 1 Mol NaOH hergestellt. Trikaliumphosphat (tertiäres oder dreibasiges Kaliumphosphat), K₃PO₄, ist ein weißes, zerfließliches, kömiges Pulver der Dichte 2,56 gcm⁻³, hat einen Schmelzpunkt von 1340° und ist in Wasser mit alkalischer Reaktion leicht löslich. Es entsteht zum Beispiel beim Erhitzen von Thomasschlacke mit Kohle und Kaliumsulfat. Trotz des höheren Preises werden in der Reinigungsmittel-Industrie die leichter löslichen, daher hochwirksamen, Kaliumphosphate gegenüber entsprechenden Natrium-Verbindungen vielfach bevorzugt.

Tetranatriumdiphosphat (Natriumpyrophosphat), Na₄P₂O₇, existiert in wasserfreier Form (Dichte 2,534 gcm⁻³, Schmelzpunkt 988°, auch 880° angegeben) und als Decahydrat (Dichte 1,815-1,836 gcm⁻³, Schmelzpunkt 94° unter Wasserverlust). Beide Substanzen sind farblose, in Wasser mit alkalischer Reaktion lösliche Kristalle. Na₄P₂O₇ entsteht beim Erhitzen von Dinatriumphosphat auf >200° oder indem man Phosphorsäure mit Soda im stöchiometrischem Verhältnis umsetzt und die Lösung durch Versprühen entwässert. Das Decahydrat komplexiert Schwermetall-Salze und Härtebildner und verringert daher die Härte des Wassers. Kaliumdiphosphat (Kaliumpyrophosphat), K₄P₂O₇, existiert in Form des Trihydrats und stellt ein farbloses, hygroskopisches Pulver mit der Dichte 2,33 gcm⁻³ dar, das in Wasser löslich ist, wobei der pH-Wert der 1%igen Lösung bei 25° 10,4 beträgt.

Durch Kondensation des NaH₂PO₄ beziehungsweise des KH₂PO₄ entstehen höhermolekulare Natrium- und Kaliumphosphate, bei denen man cyclische Vertreter, die Natrium- beziehungsweise Kaliummetaphosphate und kettenförmige Typen, die Natriumbeziehungsweise Kaliumpolyphosphate, unterscheiden kann. Insbesondere für letztere sind eine Vielzahl von Bezeichnungen in Gebrauch: Schmelz- oder Glühphosphate, Grahamsches Salz, Kurrolsches und Maddrellsches Salz. Alle höheren Natrium- und Kaliumphosphate werden gemeinsam als kondensierte Phosphate bezeichnet.

Das technisch wichtige Pentanatriumtriphosphat, Na₅P₃O₁₀ (Natriumtripolyphosphat), ist ein wasserfrei oder mit 6 H₂O kristallisierendes, nicht hygroskopisches, weißes, wasserlösliches Salz der allgemeinen Formel NaO-[P(O)(ONa)-O]ₙ-Na mit n=3. In 100 g Wasser lösen sich bei Zimmertemperatur etwa 17 g, bei 60° ca. 20 g, bei 100° rund 32 g des kristallwasserfreien Salzes; nach zweistündigem Erhitzen der Lösung auf 100° entstehen durch Hydrolyse etwa 8% Orthophosphat und 15% Diphosphat. Bei der Herstellung von Pentanatriumtriphosphat wird Phosphorsäure mit Sodalösung oder Natronlauge im stöchiometrischen Verhältnis zur Reaktion gebracht und die Lösung durch Versprühen entwässert. Ähnlich wie Grahamsches Salz und Natriumdiphosphat löst Pentanatriumtriphosphat viele unlösliche Metall-Verbindungen (auch Kalkseifen usw.). Pentakaliumtriphosphat, K₅P₃O₁₀ (Kaliumtripolyphosphat), kommt beispielsweise in Form einer 50 Gew.-%-igen Lösung (> 23% P₂O₅, 25% K₂O) in den Handel. Die Kaliumpolyphosphate finden in der Wasch- und Reinigungsmittel-Industrie breite Verwendung. Weiter existieren auch Natriumkaliumtripolyphosphate, welche ebenfalls im Rahmen der vorliegenden Erfindung einsetzbar sind. Diese entstehen beispielsweise, wenn man Natriumtrimetaphosphat mit KOH hydrolysiert:

(NaPO₃)₃ + 2 KOH → Na₃K₂P₃O₁₀ + H₂O

Diese sind erfindungsgemäß genau wie Natriumtripolyphosphat, Kaliumtripolyphosphat oder Mischungen aus diesen beiden einsetzbar; auch Mischungen aus Natriumtripolyphosphat und Natriumkaliumtripolyphosphat oder Mischungen aus Kaliumtripolyphosphat und Natriumkaliumtripolyphosphat oder Gemische aus Natriumtripolyphosphat und Kaliumtripolyphosphat und Natriumkaliumtripolyphosphat sind erfindungsgemäß einsetzbar.

Als organische Cobuilder können in den erfindungsgemäßen Wasch- und Reinigungsmitteln insbesondere Polycarboxylate oder Polycarbonsäuren, polymere Polycarboxylate, Polyasparaginsäure, Polyacetale, gegebenenfalls oxidierte Dextrine, weitere organische Cobuilder (siehe unten) sowie Phosphonate eingesetzt werden. Diese Stoffklassen werden nachfolgend beschrieben.

Brauchbare organische Gerüstsubstanzen sind beispielsweise die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies. Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), sofern ein derartiger Einsatz aus ökologischen Gründen nicht zu vermeiden ist, sowie Mischungen aus diesen. Bevorzugte Salze sind die Salze der Polycarbonsäuren wie Citronensäure, Adipinsäure, Bemsteinsäure, Glutarsäure, Weinsäure, Zuckersäuren und Mischungen aus diesen.

Auch die Säuren an sich können eingesetzt werden. Sie besitzen neben ihrer Builderwirkung typischerweise auch die Eigenschaft einer Säuerungskomponente und dienen somit auch zur Einstellung eines niedrigeren und milderen pH-Wertes von Wasch- oder Reinigungsmitteln, sofern nicht der sich durch die Mischung der übrigen Komponenten ergebende pH-Wert gewünscht ist. Insbesondere sind hierbei systemunbd umweltverträgliche Säuren wie Citronensäure, Essigsäure, Weinsäure, Äpfelsäure, Milchsäure, Glykolsäure, Bernsteinsäure, Glutarsäure, Adipinsäure, Gluconsäure und beliebige Mischungen aus diesen zu nennen. Aber auch Mineralsäuren, insbesondere Schwefelsäure oder Basen, insbesondere Ammonium- oder Alkalihydroxide können als pH-Regulatoren dienen. Derartige Regulatoren sind in den erfindungemäßen Mitteln in Mengen von vorzugsweise nicht über 20 Gew.-%, insbesondere von 1,2 Gew.-% bis 17 Gew.-%, enthalten.

Als Builder sind weiter polymere Polycarboxylate geeignet, dies sind beispielsweise die Alkalimetallsalze der Polyacrylsäure oder der Polymethacrylsäure, beispielsweise solche mit einer relativen Molekülmasse von 500 bis 70 000 g/mol.

Bei den für polymere Polycarboxylate angegebenen Molmassen handelt es sich im Sinne dieser Schrift um gewichtsmittlere Molmassen M_{w} der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsäure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsulfonsäuren gemessenen Molmassen sind in der Regel deutlich höher als die in dieser Schrift angegebenen Molmassen.

Geeignete Polymere sind insbesondere Polyacrylate, die bevorzugt eine Molekülmasse von 2 000 bis 20 000 g/mol aufweisen. Aufgrund ihrer überiegenen Löslichkeit können aus dieser Gruppe wiederum die kurzkettigen Polyacrylate, die Molmassen von 2 000 bis 10 000 g/mol, und besonders bevorzugt von 3 000 bis 5 000g/mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im allgemeinen 2 000 bis 70 000 g/mol, vorzugsweise 20 000 bis 50 000 g/mol und insbesondere 30 000 bis 40 000 g/mol. Die (co-) polymeren Polycarboxylate können entweder als Pulver oder als wässerige Lösung eingesetzt werden. Der Gehalt der Mittel an (co-)polymeren Polycarboxylaten kann von 0,5 bis 20 Gew.-%, insbesondere 1 bis 10 Gew.-%, betragen.

Zur Verbesserung der Wasserlöslichkeit können die Polymere auch Allylsulfonsäuren, wie beispielsweise Allyloxybenzolsulfonsäure und Methallylsulfonsäure, als Monomer enthalten.

Insbesondere bevorzugt sind auch biologisch abbaubare Polymere aus mehr als zwei verschiedenen Monomereinheiten, beispielsweise solche, die als Monomere Salze der Acrylsäure und der Maleinsäure sowie Vinylalkohol beziehungsweise Vinylalkohol-Derivate oder die als Monomere Salze der Acrylsäure und der 2-Alkylallylsulfonsäure sowie Zucker-Derivate enthalten.

Weitere bevorzugte Copolymere sind solche, die als Monomere vorzugsweise Acrolein und Acrylsäure/Acrylsäuresalze beziehungsweise Acrolein und Vinylacetat aufweisen.

Ebenso sind als weitere bevorzugte Buildersubstanzen polymere Aminodicarbonsäuren, deren Salze oder deren Vorläufersubstanzen zu nennen. Besonders bevorzugt sind Polyasparaginsäuren beziehungsweise deren Salze und Derivate.

Weitere geeignete Buildersubstanzen sind Polyacetale, welche durch Umsetzung von Dialdehyden mit Polyolcarbonsäuren, welche 5 bis 7 C-Atome und mindestens 3 Hydroxylgruppen aufweisen, erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Weitere geeignete organische Buildersubstanzen sind Dextrine, beispielsweise Oligomere beziehungsweise Polymere von Kohlenhydraten, die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 bis 500 000 g/mol. Dabei ist ein Polysaccharid mit einem Dextrose-Äquivalent (DE) im Bereich von 0,5 bis 40, insbesondere von 2 bis 30 bevorzugt, wobei DE ein gebräuchliches Maß für die reduzierende Wirkung eines Polysaccharids im Vergleich zu Dextrose ist, welche ein DE von 100 besitzt. Brauchbar sind sowohl Maltodextrine mit einem DE zwischen 3 und 20 und Trockenglucosesirupe mit einem DE zwischen 20 und 37 als auch sogenannte Gelbdextrine und Weißdextrine mit höheren Molmassen im Bereich von 2 000 bis 30 000 g/mol.

Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren. Besonders bevorzugte organische Builder für erfindungsgemäße Mittel sind oxidierte Stärken, beziehungsweise deren Derivate aus den Anmeldungen EP 472 042, WO 97/25399, und EP 755 944.

Auch Oxydisuccinate und andere Derivate von Disuccinaten, vorzugsweise Ethylendiamindisuccinat, sind weitere geeignete Cobuilder. Dabei wird Ethylendiamin-N,N'-disuccinat (EDDS) bevorzugt in Form seiner Natrium- oder Magnesiumsalze verwendet. Weiterhin bevorzugt sind in diesem Zusammenhang auch Glycerindisuccinate und Glycerintrisuccinate. Geeignete Einsatzmengen liegen in zeolithhaltigen und/oder silicathaltigen Formulierungen zwischen 3 und 15 Gew.-%.

Weitere brauchbare organische Cobuilder sind beispielsweise acetylierte Hydroxycarbonsäuren beziehungsweise deren Salze; welche gegebenenfalls auch in Lactonform vorliegen können und welche mindestens 4 Kohlenstoffatome und mindestens eine Hydroxygruppe sowie maximal zwei Säuregruppen enthalten.

Eine weitere Substanzklasse mit Cobuildereigenschaften stellen die Phosphonate dar. Dabei handelt es sich insbesondere um Hydroxyalkan- beziehungsweise Aminoalkanphosphonate. Unter den Hydroxyalkanphosphonaten ist das 1-Hydroxyethan-1,1-diphosphonat (HEDP) von besonderer Bedeutung als Cobuilder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, zum Beispiel als Hexanatriumsalz der EDTMP beziehungsweise als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden.

Darüberhinaus können alle Verbindungen, die in der Lage sind, Komplexe mit Erdalkaliionen auszubilden, als Cabuilder eingesetzt werden.

Buildersubstanzen können in den erfindungsgemäßen Mitteln gegebenenfalls in Mengen bis zu 90 Gew.-% enthalten sein. Sie sind vorzugsweise in Mengen bis zu 75 Gew.-% enthalten. Erfindungsgemäße Waschmittel weisen Buildergehalte von insbesondere 5 Gew.-% bis 50 Gew.-% auf. In erfindungsgemäßen Mitteln für die Reinigung harter Oberflächen, insbesondere zur maschinellen Reinigung von Geschirr, beträgt der Gehalt an Buildersubstanzen insbesondere 5 Gew.-% bis 88 Gew.-%, wobei in derartigen Mitteln vorzugsweise keine wasserunlöslichen Buildermaterialien eingesetzt werden. In einer bevorzugten Ausführungsform erfindungsgemäßer Mittel zur insbesondere maschinellen Reinigung von Geschirr sind 20 Gew.-% bis 40 Gew.-% wasserlöslicher organischer Builder, insbesondere Alkalicitrat, 5 Gew.-% bis 15 Gew.-% Alkalicarbonat und 20 Gew.-% bis 40 Gew.-% Alkalidisilikat enthalten.

Lösungsmittel, die in den flüssigen bis gelförmigen Zusammensetzungen von Wasch- und Reinigungsmitteln eingesetzt werden können, stammen-beispielsweise aus der Gruppe ein- oder mehrwertigen Alkohole, Alkanolamine oder Glycolether, sofern sie im angegebenen Konzentrationsbereich mit Wasser mischbar sind. Vorzugsweise werden die Lösungsmittel ausgewählt aus Ethanol, n- oder i-Propanol, Butanolen, Ethylenglykolmethylether, Ethylenglykolethylether, Ethylenglykolpropylether, Ethylenglykolmono-n-butylether, Diethylenglykol-methylether, Diethylenglykolethylether, Propylenglykolmethyl-, -ethyl- oder -propyl-ether, Dipropylenglykolmonomethyl-, oder - ethylether, Di-isopropylenglykolmonomethyl-, oder -ethylether, Methoxy-, Ethoxy- oder Butoxytriglykol, 1-Butoxyethoxy-2-propanol, 3-Methyl-3-methoxybutanol, Propylen-glykolt-butylether sowie Mischungen dieser Lösungsmittel.

Lösungsmittel können in den erfindungsgemäßen flüssigen bis gelförmigen Wasch- und Reinigungsmitteln in Mengen zwischen 0,1 und 20 Gew.-%, bevorzugt aber unter 15 Gew.-% und insbesondere unterhalb von 10 Gew.-% eingesetzt werden.

Zur Einstellung der Viskosität können der erfindungsgemäßen Zusammensetzung ein oder mehrere Verdicker, beziehungsweise Verdickungssysteme zugesetzt werden. Diese hochmolekularen Stoffe, die auch Quell(ungs)mittel genannt werden, saugen meist die Flüssigkeiten auf und quellen dabei auf, um schließlich in zähflüssige echte oder kolloide Lösungen überzugehen.

Geeignete Verdicker sind anorganische oder polymere organische Verbindungen. Zu den anorganischen Verdickern zählen beispielsweise Polykieselsäuren, Tonmineralien wie Montmorillonite, Zeolithe, Kieselsäuem und Bentonite. Die organischen Verdicker stammen aus den Gruppen der natürlichen Polymere, der abgewandelten natürlichen Polymere und der vollsynthetischen Polymere. Solche aus der Natur stammenden Polymere sind beispielsweise Agar-Agar, Carrageen, Tragant, Gummi arabicum, Alginate, Pektine, Polyosen, Guar-Mehl, Johannisbrotbaumkemmehl, Stärke, Dextrine, Gelatine und Casein. Abgewandelte Naturstoffe, die als Verdicker verwendet werden, stammen vor allem aus der Gruppe der modifizierten Stärken und Cellulosen. Beispielhaft seien hier Carboxymethylcellulose und andere Celluloseether, Hydroxyethyl-und -propylcellulose sowie Kemmehlether genannt. Vollsynthetische Verdicker sind Polymere wie Polyacryl- und Polymethacryl-Verbindungen, Vinylpolymere, Polycarbonsäuren, Polyether, Polyimine, Polyamide und Polyurethane.

Die Verdicker können in einer Menge bis zu 5 Gew.-%, vorzugsweise von 0,05 bis 2 Gew.-%, und besonders bevorzugt von 0,1 bis 1,5 Gew.-%, bezogen auf die fertige Zusammensetzung, enthalten sein.

Das erfindungsgemäße Wasch- und Reinigungsmittel kann gegebenenfalls als weitere übliche Inhaltsstoffe Sequestrierungsmittel, Elektrolyte und weitere Hilfsstoffe, wie optische Aufheller, Vergrauungsinhibitoren, Silberkorrosionsinhibitoren, Farbübertragungsinhibitoren, Schauminhibitoren, Abrasivstoffe, Farb- und/oder Duftstoffe, sowie mikrobielle Wirkstoffe und/oder UV-Absorbenzien enthalten.

Erfindungsgemäße Textilwaschmittel können als optische Aufheller Derivate der Diaminostilbendisulfonsäure beziehungsweise deren Alkalimetallsalze enthalten. Geeignet sind zum Beispiel Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, zum Beispiel die Alkalisalze des 4,4'-Bis(2-sulfostyryl)-diphenyls, 4,4-Bis(4-chlor-3-sulfostyryl)-diphenyls, oder 4-(4-Chlorstyryl)-4'-(2-sulfostyryl)-diphenyls. Auch Gemische der vorgenannten optischen Aufheller können verwendet werden.

Vergrauungsinhibitoren haben die Aufgabe, den von der Textilfaser abgelösten Schmutz in der Flotte suspendiert zu halten. Hierzu sind wasserlösliche Kolloide meist organischer Natur geeignet, beispielsweise Stärke, Leim, Gelatine, Salze von Ethercarbonsäuren oder Ethersulfonsäuren der Stärke oder der Cellulose oder Salze von sauren Schwefelsäureestem der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt werden Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, beispielsweise in Mengen von 0,1 bis 5 Gew.-%, bezogen auf die Mittel, eingesetzt.

Um einen Silberkorrosionsschutz zu bewirken, können in erfindungsgemäßen Reinigungsmitteln für Geschirr Silberkorrosionsinhibitoren eingesetzt werden. Solche sind aus dem Stand der Technik bekannt, beispielsweise Benzotriazole, Eisen(III)-chlorid oder CoSO₄. Wie beispielsweise aus der europäischen Patentschrift EP 0 736084 B1 bekannt ist, sind für die gemeinsame Verwendung mit Enzymen besonders geeignete Silberkorrosionsinhibitoren Mangan-, Titan-, Zirkonium-, Hafnium-, Vanadium-, Cobalt-oder Cersalze und/oder -komplexe, in denen die genannten Metalle in einer der Oxidationsstufen II, III, IV, V oder VI vorliegen. Beispiele für derartige Verbindungen sind MnSO₄, V₂O₅, V₂O₄, VO₂, TiOSO₄, K₂TiF₆, K₂ZrF₆, Co(NO₃)₂, Co(NO₃)₃, sowie deren Gemische.

"Soil-Release"-Wirkstoffe oder "Soil-Repellents" sind zumeist Polymere, die bei der Verwendung in einem Waschmittel der Wäschefaser schmutzabstoßende Eigenschaften verleihen und/oder das Schmutzablösevermögen der übrigen Waschmittelbestandteile unterstützen. Ein vergleichbarer Effekt kann auch bei deren Einsatz in Reinigungsmitteln für harte Oberflächen beobachtet werden.

Besonders wirksame und seit langer Zeit bekannte Soil-Release-Wirkstoffe sind Copolyester mit Dicarbonsäure-, Alkylenglykol- und Polyalkylenglykoleinheiten. Beispiele dafür sind Copolymere oder Mischpolymere aus Polyethylenterephthalat und Polyoxyethylenglykol (DT 1617141, beziehungsweise DT 22 00 911). In der deutschen Offenlegungsschrift DT 22 53 063 sind saure Mittel genannt, die unter anderem ein Copolymer aus einer dibasigen Carbonsäure und einem Alkylen- oder Cycloalkylenpolyglykol enthalten. Polymere aus Ethylenterephthalat und Polyethylenoxid-terephthalat und deren Einsatz in Waschmitteln sind in den deutschen Schriften DE 28 57 292 und DE 33 24 258 und der Europäischen Patentschrift EP 0 253 567 beschrieben. Das europäische Patent EP 066944 betrifft Mittel, die einen Copolyester aus Ethylenglykol, Polyethylenglykol, aromatischer Dicarbonsäure und sulfonierter aromatischer Dicarbonsäure in bestimmten Molverhältnissen enthalten. Aus dem europäischen Patent EP 0185 427 sind Methyl- oder Ethylgruppenendverschlossene Polyester mit Ethylen- und/oder Propylen-terephthalat- und Polyethylenoxid-terephthalat-Einheiten und Waschmitel, die derartiges Soil-release-Polymer enthalten, bekannt. Das europäische Patent EP 0 241984 betrifft einen Polyester, der neben Oxyethylen-Gruppen und Terephthalsäureeinheiten auch substituierte Ethyleneinheiten sowie Glycerineinheiten enthält. Aus dem europäischen Patent EP 0 241 985 sind Polyester bekannt, die neben Oxyethylen-Gruppen und Terephthalsäureeinheiten 1,2-Propylen-, 1,2-Butylen- und/oder 3-Methoxy-1,2-propylengruppen sowie Glycerineinheiten enthalten und mit C₁- bis C₄-Alkylgruppen endgruppenverschlossen sind. Aus der europäischen Patentanmeldung EP 0 272033 sind zumindest anteilig durch C₁₋₄-Alkyl- oder Acylreste endgruppenverschlossene Polyester mit Poly-propylenterephthalat- und Polyoxyethylenterephthalat-Einheiten bekannt. Das europäische Patent EP 0 274 907 beschreibt sulfoethylendgruppenverschlossene terephthalathaltige Soil-release-Polyester. Gemäß der europäischen Patentanmeldung EP 0 357 280 werden durch Sulfonierung ungesättigter Endgruppen Soil-Release-Polyester mit Terephthalat-, Alkylenglykol- und Poly-C₂₋₄-Glykol-Einheiten hergestellt. Die internationale Patentanmeldung WO 95/32232 betrifft saure, aromatische schmutzablösevermögende Polyester. Aus der internationalen Patentanmeldung WO 97/31085 sind nicht polymere soil-repellent-Wirkstoffe für Materialien aus Baumwolle mit mehreren funktionellen Einheiten bekannt: Eine erste Einheit, die beispielsweise kationisch sein kann, ist zur Adsorption auf die Baumwolloberfläche durch elektrostatische Wechselwirkung befähigt, und eine zweite Einheit, die hydrophob ausgebildet ist, ist verantwortlich für das Verbleiben des Wirkstoffs an der Wasser/ Baumwolle-Grenzfläche.

Zu den für den Einsatz in erfindungsgemäßen Textilwaschmitteln in Frage kommenden Farbübertragungsinhibitoren gehören insbesondere Polyvinylpyrrolidone, Polyvinylimidazole, polymere N-Oxide wie Poly-(vinylpyridin-N-oxid) und Copolymere von Vinylpyrrolidon mit Vinylimidazol.

Beim Einsatz in maschinellen Reinigungsverfahren kann es von Vorteil sein, den Mitteln Schauminhibitoren zuzusetzen. Als Schauminhibitoren eignen sich beispielsweise Seifen natürlicher oder synthetischer Herkunft, die einen hohen Anteil an C₁₈-C₂₄-Fettsäuren aufweisen. Geeignete nichttensidartige Schauminhibitoren sind beispielsweise Organopolysiloxane und deren Gemische mit mikrofeiner, gegebenenfalls silanierter Kieselsäure sowie Paraffine, Wachse, Mikrokristallinwachse und deren Gemische mit silanierter Kieselsäure oder Bistearylethylendiamid. Mit Vorteilen werden auch Gemische aus verschiedenen Schauminhibitoren verwendet, zum Beispiel solche aus Silikonen, Paraffinen oder Wachsen. Vorzugsweise sind die Schauminhibitoren, insbesondere Silikon- und/oder Paraffin-haltige Schauminhibitoren, an eine granulare, in Wasser lösliche, beziehungsweise dispergierbare Trägersubstanz gebunden. Insbesondere sind dabei Mischungen aus Paraffinen und Bistearylethylendiamiden bevorzugt.

Ein erfindungsgemäßes Reinigungsmittel für harte Oberflächen kann darüber hinaus abrasiv wirkende Bestandteile, insbesondere aus der Gruppe umfassend Quarzmehle, Holzmehle, Kunststoffmehle, Kreiden und Mikroglaskugeln sowie deren Gemische, enthalten. Abrasivstoffe sind in den erfindungsgemäßen Reinigungsmitteln vorzugsweise nicht über 20 Gew.-%, insbesondere von 5 Gew.-% bis 15 Gew.-%, enthalten.

Farb- und Duftstoffe werden Wasch- und Reinigungsmitteln zugesetzt, um den ästhetischen Eindruck der Produkte zu verbessern und dem Verbraucher neben der Wasch- und Reinigungsleistung ein visuell und sensorisch "typisches und unverwechselbares" Produkt zur Verfügung zu stellen. Als Parfümöle beziehungsweise Duftstoffe können einzelne Riechstoffverbindungen, zum Beispiel die synthetischen Produkte vom Typ der Ester, Ether, Aldehyde, Ketone, Alkohole und Kohlenwasserstoffe verwendet werden. Riechstoffverbindungen vom Typ der Ester sind zum Beispiel Benzylacetat, Phenoxyethylisobutyrat, p-tert.-Butylcyclohexylacetat, Linalylacetat, Dimethylbenzyl-carbinylacetat, Phenylethylacetat, Linalylbenzoat, Benzylformiat, Ethylmethylphenyl-glycinat, Allylcyclohexylpropionat, Styrallylpropionat und Benzylsalicylat. Zu den Ethem zählen beispielsweise Benzylethylether, zu den Aldehyden zum Beispiel die linearen Alkanale mit 8-18 C-Atomen, Citral, Citronellal, Citronellyloxyacetaldehyd, Cyclamenaldehyd, Hydroxycitronellal, Lilial und Bourgeonal, zu den Ketonen zum Beispiel die Jonone, α-Isomethylionon und Methyl-cedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind, zum Beispiel Pine-, Citrus-, Jasmin-, Patchouly-, Rosen- oder Ylang-Ylang-Öl. Ebenfalls geeignet sind Muskateller, Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl. Üblicherweise liegt der Gehalt von Wasch- und Reinigungsmitteln an Farbstoffen unter 0,01 Gew.-%, während Duftstoffe bis zu 2 Gew.-% der gesamten Formulierung ausmachen können.

Die Duftstoffe können direkt in die Wasch- und Reinigungsmittel eingearbeitet werden, es kann aber auch vorteilhaft sein, die Duftstoffe auf Träger aufzubringen, die die Haftung des Parfüms auf dem Reinigungsgut verstärken und durch eine langsamere Duftfreisetzung für langanhaltenden Duft, insbesondere von behandelten Textilien sorgen. Als solche Trägermaterialien haben sich beispielsweise Cyclodextrine bewährt, wobei die Cyclodextrin-Parfüm-Komplexe zusätzlich noch mit weiteren Hilfsstoffen beschichtet werden können. Ein weiter bevorzugter Träger für Duftstoffe ist der beschriebene Zeolith X, der anstelle von oder in Mischung mit Tensiden auch Duftstoffe aufnehmen kann. Bevorzugt sind daher Wasch- und Reinigungsmittel, die den beschriebenen Zeolith X und Duftstoffe, die vorzugsweise zumindest teilweise an dem Zeolithen absorbiert sind, enthalten.

Bevorzugte Farbstoffe, deren Auswahl dem Fachmann keinerlei Schwierigkeit bereitet, besitzen eine hohe Lagerstabilität und Unempfindlichkeit gegenüber den übrigen Inhaltsstoffen der Mittel und gegen Licht sowie keine ausgeprägte Substantivität gegenüber Textilfasern, um diese nicht anzufärben.

Zur Bekämpfung von Mikroorganismen können Wasch- oder Reinigungsmittel antimikrobielle Wirkstoffe enthalten. Hierbei unterscheidet man je nach antimikrobiellem Spektrum und Wirkungsmechanismus zwischen Bakteriostatika und Bakteriziden, Fungistatika und Fungiziden usw. Wichtige Stoffe aus diesen Gruppen sind beispielsweise Benzalkoniumchloride, Alkylarylsulfonate, Halogenphenole und Phenolmercuriacetat. Die Begriffe antimikrobielle Wirkung und antimikrobieller Wirkstoff haben im Rahmen der erfindungsgemäßen Lehre die fachübliche Bedeutung, die beispielsweise von *K. H. Wallhäußer* in "Praxis der Sterilisation, Desinfektion - Konservierung : Keimidentifizierung - Betriebshygiene" (5. Aufl. - Stuttgart; New York : Thieme, 1995) wiedergegeben wird, wobei alle dort beschriebenen Substanzen mit antimikrobieller Wirkung eingesetzt werden können. Geeignete antimikrobielle Wirkstoffe sind vorzugsweise ausgewählt aus den Gruppen der Alkohole, Amine, Aldehyde, antimikrobiellen Säuren beziehungsweise deren Salze, Carbonsäureester, Säureamide, Phenole, Phenolderivate, Diphenyle, Diphenylalkane, Harnstoffderivate, Sauerstoff-, Stickstoff-acetale sowie -formale, Benzamidine, Isothiazoline, Phthalimidderivate, Pyridinderivate, antimikrobiellen oberflächenaktiven Verbindungen, Guanidine, antimikrobiellen amphoteren Verbindungen, Chinoline, 1,2-Dibrom-2,4-dicyanobutan, Iodo-2-propyl-butyl-carbamat, lod, lodophore, Peroxoverbindungen, Halogenverbindungen sowie beliebigen Gemischen der voranstehenden.

Der antimikrobielle Wirkstoff kann dabei ausgewählt sein aus Ethanol, n-Propanol, i-Propanol, 1,3-Butandiol, Phenoxyethanol, 1,2-Propylenglykol, Glycerin, Undecylensäure, Benzoesäure, Salicylsäure, Dihydracetsäure, o-Phenylphenol, N-Methylmorpholinacetonitril (MMA), 2-Benzyl-4-chlorphenol, 2,2'-Methylen-bis-(6-brom-4-chlorphenol), 4,4'-Dichlor-2'-hydroxydiphenylether (Dichlosan), 2,4,4'-Trichlor-2'-hydroxydiphenylether (Trichlosan), Chlorhexidin, N-(4-Chlorphenyl)-N-(3,4-dichlorphenyl)-harnstoff, N,N'-(1,10-decan-diyldi-1-pyridinyl-4-yliden)-bis-(1-octanamin)-dihydrochlorid, N,N'-Bis-(4-chlorphenyl)-3,12-diimino-2,4,11,13-tetraaza-tetradecandiimidamid, Glucoprotaminen, antimikrobiellen oberflächenaktiven quaternären Verbindungen, Guanidinen einschl. den Bi- und Polyguanidinen, wie beispielsweise 1,6-Bis-(2-ethylhexyl-biguanido-hexan)-dihydrochlorid, 1,6-Di-(N₁,N₁'-phenyldiguanido-N₅,N₅')-hexan-tetrahydochlorid, 1,6-Di-(N₁,N₁'-phenyl-N₁,N₁-methyldiguanido-N₅,N₅')-hexan-dihydrochlorid, 1,6-Di-(N₁,N₁'-o-chlorophenyldiguanido- N₅,N₅')-hexan-dihydrochlorid, 1,6-Di-(N₁,N₁'-2,6-dichlorophenyldiguanido-N₅,N₅')hexan-dihydrochlorid, 1,6-Di-[N₁,N₁'-beta-(p-methoxyphenyl) diguanido-N₅,N₅']-hexane-dihydrochlorid, 1,6-Di-(N₁,N₁'-alpha-methyl-.beta.-phenyldiguanido-N₅,N₅')-hexan-dihydrochlorid, 1,6-Di-(N₁,N₁'-p-nitrophenyldiguanido-N₅,N₅')hexan-dihydrochlorid, omega:omega-Di-(N₁,N₁'-phenyldiguanido-N₅,N₅')-di-n-propylether-dihydrochlorid, omega:omega'-Di-(N₁,N₁'-p-chlorophenyldiguanido-N₅,N₅')-di-n-propylether-tetrahydrochlorid, 1,6-Di-(N₁,N₁'-2,4-dichlorophenyldiguanido-N₅,N₅')hexan-tetrahydrochlorid, 1,6-Di-(N₁,N₁'-p-methylphenyldiguanido- N₅,N₅')hexan-dihydrochlorid, 1,6-Di-(N₁,N₁'-2,4,5-trichlorophenyldiguanido-N₅,N₅')hexan-tetrahydrochlorid, 1,6-Di-[N₁,N₁'-alpha-(p-chlorophenyl) ethyldiguanido-N₅,N₅'] hexan-dihydrochlorid, omega:omega-Di-(N₁,N₁'-p-chlorophenyldiguanido-N₅,N₅')m-xylene-dihydrochlorid, 1,12-Di-(N₁,N₁'-p-chlorophenyldiguanido-N₅,N₅') dodecan-dihydrochlorid, 1,10-Di-(N₁,N₁'-phenyldiguanido-N₅,N₅')-decan-tetrahydrochlorid, 1,12-Di-(N₁,N₁'-phenyldiguanido- N₅,N₅') dodecantetrahydrochlorid, 1,6-Di-(N₁,N₁'-o-chlorophenyldiguanido- N₅,N₅') hexan-dihydrochlorid, 1,6-Di-(N₁,N₁'-o-chlorophenyldiguanido- N₅,N₅') hexan-tetrahydrochlorid, Ethylen-bis-(1-tolyl biguanid), Ethylen-bis-(p-tolyl biguanide), Ethylen-bis-(3,5-dimethylphenylbiguanid), Ethylen-bis-(p-tert-amylphenylbiguanid), Ethylen-bis-(nonylphenylbiguanid), Ethylen-bis-(phenylbiguanid), Ethylen-bis-(N-butylphenylbiguanid), Ethylen-bis (2,5-diethoxyphenylbiguanid), Ethylen-bis (2,4-dimethylphenyl biguanid), Ethylen-bis (o-diphenylbiguanid), Ethylen-bis (mixed amyl naphthylbiguanid), N-Butyl-ethylen-bis-(phenylbiguanid), Trimethylen bis (o-tolylbiguanid), N-Butyl-trimethyle- bis-(phenyl biguanide) und die entsprechenden Salze wie Acetate, Gluconate, Hydrochloride, Hydrobromide, Citrate, Bisulfite, Fluoride, Polymaleate, N-Cocosalkylsarcosinate, Phosphite, Hypophosphite, Perfluorooctanoate, Silicate, Sorbate, Salicylate, Maleate, Tartrate, Fumarate, Ethylendiamintetraacetate, Iminodiacetate, Cinnamate, Thiocyanate, Arginate, Pyromellitate, Tetracarboxybutyrate, Benzoate, Glutarate, Monofluorphosphate, Perfluorpropionate sowie beliebige Mischungen davon. Weiterhin eignen sich halogenierte Xylol- und Kresolderivate, wie p-Chlormetakresol oder p-Chlormeta-xylol, sowie natürliche antimikrobielle Wirkstoffe pflanzlicher Herkunft (zum Beispiel aus Gewürzen oder Kräutern), tierischer sowie mikrobieller Herkunft. Vorzugsweise können antimikrobiell wirkende oberflächenaktive quatemäre Verbindungen, ein natürlicher antimikrobieller Wirkstoff pflanzlicher Herkunft und/oder ein natürlicher antimikrobieller Wirkstoff tierischer Herkunft, äußerst bevorzugt mindestens ein natürlicher antimikrobieller Wirkstoff pflanzlicher Herkunft aus der Gruppe, umfassend Coffein, Theobromin und Theophyllin sowie etherische Öle wie Eugenol, Thymol und Geraniol, und/oder mindestens ein natürlicher antimikrobieller Wirkstoff tierischer Herkunft aus der Gruppe, umfassend Enzyme wie Eiweiß aus Milch, Lysozym und Lactoperoxidase, und/oder mindestens eine antimikrobiell wirkende oberflächenaktive quatemäre Verbindung mit einer Ammonium-, Sulfonium-, Phosphonium-, lodonium- oder Arsoniumgruppe, Peroxoverbindungen und Chlorverbindungen eingesetzt werden. Auch Stoffe mikrobieller Herkunft, sogenannte Bakteriozine, können eingesetzt werden.

Die als antimikrobielle Wirkstoffe geeigneten quatemären Ammoniumverbindungen (QAV) weisen die allgemeine Formel (R¹)(R²)(R³)(R⁴) N⁺ X⁻ auf, in der R¹ bis R⁴ gleiche oder verschiedene C₁-C₂₂-Alkylreste, C₇-C₂₈-Aralkylreste oder heterozyklische Reste, wobei zwei oder im Falle einer aromatischen Einbindung wie im Pyridin sogar drei Reste gemeinsam mit dem Stickstoffatom den Heterozyklus, zum Beispiel eine Pyridinium- oder Imidazoliniumverbindung, bilden, darstellen und X⁻ Halogenidionen, Sulfationen, Hydroxidionen oder ähnliche Anionen sind. Für eine optimale antimikrobielle Wirkung weist vorzugsweise wenigstens einer der Reste eine Kettenlänge von 8 bis 18, insbesondere12 bis 16, C-Atomen auf.

QAV sind durch Umsetzung tertiärer Amine mit Alkylierungsmitteln, wie zum Beispiel Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quatemierung von tertiären Aminen mit zwei langen Resten und einer Methyl-Gruppe kann mit Hilfe von Methylchlorid unter milden Bedingungen durchgeführt werden. Amine, die über drei lange Alkyl-Reste oder Hydroxy-substituierte Alkyl-Reste verfügen, sind wenig reaktiv und werden bevorzugt mit Dimethylsulfat quaterniert.

Geeignete QAV sind beispielweise Benzalkoniumchlorid (N-Alkyl-N,N-dimethyl-benzylammoniumchlorid, CAS No. 8001-54-5), Benzalkon B (*m*,*p*-Dichlorbenzyl-dimethyl-C12-alkylammoniumchlorid, CAS No. 58390-78-6), Benzoxoniumchlorid (Benzyl-dodecyl-bis-(2-hydroxyethyl)-ammonium-chlorid), Cetrimoniumbromid (N-Hexadecyl-N,N-trimethylammoniumbromid, CAS No. 57-09-0), Benzetoniumchlorid (N,N-Dimethyl-N-[2-[2-[*p*-(1,1,3,3-tetramethylbutyl)-pheno-xy]ethoxy]ethyl]-benzylammoniumchlorid, CAS No. 121-54-0), Dialkyldimethylammonium-chloride wie Di-*n*-decyl-dimethyl-ammoniumchlorid (CAS No. 7173-51-5-5), Didecyldi-methylammoniumbromid (CAS No. 2390-68-3), Dioctyl-dimethyl-ammoniumchloric, 1-Cetylpyridiniumchlorid (CAS No. 123-03-5) und Thiazoliniodid (CAS No. 15764-48-1) sowie deren Mischungen. Besonders bevorzugte QAV sind die Benzalkoniumchloride mit C₈-C₁₈-Alkylresten, insbesondere C₁₂-C₁₄-Aklyl-benzyl-dimethyl-ammoniumchlorid.

Benzalkoniumhalogenide und/oder substituierte Benzalkoniumhalogenide sind beispielsweise kommerziell erhältlich als Barquat® ex Lonza, Marquat® ex Mason, Variquat® ex Witco/ Sherex und Hyamine® ex Lonza, sowie Bardac® ex Lonza. Weitere kommerziell erhältliche antimikrobielle Wirkstoffe sind N-(3-Chlorallyl)-hexaminiumchlorid wie Dowicide® und Dowicil® ex Dow, Benzethoniumchlorid wie Hyamine® 1622 ex Rohm & Haas, Methylbenzethoniumchlorid wie Hyamine® 10X ex Rohm & Haas, Cetylpyridiniumchlorid wie Cepacolchlorid ex Merrell Labs.

Die antimikrobiellen Wirkstoffe werden in Mengen von 0,0001 Gew.-% bis 1 Gew.-%, bevorzugt von 0,001 Gew.-% bis 0,8 Gew.-%, besonders bevorzugt von 0,005 Gew.-% bis 0,3 Gew.-% und insbesondere von 0,01 bis 0,2 Gew.-% eingesetzt.

Die Mittel können UV-Absorbenzien (UV-Absorber) enthalten, die auf die behandelten Textilien aufziehen und die Lichtbeständigkeit der Fasern und/oder die Lichtbeständigkeit sonstiger Rezepturbestandteile verbessern. Unter UV-Absorber sind organische Substanzen (Lichtschutzfilter) zu verstehen, die in der Lage sind, ultraviolette Strahlen zu absorbieren und die aufgenommene Energie in Form längerwelliger Strahlung, zum Beispiel Wärme wieder abzugeben.

Verbindungen, die diese gewünschten Eigenschaften aufweisen, sind beispielsweise die durch strahlungslose Desaktivierung wirksamen Verbindungen und Derivate des Benzophenons mit Substituenten in 2- und/oder 4-Stellung. Weiterhin sind auch substituierte Benzotriazole, in 3-Stellung Phenylsubstituierte Acrylate (Zimtsäurederivate, gegebenenfalls mit Cyanogruppen in 2-Stellung), Salicylate, organische Ni-Komplexe sowie Naturstoffe wie Umbelliferon und die körpereigene Urocansäure geeignet. Besondere Bedeutung haben Biphenyl- und vor allem Stilbenderivate wie sie beispielsweise in der EP 0728749 A beschrieben werden und kommerziell als Tinosorb® FD oder Tinosorb® FR ex Ciba erhältlich sind. Als UV-B-Absorber sind zu nennen: 3-Benzylidencampher beziehungsweise 3-Benzylidennorcampher und dessen Derivate, zum Beispiel 3-(4-Methylbenzyliden)campher, wie in der EP 0693471 B1 beschrieben; 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester; Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3,3-phenylzimtsäure-2-ethylhexylester (Octocrylene); Ester der Salicylsäure, vorzugsweise Salicylsäure-2-ethylhexylester, Salicylsäure-4-isopropylbenzylester, Salicylsäurehomomenthylester, Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon; Ester der Benzalmalonsäure, vorzugsweise 4-Methoxybenzmalonsäuredi-2-ethylhexylester; Triazinderivate, wie zum Beispiel 2,4,6-Trianilino-(p-carbo-2'-ethyl-1'-hexyloxy)-1,3,5-triazin und Octyl Triazon, wie in der EP 0818450 A1 beschrieben oder Dioctyl Butamido Triazone (Uvasorb® HEB); Propan-1,3-dione, wie zum Beispiel 1-(4-tert.Butylphenyl)-3-(4'methoxyphenyl)propan-1,3-dion; Ketotricyclo(5.2.1.0)decan-Derivate, wie in der EP 0694521 B1 beschrieben. Weiterhin geeignet sind 2-Phenylbenzimidazol-5-sulfonsäure und deren Alkali-, Erdalkali-, Ammonium-, Alkylammonium-, Alkanolammonium- und Glucammoniumsalze; Sulfonsäurederivate von Benzophenonen, vorzugsweise 2-Hydroxy-4-methoxybenzophenon-5-sulfonsäure und ihre Salze; Sulfonsäurederivate des 3-Benzylidencamphers, wie zum Beispiel 4-(2-Oxo-3-bornylidenmethyl)benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beschrieben in der DE 19712033 A1 (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse, vorzugsweise nanoisierte Metalloxide beziehungsweise Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente bereits für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, das heißt hydrophilisiert oder hydrophobiert vorliegen. Typische Beispiele sind ummantelte Titandioxide, wie zum Beispiel Titandioxid T 805 (Degussa) oder Eusolex® T2000 (Merck; als hydrophobe Coatingmittel kommen dafür bevorzugt Silicone und besonders bevorzugt Trialkoxyoctylsilane oder Simethicone in Frage. Vorzugsweise wird mikronisiertes Zinkoxid verwendet. Weitere geeignete UV-Lichtschutzfilter sind der Übersicht von P. Finkel in SÖFW-Journal 122 (1996), S. 543 zu entnehmen.

Die UV-Absorbenzien werden üblicherweise in Mengen von 0,01 Gew.-% bis 5 Gew.-%, vorzugsweise von 0,03 Gew.-% bis 1 Gew.-%, eingesetzt.

Auch in Wasch- und Reinigungsmitteln können erfindungsgemäße und/oder andere Proteine eines besonderen Schutzes bedürfen. Erfindungsgemäße Mittel können zu diesem Zweck Stabilisatoren enthalten.

Eine Gruppe von Stabilisatoren sind reversible Proteaseinhibitoren, die bei Verdünnung des Mittels in der Waschflotte abdissozieren. Benzamidin-Hydrochlorid und Leupeptin sind für diesen Zweck etabliert. Häufig werden Borax, Borsäuren, Boronsäuren oder deren Salze oder Ester verwendet, darunter vor allem Derivate mit aromatischen Gruppen, etwa gemäß WO 95/12655 ortho-substituierte, gemäß WO 92/19707 metasubstituierte und gemäß US 5 972 873 para-substituierte Phenylboronsäuren, beziehungsweise deren Salze oder Ester. In den Anmeldungen WO 98/13460 und EP 583 534 werden Peptidaldehyde, das heißt Oligopeptide mit reduziertem C-Terminus, und zwar solche aus 2 - 50 Monomeren, zur reversiblen Inhibierung von Wasch- und Reinigungsmittelproteasen offenbart. Zu den peptidischen reversiblen Proteaseinhibitoren gehört unter anderem Ovomucoid (WO 93/00418). Beispielsweise mit der Anmeldung WO 00/01826 werden spezifische, reversible Peptid-Inhibitoren für die Protease Subtilisin zur Verwendung in Protease-haltigen Mitteln offenbart, und mit WO 00/01831 entsprechende Fusionsproteine aus Protease und Inhibitor.

Weitere Enzymstabilisatoren sind Aminoalkohole wie Mono-, Di-, Triethanol- und -Propanolamin und deren Mischungen, aliphatische Carbonsäuren bis zu C₁₂, wie beispielsweise aus den Anmeldungen EP 0 378 261 und WO 97/05227 bekannt ist, wie Bernteinsäure, andere Dicarbonsäuren oder Salze der genannten Säuren. Mit der Anmeldung DE 19650537 werden für diesen Zweck endgruppenverschlossene Fettsäureamidalkoxylate offenbart. Bestimmte als Builder eingesetzte organische Säuren vermögen, wie in WO 97/18287 offenbart, zusätzlich ein enthaltenes Enzym zu stabilisieren.

Niedere aliphatische Alkohole, vor allem aber Polyole, wie beispielsweise Glycerin, Ethylenglykol, Propylenglykol oder Sorbit sind weitere häufig eingesetzte Enzymstabilisatoren. Nach einer neueren Anmeldung (EP 0 965 268) schützt auch Di-Glycerinphosphat gegen Denaturierung durch physikalische Einflüsse. Ebenso werden - Calciumsalze verwendet, wie beispielsweise Calciumacetat oder das in der Patentschrift EP 0 028 865 für diesen Zweck offenbarte Calcium-Formiat, und Magnesiumsalze, etwa gemäß der europäischen Anmeldung EP 0 378 262.

Polyamid-Oligomere (WO 99/43780) oder polymere Verbindungen wie Lignin (WO 97/00932), wasserlösliche Vinyl-Copolymere (EP 828 762) oder, wie in EP 702 712 offenbart, Cellulose-Ether, Acryl-Polymere und/oder Polyamide stabilisieren die Enzym-Präparation unter anderem gegenüber physikalischen Einflüssen oder pH-Wert-Schwankungen. Polyamin-N-Oxid-enthaltende Polymere (EP 587 550 und EP 581751) wirken gleichzeitig als Enzymstabilisatoren und als Farbübertragungsinhibitoren. Andere polymere Stabilisatoren sind die in WO 97/05227 neben anderen Bestandteilen offenbarten linearen C₈-C₁₈ Polyoxyalkylene. Alkylpolyglycoside könnten wie in den Anmeldungen WO 97/43377 und WO 98/45396 die enzymatischen Komponenten des erfindungsgemäßen Mittels stabilisieren und sogar in ihrer Leistung steigern. Vemetzte N-haltige Verbindungen, wie in WO 98/17764 offenbart, erfüllen eine Doppelfunktion als Soil-release-Agentien und als Enzym-Stabilisatoren. Hydrophobes, nichtionisches Polymer wirkt in einer Mischung mit anderen Stabilisatoren gemäß der Anmeldung WO 97/32958 stabilisierend auf eine Cellulase, so daß sich solche oder ähnliche Bestandteile auch für das erfindungswesentliche Enzym eignen könnten.

Reduktionsmittel und Antioxidantien erhöhen, wie unter anderem in EP 780 466 offenbart, die Stabilität der Enzyme gegenüber oxidativem Zerfall. Schwefelhaltige Reduktionsmittel sind beispielsweise aus den Patentschriften EP 0 080748 und EP 0 080 223 bekannt. Andere Beispiele sind Natrium-Sulfit (EP 533 239) und reduzierende Zucker (EP 656 058).

Vielfach werden auch Kombinatonen von Stabilisatoren verwendet, beispielsweise aus Polyolen, Borsäure und/oder Borax in der Anmeldung WO 96/31589, die Kombination von Borsäure oder Borat, reduzierenden Salzen und Bernsteinsäure oder anderen Dicarbonsäuren in der Anmeldung EP 126 505 oder die Kombination von Borsäure oder Borat mit Polyolen oder Polyaminoverbindungen und mit reduzierenden Salzen, wie in der Anmeldung EP 080 223 offenbart. Die Wirkung von Peptid-Aldehyd-Stabilisatoren wird gemäß WO 98/13462 durch die Kombination mit Borsäure und/oder Borsäurederivaten und Polyolen gesteigert und gemäß WO 98/13459 durch die zusätzliche Verwendung von Calcium-Ionen noch weiter verstärkt.

Mittel mit stabilisierten Enzymaktivitäten stellen bevorzugte Ausführungsformen der vorliegenden Erfindung dar. Besonders bevorzugt sind solche mit Enzymen, die auf mehrere der dargestellten Weisen stabilisiert sind.

Erfindungsgemäße Mittel enthalten erfindungswesentliche Proteine oder Derivate vorzugsweise in Mengen von 0,000001 Gewichts-Prozent bis 5 Gew.-%, und zunehmend bevorzugt von 0,00005 bis 4 Gew.-%, von 0,00001 bis 3 Gew.-%, von 0,0001 bis 2 Gew.-%, und besonders bevorzugt von 0,001 bis 1 Gew.-%. Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren (A. G. Gomall, C. S. Bardawill und M.M. David, *J. Biol. Chem.* 177 (1948), S. 751-766) bestimmt werden.

Erfindungsgemäße Mittel können zusätzlich zu dem erfindungswesentlichen Protein andere amylolytische Enzyme, insbesondere α-Amylasen enthalten. Darunter können sich auch die eingangs dargestellten, für die Verwendung in Wasch- und Reinigungsmitteln etablierten Enzyme befinden. Beispiele für kommerziell erhältliche Amylasen sind BAN®, Termamyl®, Purastar®, Amylase-LT®, Maxamyl®, Duramyl® und/oder Purafect® OxAm. Dies ist dann angebracht, wenn sich die verschiedenen Enzyme einander zu ergänzen vermögen. Solch eine Ergänzung kann beispielsweise in regulatorischer Hinsicht erfolgen, etwa durch gegenseitige Aktivierung oder durch Inaktivierung. Sie kann sich beispielsweise dadurch ergeben, daß mindestens ein nicht zu den bekannten α-Amylasen homologer Teil des erfindungswesentlichen Enzyms Einfluß auf die nicht erfindungswesentlichen amylolytischen Aktivitäten nimmt. Die gemeinsame Verwendung kann aber auch aufgrund abweichender Sabstratspezifitäten sinnvoll sein. Beides sind Ausführungsformen der vorliegenden Erfindung.

Insbesondere an chemisch diversen Anschmutzungen kann es vorteilhaft sein, amylolytische Enzyme in Wasch- und Reinigungsmitteln zusammen mit anderen wasch- und/oder reinigungsaktiven Enzymen einzusetzen. Somit stellen Wasch- oder Reinigungsmittel, die über ein erfindungsgemäßes Protein hinaus durch zusätzlich weitere Enzyme gekennzeichnet sind, bevorzugte Ausführungsformen der vorliegenden Erfindung dar.

Dazu gehören neben weiteren Amylasen beispielsweise Proteasen, aber auch Lipasen, Cutinasen, Esterasen, Pullulanasen, Cellulasen, Hemicellulasen und/oder Xylanasen, sowie deren Gemische. Besonders bevorzugt sind Proteasen, Lipasen, β-Glucanasen und/oder Cellulasen. Weitere Enzyme erweitem die Reinigunsgleistung entsprechender Mittel um ihre jeweils spezifische enzymatische Leistung. Dazu gehören beispielsweise Oxidoreductasen oder Peroxidasen als Komponenten von enzymatischen Bleichsystemen, Laccasen (WO 00/39306), β-Glucanasen (WO 99/06515 und WO 99/06516) oder Pektin-lösende Enzyme (WO 00/42145), die insbesondere in Spezialwaschmitteln zum Einsatz kommen.

Beispiele für kommerziell erhältliche Enzyme zum Gebrauch in erfindungsgemäßen Mitteln sind Proteasen wie Subtilisin BPN', Properase®, BLAP®, Optimase®, Opticlean®, Maxatase®, Maxacal®, Maxapem®, Alcalase®, Esperase®, Savinase®, Durazym®, Everlase® und/oder Purafect®G oder Purafect®OxP und Lipasen wie Lipolase®, Lipomax®, Lumafast® und/oder Lipozym®.

Die Proteaseaktivität in derartigen Mitteln kann nach der in *Tenside,* Bd. 7 (1970), S. 125-132 beschriebenen Methode ermittelt werden. Sie wird dementsprechend in PE (Protease-Einheiten) angegeben. Die Proteaseaktivität bevorzugter Mittel kann bis zu 1.500.000 Proteaseeinheiten pro Gramm Zubereitung betragen (PE, bestimmt nach der in Tenside, Bd. 7 (1970), S. 125-132 beschriebenen Methode).

Hinsichtlich ihrer Gewinnung kommen unter den verwendbaren Enzymen in erster Linie die aus Mikroorganismen, wie Bakterien oder Pilzen, gewonnenen in Frage, beispielsweise aus *Bacillus subtilis, Bacillus licheniformis, Streptomyces griseus,* *Humicola lanuginosa, Humicola insolens, Pseudomonas pseudoalcaligenes* oder *Pseudomonas cepacia,* insbesondere die von diesen Stämmen natürlicherweise gebildeten Enzymgemische, beziehungsweise Gemische mit denen anderer Stämme. Sie werden in bekannter Weise durch Fermentationsprozesse aus geeigneten Mikroorganismen gewonnen, die zum Beispiel in den deutschen Offenlegungsschriften DE 19 40 488 und DE 21 21 397, den US-amerikanischen Patentschriften US 3 623957 und US 4 264 738, der europäischen Patentanmeldung EP 006 638, sowie der internationalen Patentanmeldung WO 91/02792 beschrieben sind.

Auch diese gegebenenfalls zusätzlich verwendeten Enzyme können, wie zum Beispiel in der europäischen Patentschrift EP 0 564 476 oder in der internationalen Patentanmeldungen WO 94/23005 beschrieben, an Trägerstoffen adsorbiert und/oder in Hüllsubstanzen eingebettet sein, um sie gegen vorzeitige Inaktivierung zu schützen. Sie sind in Waschmitteln vorzugsweise in Mengen bis zu 10 Gew.-%, insbesondere von 0,2 Gew.-% bis 2 Gew.-%, enthalten, wobei besonders bevorzugt gegen oxidativen Abbau stabilisierte Enzyme, wie zum Beispiel aus den internationalen Patentanmeldungen WO 94/18314 bekannt, eingesetzt werden.

Erfindungsgemäße Mittel können, beispielsweise um die enthaltenen Wirkstoffe zeitlich oder räumlich voneinander getrennt freizusetzen, aus mehreren Phasen bestehen. Dabei kann es sich um Phasen in verschiedenen Aggregatzuständen, insbesondere aber um zwei Phasen in denselben Aggregatzuständen handeln.

Erfindungsgemäße Mittel, die aus mehreren festen Komponenten zusammengesetzt sind, können auf einfache Weise dadurch hergestellt werden, daß verschiedene Pulver oder Granulate mit verschiedenen Inhaltsstoffen und/oder unterschiedlichem Freisetzungsverhalten in insgesamt loser Form miteinander vermischt werden. Die Herstellung erfindungsgemäßer fester Mittel aus einer oder mehreren Phasen kann auf bekannte Weise, zum Beispiel durch Sprühtrocknen oder Granulation erfolgen, wobei die Enzyme und eventuelle weitere thermisch empfindliche Inhaltsstoffe wie zum Beispiel Bleichmittel gegebenenfalls später separat zugesetzt werden. Zur Herstellung erfindungsgemäßer Mittel mit erhöhtem Schüttgewicht, insbesondere im Bereich von 650 g/l bis 950 g/l, ist ein aus der europäischen Patentschrift EP 0 486 592 bekanntes, einen Extrusionschritt aufweisendes Verfahren bevorzugt. Eine weitere bevorzugte Herstellung mit Hilfe eines Granulationsverfahrens ist in der europäischen Patentschrift EP 0 642 576 beschrieben.

Proteine können für feste Mittel beispielsweise in getrockneter, granulierter, verkapselter oder verkapselter und zusätzlich getrockneter Form eingesetzt werden. Sie können separat, das heißt als eigene Phase, oder mit anderen Bestandteilen zusammen in derselben Phase, mit oder ohne Kompaktierung zugesetzt werden. Sollen mikroverkapselte Enzyme in fester Form verarbeitet werden, so kann das Wasser mit aus dem Stand der Technik bekannten Verfahren aus den sich aus der Aufarbeitung ergebenden wäßrigen Lösungen entfemt werden, wie Sprühtrocknung, Abzentrifugieren oder durch Umsolubilisieren. Die auf diese Weise erhaltenen Teilchen haben üblicherweise eine Teilchengröße zwischen 50 und 200 µm.

Die verkapselte Form bietet sich an, um die Enzyme vor anderen Bestandteilen, wie beispielsweise Bleichmitteln, zu schützen oder um eine kontrollierte Freisetzung (controlled release) zu ermöglichen. Je nach der Größe dieser Kapseln wird nach Milli-, Mikro- und Nanokapseln unterschieden, wobei Mikrokapseln für Enzyme besonders bevorzugt sind. Solche Kapseln werden beispielsweise mit den Patentanmeldungen WO 97/24177 und DE 19918 267 offenbart. Eine weitere mögliche Verkapselungsmethode besteht darin, daß die für die Verwendung in Wasch- oder Reinigungsmitteln geeigneten Enzyme, ausgehend von einer Mischung der Enzymlösung mit einer Lösung oder Suspension von Stärke oder einem Stärkederivat, in Stärke, beziehungsweise dem Stärkederivat verkapselt werden. Ein solches Verkapselungsverfahren wird mit der deutschen Anmeldung DE 199 56 382 mit dem Titel "Verfahren zur Herstellung von mikroverkapselten Enzymen" beschrieben.

Eine andere Möglichkeit, ein festes erfindungsgemäßes Mittel zur Verfügung zu stellen, ist das Verpressen oder Kompaktieren zu Tabletten. Solche Tabletten können ein- oder mehrphasig sein. Damit bietet auch diese Darreichungsform die Möglichkeit, ein festes erfindungsgemäßes Mittel mit zwei festen Phasen vorzulegen. Zur Herstellung von erfindungsgemäßen Mitteln in Tablettenform, die einphasig oder mehrphasig, einfarbig oder mehrfarbig sein können und/oder aus einer mehreren Schichten bestehen können, werden vorzugsweise alle Bestandteile -gegebenenfalls je einer Schicht - in einem Mischer miteinander vermischt und das Gemisch mittels herkömmlicher Tablettenpressen, beispielsweise Exzenterpressen oder Rundläuferpressen, mit Preßkräften im Bereich von etwa 50 bis 100 kN/cm², vorzugsweise bei 60 bis 70 kN/cm² verpreßt. Insbesondere bei mehrschichtigen Tabletten kann es von Vorteil sein, wenn mindestens eine Schicht vorverpreßt wird. Dies wird vorzugsweise bei Preßkräften zwischen 5 und 20 kN/cm², insbesondere bei 10 bis 15 kN/cm² durchgeführt. Vorzugsweise weist eine derart hergestellte Tablette ein Gewicht von 10 g bis 50 g, insbesondere von 15 g bis 40 g auf. Die Raumform der Tabletten ist beliebig und kann rund; oval oder eckig sein, wobei auch Zwischenformen möglich sind.

Flüssigen, gelförmigen oder pastösen erfindungsgemäßen Mitteln können die Enzyme, und auch ein erfindungswesentliches Protein ausgehend von einer nach dem Stand der Technik durchgeführten Proteingewinnung und Präparation in konzentrierter wäßriger oder nichtwäßriger Lösung beispielsweise in flüssiger Form, etwa als Lösung, Suspension oder Emulsion, aber auch in Gelform oder verkapselt oder als getrocknetes Pulver zugesetzt werden. Derartige erfindungsgemäße Wasch- oder Reinigungsmittel in Form von Lösungen in üblichen Lösungsmitteln werden in der Regel durch einfaches Mischen der Inhaltsstoffe hergestellt, die in Substanz oder als Lösung in einen automatischen Mischer gegeben werden können.

Eine Ausführungsform der vorliegenden Erfindung sind solche flüssigen, gelförmigen oder pastösen Mittel, denen ein erfindungswesentliches Protein und/oder eines der anderen enthaltenen Proteine und/oder einer der anderen enthaltenene Inhaltsstoffe in Form von Mikrokapseln zugesetzt worden ist. Besonders bevorzugt sind darunter solche mit Kapseln aus amylasesensitivem Material. Solch eine gemeinsame Verwendung von Amylase-sensitiven Materialien und dem erfindungswesentlichen amylolytischen Enzym in einem Wasch- oder Reinigungsmittel kann Synergieeffekte zeigen, etwa dergestalt daß das stärkespaltende Enzym die Spaltung der Mikrokapseln unterstützt und somit den Freisetzungsprozeß der verkapselten Inhaltsstoffe steuert, so daß deren Freisetzung nicht während der Lagerung und/oder nicht zu Beginn des Reinigungsvorgangs, sondern erst zu einem bestimmten Zeitpunkt erfolgt. Auf diesem Mechanismus können komplexe Wasch- und Reinigungsmittelsysteme mit verschiedensten Inhaltsstoffen und verschiedensten Kapseltypen beruhen, die besonders bevorzugte Ausführungsformen der vorliegenden Erfindung darstellen.

Ein vergleichbarer Effekt ist dann gegeben, wenn sich die Inhaltsstoffe des Wasch- oder Reinigungsmittels auf mindestens zwei unterschiedliche Phasen verteilen, beispielsweise zwei oder mehr feste, miteinander verbundene Phasen eines tablettenförmigen Wasch- oder Reinigungsmittels, oder verschiedene Granulate innerhalb desselben pulverförmigen Mittels. Zwei- oder Mehrphasenreiniger sind für die Anwendung sowohl in maschinellen Geschirrspülern als auch in Waschmitteln Stand der Technik. Die Aktivität eines amylolytischen Enzyms in einer früher aktivierten Phase ist Voraussetzung für die Aktivierung einer späteren Phase, wenn diese von einer Amylase-sensitiven Hülle oder Beschichtung umgeben ist oder das Amylase-sensitive Material einen integralen Bestandteil der festen Phase darstellt, bei dessen teilweiser oder vollständiger Hydrolyse die betreffende Phase desintegriert. Der Einsatz des erfindungswesentlichen Enzyms für diesen Zweck stellt somit eine bevorzugte Ausführungsform der vorliegenden Erfindung dar.

Die Inhaltsstoffe von Wasch- und Reinigungsmitteln vermögen sich geeigneterweise gegenseitig in ihrer Leistung zu unterstützen. Die synergistische Verwendung von Amylase und Farbübertragungsinhibitoren zur Steigerung der Reinigungsleistung wird beispielsweise mit der Anmeldung WO 99/63035 offenbart. Es ist auch bekannt, daß Polymere, die gleichzeitig als Cobuilder eingesetzt werden können, wie beispielsweise Alkyl-Poly-Glykoside, die Aktivität und die Stabilität von enthaltenen Enzymen stabilisieren und steigem können, so aus der Anmeldung WO 98/45396. Ebenso ist es möglich, daß auch eine erfindungswesentliche amylolytische Aktivität durch einen der übrigen, oben aufgeführten Bestandteile modifiziert, insbesondere stabilisiert und/oder gesteigert wird. Entsprechend abgestimmte Rezepturen für erfindungsgemäße Mittel stellen somit besonders bevorzugte Ausführungsformen der vorliegenden Erfindung dar. Dies gilt insbesondere dann, wenn dadurch die Wasch-, beziehungsweise Reinigungsleistung des Mittels gesteigert wird.

Auch in Verfahren zur Reinigung von Textilien oder von harten Oberflächen, die dadurch gekennzeichnet sind, daß in wenigstens einem der Verfahrensschritte ein erfindungsgemäßes amylolytisches Protein oder Derivat aktiv wird, wird der Gedanke der vorliegenden Erfindung verwirklicht.

Gerade maschinelle Reinigungsverfahren zeichnen sich durch mehrstufige Reinigungsprogramme aus, also daß verschiedene reinigungsaktive Komponenten zeitlich voneinander getrennt auf das Reinigungsgut aufgebracht werden. Solche Verfahren werden beispielsweise bei der Reinigung von gewerblichen Produktionsanlagen für Lebensmittel angewendet. Andererseits besitzen erfindungswesentliche Proteine aufgrund ihrer enzymatischen Aktivität selbst die Fähigkeit, kohlenhydrathaltige Anschmutzungen anzugreifen, und zwar auch in teilweiser oder völliger Abwesenheit von Detergenzien oder anderen für Wasch- oder Reinigungsmittel charakteristischen Inhaltsstoffen. Gemäß einer Ausführungsform der vorliegenden Erfindung kann somit auch ein maschinelles Verfahren zur Reinigung von Textilien oder von festen Stoffen gewählt werden, in welchem ein erfindungswesentliches Protein über einen gewissen Zeitraum hinweg ohne andere reinigungsaktive Komponenten auf die Anschmutzungen einwirkt.

Bevorzugte Ausführungsformen der vorliegenden Erfindung stellen alle Reinigungsverfahren dar, darunter manuelle, insbesondere aber maschinelle Reinigungsverfahren, die dadurch gekennzeichnet sind, daß in wenigstens einem der Verfahrensschritte ein erfindungswesentliches amylolytisches Protein oder Derivat aktiv wird. Das können sowohl Verfahren zur Reinigung von Textilien oder vergleichbaren Materialien, als auch solche zur Reinigung harter Oberflächen sein.

Wie in den Beispielen belegt, eignet sich die erfindungswesentliche α-Amylase aus *Bacillus sp*. A 7-7 (DSM 12368), wenn sie in zweckmäßige Wasch- oder Reinigungsmittel eingebunden ist, sowohl zur Steigerung der Waschleistung von maschinellen Waschmitteln für Textilien als auch zur Steigerung der Reinigungsleistung von maschinellen Geschirrspülmitteln.

Ebenso bevorzugte Ausführungsformen der vorliegenden Erfindung stellen somit alle Reinigungsverfahren dar, darunter manuelle, insbesondere aber maschinelle Reinigungsverfahren, die dadurch gekennzeichnet sind, daß in wenigstens einem der Verfahrensschritte ein erfindungsgemäßes Mittel eingesetzt wird. Das können sowohl Verfahren zur Reinigung von Textilien oder vergleichbaren Materialien, als auch solche zur Reinigung harter Oberflächen sein.

Als für Reinigungsverfahren, beispielsweise in gängigen Haushaltsgeschirrspülmaschinen oder Haushaltswaschmaschinen besonders geeignete Mengen an erfindungsgemäßem Protein oder Fragment wurden von 0,02 mg bis 400 mg des amylolytischen Proteins oder Fragments, bevorzugt von 0,01 mg bis 200 mg, insbesondere 0,02 mg bis 100 mg des amylolytischen Enzyms oder Fragments pro Anwendung ermittelt.

In einer möglichen Ausführungsform maschineller Verfahren zur Reinigung von Textilien oder festen Oberflächen haben sich für ein erfindungswesentliches Protein aktive Konzentrationen von 0,0005 mg bis 10 mg pro I Waschflotte, vorzugsweise von 0,005 mg bis 8 mg pro I Waschflotte als besonders geeignet herausgestellt. Sie stellen somit Ausführungsformen der vorliegenden Erfindung dar. In anderen geeigneten Ausführungsformen können sich davon deutlich abweichende Werte ergeben, wenn man berücksichtigt, daß für maschinelle Reinigungsverfahren Geräte in Gebrauch sind, die zwischen 5 und 70 I Waschflotte bei nahezu identischer Wasch-, beziehungsweise Spülmittelmenge umsetzen.

Auch die Verwendung eines erfindungsgemäßen Proteins oder eines erfindungsgemäßen Mittels stellt eine Ausführungsform der vorliegenden Erfindung dar. Sie kann maschinell oder auf sonstige, insbesondere manuelle Weise erfolgen. Dies betrifft die Reinigung von allen erdenklichen Materialien, insbesondere von Textilien oder von harten Oberflächen. Dabei kann das erfindungswesentliche Protein in eine Rezeptur aus anderen waschaktiven Substanzen eingebettet sein oder entsprechend seiner Natur auch weitgehend ohne solche Verbindungen erfolgen.

Eine entsprechende Ausführungsform stellt auch die Verwendung allein eines erfindungsgemäßen Proteins zur Reinigung von Textilien oder harten Oberflächen dar, insbesondere innerhalb eines mehrstufigen Reinigungsverfahrens.

Eine bevorzugte Ausführungsform stellen all jene Verwendungsmöglichkeiten dar, bei denen erfindungsgemäße Mittel zur Reinigung von Textilien oder von harten Oberflächen eingesetzt werden.

Eine bevorzugte Verwendung ist dadurch gekennzeichnet, daß pro Anwendung in einer Geschirrspülmaschine oder einer Waschmaschine, 0,02 mg bis 400 mg, bevorzugt von 0,01 mg bis 200 mg, insbesondere 0,02 mg bis 100 mg des amylolytischen Proteins oder Fragments eingesetzt werden.

Die Verwendung erfindungsgemäßer Proteine oder Derivate zur teilweisen oder vollständigen Auflösung von Schutzschichten um Bestandteile fester Wasch- oder Reinigungsmittel oder die Desintegration fester Phasen mit enthaltenen oder umgebenden Amylase-sensitiven Materialien, stellt ebenfalls eine Ausführungsform der vorliegenden Erfindung dar. Dies gilt auch für die Ausführungsformen, bei denen in einer dieser Phasen erfindungsgemäße amylolytische Proteine allein oder zusammen mit mindestens einem anderen reinigungsaktiven oder die Reinigungswirkung unterstützenden Wirkstoff enthalten sind. Insofern dient das amylolytische Protein der Aktivierung der eigenen oder anderer Phasen in einem aus mehr als einer Phase bestehenden Wasch- oder Reinigungsmittel. Besonders bevorzugt ist auch unter diesem Aspekt die Freisetzung der Inhaltsstoffe zur Herbeiführung eines reinigenden Effekts der Inhaltsstoffe auf harte Oberflächen oder ein textilartiges Material.

Die Verwendung des erfindungsgemäßen Enzyms, um die teilweise oder vollständige Auflösung von kohlenhydrathaltigen Kapseln, insbesondere Nano-, Mikro- oder Millikapseln in flüssigen, gelförmigen oder pastösen Mitteln herbeizuführen, stellt eine Ausführungsform der vorliegenden Erfindung dar, deren Bedeutung für einen kontrollierten Freisetzungsprozeß der verkapselten Bestandteile des Mittels oben bereits erörtert worden ist. Es handelt sich dann um eine besonders bevorzugte Ausführungsform dieser Erfindung, wenn die Freisetzung der Inhaltsstoffe zur Herbeiführung eines reinigenden Effekts der Inhaltsstoffe auf eine harte Oberfläche oder ein textilartiges Material erfolgt.

Eine weitere Ausführungsform stellt die Verwendung eines erfindungsgemäßen Proteins oder Derivats zur Behandlung von Rohmaterialien oder Zwischenprodukten in der Textilherstellung dar, insbesondere zum Entschlichten von Baumwolle.

Rohmaterialien und Zwischenprodukte der Textilherstellung, beispielsweise für solche auf Baumwollbasis, werden im Rahmen ihrer Herstellung und Weiterverarbeitung mit Stärke ausgerüstet, um eine bessere Verarbeitung zu ermöglichen. Dieses sowohl auf Game, auf Zwischenprodukte, als auch auf Textilien angewendete Verfahren nennt man Schlichten (sizing). Zur Entfernung der Schlichte, also der stärkehaltigen Schutzschicht (Entschlichten, desizing) sind erfindungsgemäße amylolytische Proteine geeignet.

Eine weitere Ausführungsform stellen Verfahren zur Stärkeverflüssigung, insbesondere zur Ethanolproduktion dar, die dadurch gekennzeichnet sind, daß darin ein erfindungsgemäßes Protein oder Derivat eingesetzt wird.

Zur Stärkeverflüssigung wird in Wasser oder Puffer gequollene Stärke mit amylolytischen Enzymen inkubiert, wodurch das Polysaccharid in kleinere Bestandteile, zuletzt überwiegend in Maltose gespalten wird. Erfindungsgemäße Enzyme werden bevorzugt für ein solches Verfahren oder einen Teilschritt davon eingesetzt, wenn sie sich aufgrund ihrer biochemischen Eigenschaften gut in ein entsprechendes Produktionsverfahren einpassen lassen. Dies kann beispielsweise dann der Fall sein, wenn sie in einem Schritt zusätzlich zu anderen Enzymen eingebracht werden sollen, die die gleichen Reaktionsbedingungen benötigen. Besonders bevorzugt sind erfindungsgemäße amylolytische Proteine, wenn gerade die von ihnen selbst gebildeten Produkte im Zentrum des Interesses stehen. Die Stärkeverflüssigung kann auch einen Schritt in einem mehrstufigen Verfahren zur Gewinnung von Ethanol oder davon abgeleiteten Folgeprodukten, beispielsweise Essigsäure darstellen.

Eine weitere Ausführungsform stellt die Verwendung eines erfindungsgemäßen Proteins oder Derivats zur Herstellung von linearen und/oder kurzkettigen Oligosacchariden dar.

Aufgrund ihrer enzymatischen Aktivität bilden erfindungsgemäße amylolytische Proteine aus stalkeähnlichen Polymeren nach kürzerer Inkubationszeit uberwiegend höhermolekulare Oligosaccharide, wie beispielsweise Maltohexaose, Maltoheptaose oder Maltooctaose. Nach längerer Inkubationszeit steigt unter den Reaktionsprodukten der Anteil niedrigerer Oligosaccharide, wie beispielsweise Maltose oder Maltotriose an. Bei besonderem Interesse an bestimmten Reaktionsprodukten können entsprechende Varianten erfindungsgemäßer Proteine verwendet und/oder die Reaktionsbedingungen entsprechend gestaltet werden. Dies ist insbesondere dann attraktiv, wenn es nicht auf reine Verbindungen, sondern auf Gemische ähnlicher Verbindungen ankommt, wie beispielsweise bei der Bildung von Lösungen, Suspensionen oder Gelen mit lediglich bestimmten physikalischen Eigenschaften.

Eine weitere Ausführungsform stellt die Verwendung eines erfindungsgemäßen Proteins oder Derivats zur Hydrolyse von Cyclodextrinen dar.

Cyclodextrine sind α-1,4-glycosidisch verknüpfte, cyclische Oligosaccharide, von denen die aus 6, 7 oder 8 Glucose-Monomeren, die α-, β-, beziehungsweise γ-Cyclodextrine (oder Cyclohexa-, -hepta-, beziehungsweise -octa-Amylosen), wirtschaftlich die größte Bedeutung besitzen. Sie können mit hydrophoben Gastmolekülen, wie beispielsweise Duftstoffen, Geschmacksstoffen oder pharmazeutischen Wirkstoffen, Einschlußverbindungen bilden, aus welchen die Gastmoleküle bei Bedarf wieder freigesetzt werden können. Solche Einschlußverbindungen sind je nach Einsatzgebiet der Inhaltsstoffe beispielsweise für die Herstellung von Nahrungsmitteln, die Pharmazie oder die Kosmetik, beispielsweise in entsprechenden Produkten für den Endverbraucher von Bedeutung. Die Freisetzung von Inhaltsstoffen aus Cydodextrinen stellt somit eine Verwendungsmöglichkeit für erfindungsgemäße Proteine dar.

Eine weitere Ausführungsform stellt die Verwendung eines erfindungsgemäßen Proteins oder Derivats zur Freisetzung von niedermolekularen Verbindungen aus Polysaccharidträgem oder Cyclodextrinen dar.

Aufgrund ihrer enzymatischen Aktivität können erfindungsgemäße amylolytische Proteine niedermolekulare Verbindungen auch aus anderen α-1,4-glycosidisch verknüpften Polysacchariden freisetzen. Dieses kann sich wie bei den Cyclodextrinen auf molekularer Ebene abspielen als auch an größeren Systemen, wie beispielsweise Inhaltsstoffen, die in Form von Mikrokapseln verkapselt sind. Beispielsweise Stärke ist ein im Stand der Technik etabliertes Material, um Verbindungen wie beispielsweise Enzyme, die in definierten Mengen in Reaktionsansätze eingebracht werden sollen, während der Lagerung einzukapseln. Der kontrollierte Freisetzungsprozeß aus derartigen Kapseln kann von erfindungsgemäßen amylolytischen Enzymen unterstützt werden.

Eine weitere Ausführungsform stellt die Verwendung eines erfindungsgemäßen Proteins oder Derivats zur Herstellung von Lebensmitteln und/oder Lebensmittelbestandteilen dar.

Ebenso stellt die Verwendung eines erfindungsgemäßen Proteins oder Derivats zur Herstellung von Tierfutter und/oder Tierfutterbestandteilen eine Ausführungsform dieses Erfindungsgegenstands dar.

Wo immer Stärke oder von Stärke abgeleitete Kohlenhydrate als Lebensmittel- oder Tiernahrungsbestandteil eine Rolle spielen, kann eine amylolytische Aktivität zur Herstellung dieser Artikel zum Einsatz kommen. Sie erhöht den Anteil von Mono-, oder Oligomeren gegenüber dem polymeren Zucker, was beispielsweise dem Geschmack, der Verdaubarkeit oder der Konsistenz des Lebensmittels zugute kommen kann. Dies kann zur Herstellung bestimmter Tierfutter erforderlich sein, beispielsweise aber auch bei der Herstellung von Fruchtsäften, Wein oder anderer Lebensmittel, wenn der Anteil polymerer Zucker verringert und der von süßen und/oder leichter löslichen Zuckern erhöht werden soll. Die weiter oben ausgeführte Verwendungsmöglichkeit zur Stärkeverflüssigung und/oder Ethanolproduktion kann als großtechnische Variante dieses Prinzips angesehen werden.

Amylasen wirken darüberhinaus auch dem unter Altbacken-Werden bekannten Geschmacksverlust von Backwaren (Anti staling-Effekt) entgegen. Dafür werden sie geeigneterweise dem Teig vor dem Backen zugesetzt. Bevorzugte Ausführungsformen der vorliegenden Erfindung sind also solche, bei denen erfindungsgemäße Proteine zur Herstellung von Backwaren verwendet werden.

Eine weitere Ausführungsform stellt die Verwendung eines erfindungsgemäßen Proteins oder Derivats zur Auflösung stärkehaltiger Klebeverbindungen dar.

Ebenso stellen temporäre Klebeverfahren, die dadurch gekennzeichnet sind, daß dann ein erfindungsgemäßes Protein oder Derivat eingesetzt wird, eine Ausführungsform dieses Erfindungsgegenstands dar.

Neben anderen Naturstoffen wird auch Stärke bereits seit Jahrhunderten als Bindemittel in der Papierherstellung und dem Verkleben unterschiedlicher Papiere und Pappen verwendet. Dies betrifft beispeilsweise Graphiken und Bücher. Im Laufe langer Zeiträume können solche Papiere unter ungünstigen Einflüssen, wie beispielsweise Feuchtigkeit, Wellen aufwerfen oder brechen, was bis zu deren völliger Zerstörung führen kann. Bei der Restaurierung solcher Papiere und Pappen kann das Auflösen der Klebeschichten erforderlich sein und durch Verwendung eines erfindungsgemäßen amylolytischen Proteins erheblich erleichtert werden.

Pflanzliche Polymere wie Stärke oder Cellulose und deren wasserlösliche Derivate werden unter anderem als Klebstoffe oder Kleister verwendet. Dafür müssen sie in Wasser zunächst quellen und nach Aufbringen auf dem Klebegut trocknen, wodurch dieses am Untergrund fixiert wird. Das erfindungsgemäße Enzym kann solch einer wäßrigen Suspension zugesetzt werden, um die Klebeeigenschaften des entstehenden Kleisters zu beeinflussen. Es kann aber auch stattdessen oder zusätzlich zu dieser Funktion dem Kleister zugesetzt werden, um nach dem Antrocknen für lange Zeit, beispielsweise einige Jahre, inaktiv auf dem Klebegut zu verharren. Gezieltes Ändern der Umgebungsbedingungen, beispielsweise durch Anfeuchten, kann dann dazu verwendet werden, um das Enzym zu einem späteren Zeitpunkt zu aktivieren und damit ein Auflösen des Kleisters herbeizuführen. Auf diese Weise ist das Klebegut leichter wieder vom Untergrund abzulösen. In diesem Verfahren fungiert das erfindungsgemäße Enzym aufgrund seiner amylolytischen Aktivität als scheidendes Agens in einem temporären Klebeverfahren oder als sogenannter "Schalter" zum Ablösen des Klebeguts.

### Beispiele

### Beispiel 1

Unter den Kandidaten eines mikrobiologischen Screenings auf amylasebildende Mikroorganismen mit dem Auswahlkriterium Wachstum und Hofbildung auf Agarplatten mit Stärke als einziger Kohlenstoff-Quelle befand sich auch der Bacillus-Stamm *Bacillus sp.* (RNA-Gruppe VI, alcaliphil) A 7-7, der bei der DSMZ hinterlegt worden ist. (DSM ID 98-587, Hinterlegung DSM 12368).

Die Anzucht erfolgte in YPSS-Medium, enthaltend 15 g/l lösliche Stärke, 4 g/l Hefeextrakt, 1 g/l K₂HPO₄ und 0,5 g/l MgSO₄ x7 H₂O. Der pH-Wert wurde nach dem Autoklavieren mit 20%iger Natriumcarbonatlösung auf 10,3 eingestellt. Jeweils 25 ml Medium wurden in sterile 100ml-Erlenmeyerkolben mit Schikane eingefüllt und mit einer Kultur *Bacillus sp*. A 7-7 (DSM 12368) beimpft, die auf einer YPSS-Agarplatte gewachsen war. Die Anzucht erfolgte bei 30°C über 48 h unter Schütteln bei 200 rpm.

### Beispiel 2

Ober folgende Aufreinigungsschritte wurde aus dem Kulturmedium ein singuläres Enzym erhalten: Fällung des Kulturüberstands mit Ethanol; Aufnahme des Proteinpellets in 50 mM Tris/HCl-Puffer, pH 8,5; Dialyse gegen 50 mM Tris/HCl-Puffer, pH 8,5; Kationenaustauschchromatographie an Fast-flow-S-Sepharose® (Fa. Pharmacia-Amersham Biotech, Freiburg) mit 50 mM Tris/HCl-Puffer, pH 8,5 als Eluens; Umpufferung des Amylase-haltigen Durchbruchs gegen 20 mM Glycin/NaOH-Puffer, pH 10, über PD10®-Säulen der Firma Pharmacia-Amersham Biotech; Anionenaustauschchromatographie über Mono-Q® (Fa. Pharmacia-Amersham Biotech) unter Elution mit demselben Puffer mit einem ansteigenden Salzgradienten von 0 bis 1M NaCl. Die Amylase eluierte bei ca. 0,25 M NaCl.

Aus 250 ml Kulturmedium wurden auf diese Weise, wie über die BCA-Methode (Bicinchoninsäure; 2,2'-Bichinolyl-4,4'-dicarbonsäure) ermittelt wurde, 2,6 mg eines gemäß SDS-Gelektrophorese und Silber-Färbung reinen Proteins gewonnen.

Bei denaturierender SDS-Polyacrylgelelektrophorese in - einem 8-25%igem Gel, im PHAST®-System der Fa. Pharmacia-Amersham und bei Vergleich mit relevanten Größenmarkern weist das amylolytische Enyzm aus *Bacillus sp.* A 7-7 (DSM 12368) ein Molekulargewicht von 58 kD auf.

Gemäß isoelektrischer Fokussierung von pH 3 bis 9 im PHAST®-System der Fa. Pharmacia-Amersham liegt sein isoelektrischer Punkt bei 6,0.

Die amylolytische Aktivität des gereinigten Enzyms wurde mithilfe der sogenannten DNS-Methode, -also unter Verwendung von Dinitrosalicylsäure bestimmt. Dafür werden die durch das Enzym bei der Hydrolyse von Stärke freigesetzten Oligosaccharide, Disaccharide und Glucoseeinheiten durch Oxidation der reduzierenden Enden mit Dinitrosalicysäure (DNS) nachgewiesen. Die Intensität der Farbentwicklung ist dabei proportional zur Anzahl der entstandenen Spaltprodukte. Dieser Test wird folgendermaßen durchgeführt: Nach einer Inkubation von 50 µl Enzymlösung mit 100 µl 1 % löslicher Stärke in Tris/Maleat-Puffer pH 6.5 (12,11 g Tris + 11,61 g Maleinsäure ad 1 l, pH mit 0.2 N NaOH eingestellt) bei 50°C für 15 min werden die reduzierten Saccharide mit 300 µl DNS-Lösung (8.8 g Dinitrosalicylsäure, 915 ml destilliertes Wasser, 250 g K-Na-Tartrat, 334 ml 4.5% NaOH, 6.3 g Natriumdisulfit) bei 100°C für 20 min oxidiert. Nach Abkühlung im Eisbad wird die Absorption photometrisch bei 540 nm gegen einen Blank-Wert detektiert. Die Kalibrierung des Assays erfolgt über eine Maltose-Konzentrationsreihe. Die Aktivität wird angegeben in µmol reduzierende Zucker (bezogen auf Maltose) pro min und ml.

Das beispielgemäße Protein weist nach diesem Test eindeutig amylolytische Aktivität auf. Im Folgenden dient die auf diese Weise bestimmbare Aktivität als Parameter für die Stabilität des Enzyms unter jeweils verschiedenen Bedingungen.

Die Temperaturstabilität des Enzyms wurde jeweils bei 10minütiger Inkubation bei einem pH-Wert von 10 gemessen. Bei Raumtemperatur, 30°C ,40°C und 50°C liegt die Aktivität bei mindestens 85 %. Bei 40°C hat die Amylase ihr Maximum von 90 % Restaktivität. Bei 60°C hat das Enzym 50 % Aktivität. Bei Temperaturen von mehr als 60°C verliert es stark an Aktivität, weist aber bei 80-90°C immer noch 10 % Restaktivität auf.

Das amylolytische Enyzm aus *Bacillus sp.* A 7-7. (DSM 12368) ist bei pH-Werten zwischen 5 und 12, wenn es jeweils für 10 min bei 40°C inkubiert wird, weitgehend stabil. Bei pH-Werten von 8 bis 9 ist die Amylase bis zu 100 % stabil. Bei pH-Werten darunter und darüber fällt die Aktivität langsam ab; das Enzym verfügt bei pH 5 und pH 12 aber immer noch über 65% Restaktivität.

Zur weiteren Charakterisierung wurde die Beeinträchtigung der enzymatischen Aktivität durch möglicherweise störende Faktoren wie Protease oder Detergenzien untersucht: Nach Einwirken von 10 HPE/ml der Protease Savinase® (Novo Nordisk A/S, Bagsværd, Dänemark; die HPE-Einheiten sind bestimmbar nach der in Tenside 7 (1970), S. 125-132 angegebenen Methode) und 0,1% SDS bei pH 10 für 15 Minuten und 50°C zeigt das Enzym 74% Restaktivität. Nach Einwirken von 0,1 % SDS unter gleichen Bedingungen (pH 10, 15 Minuten, 50°C) weist das Enzym 98% Restaktivität auf. Und ebenfalls unter diesen Bedingungen (pH 10, 15 Minuten, 50°C) besitzt es in Gegenwart von 3 mM EDTA noch 10% Restaktivität und in Gegenwart von 1 mM EDTA noch 65% Restaktivität.

### Beispiel 3

Alle molekularbiologischen Arbeitsschritte folgen Standardmethoden, wie sie beispielsweise in Handbüchem wie dem von Fritsch, Sambrook und Maniatis "Molecular cloning: a laboratory manual", *Cold Spring Harbour Laboratory Press*, New York, 1989, angegeben sind.

Zunächst wurde zur Bestimmung intemer Aminosäure-Sequenzen das gemäß Beispiel 1 gewonnene und gemäß Beispiel 2 gereinigte Protein mit Ethanol gefällt und mittels denaturierender SDS-Polyacrylgelelektrophorese aufgetrennt. Nach Ausschneiden der entsprechenden Bande aus dem SDS-Polyacrylamid-Gel wurde *in situ* tryptisch verdaut, die resultierenden Peptide mittels HPLC getrennt und durch Edman-Abbau und MALDI-TOF analysiert. Aus den zahlreichen auf diese Weise erhaltenen tryptischen Fragmenten wurden folgende beiden, D1 und D6, ausgewählt:

Nach deren (links angegebenen) Aminosäuresequenzen wurden die entsprechenden degenerierten PCR-Primer konstruiert. Deren Nukleotidsequenzen sind rechts abgegeben (N steht für beliebige Basen; H steht für A oder C oder T; R steht für A oder G; D steht für A, G oder T).

Aus standardmäßig präparierter chromosomaler DNA von *Bacillus sp.* A7-7 (DSM 12368) als Matrize konnte mit diesem Primer-Paar in einer Standard-PCR ein ca. 1.000 bp großes Fragment amplifiziert werden. Dieses PCR-Fragment wurde zur Lagerung in den Vektor pGEM-Teasy® (Fa. Promega, Madison, Wisconsin, USA) kloniert.

### Beispiel 4

Das gereinigte PCR-Fragment wurde nach Anleitung mit dem nichtradioaktiven DIG-High-Prime® Labeling-Kit der Fa. Boehringer Mannheim (Deutschland; Produkt-Nr. 1745832) als DNA-Sonde markiert. Für die anschließende Hybridisierung wurde chromosomale DNA des Stammes *Bacillus sp.* A7-7 (DSM 12368) mit dem Restriktionsenzym Xba I gespalten und über ein 0,9%iges Agarosegel aufgetrennt. Die DNA konnte mit Hilfe eines Vakuum-Blotters auf eine positiv geladene Nylonmembran (Fa. Roche, Mannheim) übertragen werden. Die DNA-Hybridisierung und immunologische Detektion des als Sonde verwendeten PCR-Fragments erfolgte anhand der Vorschrift des DIG-High-Prime® Labeling-and-Detection-Starter Kits I® der Fa. Boehringer Mannheim. Es stellte sich heraus, daß sich das gesuchte Gen in einem scheinbar ca. 3.000 - 4.000 bp großen DNA-Abschnitt befindet. Die Fragmente dieser Größenordnung wurden über ein 0,9%iges Agarosegel aufgereinigt und anschließend in den mit den Restriktionsenzymen Xba I und Nhe I geschnittenen Vektor pCB56C (aus*B*. *subtilis* DB104; beschrieben in der Anmeldung WO 91/02792) ligiert. Nach Transformation in einen Amylase-negativen *Bacillus subtilis*-Stamm konnten Amylasepositive Klone auf Stärke-haltigen Agarplatten über Hofbildung identifiziert werden. Ein repräsentatives Isolat trug das gewünschte Insert.

### Beispiel 5

Die Sequenzierung des erhaltenen Plasmids erfolgte nach der Kettenabbruchmethode nach Standardverfahren. Das Plasmid enthielt ein Insert mit einer Größe von 2.015 bp. Darauf liegt das 1.545 bp umfassende Gen für das interessierende Enzym, welches unter SEQ ID NO. 1 im Sequenzprotokoll zur vorliegenden Anmeldung angegeben ist. Dem entspricht ein Polypeptid von 516 Aminosäuren, dessen Sequenz in SEQ ID NO. 2 im Sequenzprotokoll angegeben ist. Das aus dieser Aminosäuresequenz abgeleitete Molekulargewicht beträgt 59 kD, beziehungsweise für das durch Abspalten des 31 Aminosäuren umfassenden Signalpeptids erhaltene reife Protein 55,5 kD.

Sequenzvergleiche nach der BLAST-Methode (S. F. Altschul et al., *Nucl.* Acids Res., 25 (1997), S. 3389-3402), durchgeführt am 17. 3. 2000, charakterisieren das Enzym alsα-Amylase. Die Homologie dieses Proteins zu bekannten Proteinen liegt, wie folgende Tabelle 2 zeigt, auf Proteinebene zwischen 71 und 95 % Identität.

**Tabelle 2:**

| Homologie des erfindungsgemäßen Enzyms aus *Bacillus sp*. A7-7 (DSM 12368) zu den nächstähnlichen und weiteren repräsentativen Proteinen; angegeben in % Identität auf Protein-Ebene. Mit ID werden die Einträge bei der Swiss-Prot-Datenbank (Genf, Schweiz) bezeichnet. | | |
|---|---|---|
| **Organismus** | **ID** | **Identität (%)** |
| ***B. alcalophilus*** | P19571 | 95 |
| ***B. alcalophilus*** | WO 96/23873 | 91 |
| ***B. licheniformis*** | P06278 | 72 |
| ***B. amyloliquefaciens*** | P00692 | 72 |
| ***B. stearothermophilus*** | P06297 | 71 |

Ein Alignment mit repräsentativen Proteinen ist in Figur 1 dargestellt. Bei allen handelt es sich um α-Amylasen, so daß aufgrund der weitgehenden Übereinstimmungen davon ausgegangen werden muß, daß es sich auch bei dem erfindungsgemäßen Enzym um eine α-Amylase handelt. Dies deckt sich mit der ursprünglichen Gewinnung des Gens aus einem stärkeabbauenden Mikroorganismus (Beispiel 1) und der Feststellung der amylolytischen Aktivität einer Transformante mit dem insertragenden Plasmid (Beispiel 4). Die Eigenschaften des aus diesem Produktionsstamm erhältlichen amylolytischen Proteins stimmen mit denen des Wildtypstammes (Beispiel 2) überein. Solch ein Produktionsstamm kann gemäß Beispiel 4 hergestellt werden.

### Beispiel 6

Baumwolltextilien wurden standardisiert mit den vier verschiedenen Anschmutzungen A (Mousse au chocolat), B (Haferflocken mit Kakao), C (Haferflocken mit Kakao und wenig Milch) und D (Kartoffelstärke) versehen und anhand des auf diese Weise präparierten Materials verschiedene Waschmittelrezepturen launderometrisch auf ihre Waschleistung hin untersucht. Dafür wurde jeweils ein Flottenverhältnis von 1:12 eingestellt und für 30 min bei einer Temperatur von 30°C gewaschen. Die Dosierung lag bei 5,88 g des jeweiligen Waschmittels pro I Waschflotte. Die Wasserhärte betrug 16° deutscher Härte.

Als Kontroll-Waschmittel diente für A, B und C eine Waschmittel-Basis-Rezeptur folgender Zusammensetzung (alle Angaben in Gewichts-Prozent): 4 % lineares Alkylbenzolsulfonat (Natrium-Salz), 4 % C₁₂-C₁₈-Fettalkoholsulfat (Natrium-Salz), 5,5 % C₁₂-C₁₈-Fettalkohol mit 7 EO, 1 % Natrium-Seife, 11 % Natriumcarbonat, 2,5 % amorphes Natriumdisilikat, 20 % Natriumperborat-Tetrahydrat, 5,5 % TAED, 25 % Zeolith A, 4,5 % Polycarboxylat, 0,5 % Phosphonat, 2,5 % Schauminhibitorgranulat, 5 % Natriumsulfat, 1 % Proteasegranulat, Rest: Wasser, optischer Aufheller, Parfüm, Salze. Sie wurde für die verschiedenen Versuchsreihen mit verschiedenen Amylasen versetzt, so daß sich jeweils eine Endkonzentration von 44 TAU an amylolytischer Aktivität pro I Waschflotte ergab.

Zur Bestimmung der amylolytischen Aktivität in TAU wird ein modifiziertes p-Nitrophenylmaltoheptaosid verwendet, dessen endständige Glucoseeinheit durch eine Benzylidengruppe blockiert ist; dieses wird durch Amylase zu freiem p-Nitrophenyl-Oligosaccharid gespalten, welches seinerseits mittels der Hilfesenzyme Glucoamylase und alpha-Glucosidase zu Glucose und p-Nitrophenol umgesetzt wird. Damit ist die Menge an freigesetztem p-Nitrophenol der Amylase-Aktivität proportional. Die Messung erfolgt beispielsweise mit dem Quick-Start®-Testkit der Fa. Abbott, Abott Park, Illinois, USA. Die Absorptionszunahme (405 nm) im Testansatz wird bei 37°C über 3 min gegen einen Blank-Wert mittels Photometer detektiert. Die Kalibrierung erfolgt über einen Enzymstandard von bekannter Aktivität (zum Beispiel Maxamyl®/Purastar® 2900 der Firma Genencor mit 2900 TAU/g). Die Auswertung erfolgt mittels Auftragung der Absorptionsdifferenz dE (405 nm) pro min gegen die Enzymkonzentration des Standards.

Mit dem erfindungswesentlichen amylolytischen Enzym aus *Bacillus sp.* A 7-7 (DSM 12368) wurden Termamyl®, Duramyl® und BAN® (Hersteller jeweils: Novo Nordisk A/S, Bagsværd, Dänemark) verglichen. Für die Anschmutzung D wurde dieselbe Basis-Rezeptur verwendet, allerdings ohne Protease, und wie A - C als Kontrolle verwendet, beziehungsweise mit Amylasen versetzt.

Im vorliegenden Beispiel wurde der Weißheitsgrad der Textilien im CIELAB-System mit dem Meßgerät Minolta CR 310 vor und nach dem Waschen im Vergleich zu einem Standard gemessen, der auf 100 % normiert wurde. Die Differenzen aus den erhaltenen Werten werden für die jeweiligen Versuche in folgender Tabelle 3 zusammengestellt. Angegeben sind die Mittelwerte aus jeweils 5 Messungen. Sie erlauben einen unmittelbaren Rückschluß auf den Beitreg des enthaltenen Enzyms auf die Waschleistung des verwendeten Mittels.

**Tabelle 3:**

| **Basis-Waschmittel mit** | **A** | **B** | **C** | **D** |
|---|---|---|---|---|
| | | | | |
| **erfindungswesentl. Amylase** | 29,3 | 26,0 | 20,9 | 15,2 |
| **Termamyl®** | 25,9 | 22,7 | 17,4 | 12,3 |
| **Duramyl®** | 28,6 | 23,4 | 20,2 | 14,3 |
| **BAN®** | 22,5 | 22,0 | 17,0 | 13,2 |
| **Kontrolle ohne Amylase** | 22,9 | 22,2 | 11,9 | 10,0 |
| Standardabw. | 1,3 | 0,6 | 1,4 | 2,2 |

Man erkennt, daß die α-Amylase aus *Bacillus sp.* A 7-7 (DSM 12368) an der Anschmutzung B eindeutig bessere Waschleistungen als jedes der drei Referenzenzyme erbringt. Bei den übrigen Anschmutzungstypen zeigt sie im Rahmen der Fehlergrenze leicht verbesserte Waschleistungen, die immer deutlich über den Vergleichswerten ohne amylolytisches Enzym liegen. Dieses Ergebnis überzeugt umso mehr, als in allen untersuchten Waschmittelrezepturen ein Bleichmittel enthalten ist, auf welches gerade Wildtyp-Enzyme im allgemeinen sehr empfindlich reagieren.

### Beispiel 7

Baumwoll-Textilien wurden standardisiert mit den Anschmutzungen B (Haferflocken mit Kakao) und C (Haferflocken mit Kakao und wenig Milch) angeschmutzt. Die launderometrische Untersuchung erfolgte wie in Beispiel 1, unter Verwendung einer anderen Waschmittel-Basis-Rezeptur, nämlich jeweils in Gewichts-Prozent: 14 % Na-Alkylbenzolsulfonat, 6 % Na-Fettalkoholsulfonat, 6 % 7-fach ethoxylierter C₁₂-C₁₈-Fettalkohol, 1 % Seife, 25 % Zeolith Na-A, 10 % Na-Carbonat, 5 % polymeres Polycarboxylat (Sokalan® CP5), 11 % Trinatriumcitrat-dihydrat, 4 % Citronensäure, 1 % teilchenförmig konfektionierter Schauminhibitor, 1 % Proteasegranulat, 5 % Natriumsulfat, Rest: Wasser und Salze. Diese Basis-Rezeptur wurde für die verschiedenen Versuchsreihen mit verschiedenen Amylasen versetzt, so daß sich jeweils eine Endkonzentration von 33,5 TAU an amylolytischer Aktivität pro I Waschflotte ergab; wie sie nach der in Beispiel 1 dargestellten Methode bestimmt werden kann. Mit dem erfindungswesentlichen amylolytischen Enzym aus *Bacillus sp.* A 7-7 (DSM 12368) wurden Termamyl®, Duramyl® und BAN® (Hersteller jeweils: Novo Nordisk A/S, Bagsværd, Dänemark) verglichen. Die Dosierung lag bei 4,45 g des jeweiligen Waschmittels pro I Waschflotte.

Nach dem Waschen wurde der Weißheitsgrad der gewaschenen Textilien wie in dem vorangegangenen Beispiel bestimmt. Die jeweils erhaltenen Differenzwerte werden in folgender Tabelle 4 zusammengestellt. Es handelt sich jeweils um die Mittelwerte aus 5 Messungen, welche wiederum einen unmittelbaren Rückschluß auf den Beitrag des jeweiligen Enzyms auf die Waschleistung des Mittels zulassen.

**Tabelle 4:**

| **Basis-Waschmittel mit** | **B** | **C** |
|---|---|---|
| | | |
| **erfindungswesentl. Amylase** | 30,6 | 17,5 |
| **Termamyl®** | 29,3 | 15,0 |
| **Duramyl®** | 29,2 | 16,7 |
| **BAN®** | 28,9 | 15,6 |
| **Kontrolle ohne Amylase** | 28,5 | 14,5 |
| Standardabw. | 0,6 | 1,2 |

Man erkennt, daß die α-Amylase aus *Bacillus sp.* A 7-7 (DSM 12368) in dieser bleichmittelfreien Waschmittel-Rezeptur an den Anschmutzungen B und C bessere Waschleistungen als jedes der Referenzenzyme erbringt.

### Beispiel 8

Baumwoll-Textilien wurden standardisiert mit zwei verschiedenen Typen kommerziell erhältlicher Arten Milchkakao (E und F) angeschmutzt. Damit erfolgte die launderometrische Untersuchung wie in Beispiel 1 beschrieben. Als Kontroll-Waschmittel diente die Waschmittel-Basis-Rezeptur des Beispiels 2, jedoch ohne Protease. Sie wurde für die verschiedenen Versuchsreihen wie in Beispiel 2 mit den verschiedenen Amylasen versetzt und in gleicher Dosierung eingesetzt.

Nach dem Waschen wurde der Weißheitsgrad der gewaschenen Textilien im Vergleich zu dem von Bariumsulfat gemessen, der auf 100 % normiert wurde. Die Messung erfolgte an einem Spektrometer Datacolor SF500-2 bei 460 nm (UV-Spenfilter 3), 30 mm Blende, ohne Glanz, Lichtart D65, 10°, d/8°. Die erhaltenen Ergebnisse werden als % Remission, das heißt als Prozentangaben im Vergleich zu Bariumsulfat in folgender Tabelle 4 zusammengestellt. Es ist dort auch der jeweilige Anfangswert angegeben. Angegeben sind die Mittelwerte aus jeweils 5 Messungen. Sie erlauben einen unmittelbaren Rückschluß auf den Beitrag des enthaltenen amylolytischen Enzyms auf die Waschleistung des verwendeten Mittels.

**Tabelle 5:**

| **Basis-Waschmittel mit** | **E** | **F** |
|---|---|---|
| | | |
| **erfindungswesentl. Amylase** | 70,3 | 41,0 |
| **Termamyl®** | 67,3 | 39,7 |
| **Duramyl®** | 68,3 | 40,5 |
| **BAN®** | 68,7 | 39,8 |
| **Kontrolle ohne Amylase** | 61,1 | 31,4 |
| Anfangswert | 21,1 | 25,0 |
| Standardabw. | 1,0 | 1,2 |

Man erkennt, daß die α-Amylase aus *Bacillus sp.* A 7-7 (DSM 12368) in dieser Rezeptur in im Fall von E eine eindeutig bessere Waschleistung als alle getesteten Referenzenzyme erbringt und im Fall von F eine vergleichbare.

### Beispiel 9

Für anwendungsorientierte Reinigungsversuche wurden Gefäße mit harten, glatten Oberflächen standardisiert mit folgenden Anschmutzungen versehen: A (DIN-Haferflocken), B (in Wasser gequollene Haferflocken) und C (Mix-Stärke), und bei 45 °C mit dem Normalprogramm einer Haushaltsgeschirrspülmaschine Typ Miele® G 575 gespült. Pro Spülgang wurden 20 g Spülmittel verwendet; die Wasserhärte betrug 16° deutscher Härte.

Als Spülmittel diente folgende Basis-Rezeptur (alle Angaben jeweils in Gewichts-Prozent): 55 % Natriumtripolyphosphat (berechnet als wasserfrei), 4 % amorphes Natriumdisilikat (berechnet als wasserfrei), 22 % Natriumcarbonat, 9 % Natriumperborat, 2 % TAED, 2 % nichtionisches Tensid, 1,4 % Proteasegranulat, Rest: Wasser, Farbstoffe, Parfüm. Diese Basis-Rezeptur wurde für die verschiedenen Versuche mit verschiedenen Amylasen versetzt, nämlich Termamyl®, Duramyl®, BAN® (Hersteller jeweils: Novo Nordisk A/S, Bagsværd, Dänemark) oder dem erfindungswesentlichen amylolytischen Enzym aus *Bacillus sp.* A 7-7 (DSM 12368), in wirksamen Mengen von jeweils, wie gemäß der in Beispiel 1 angegebenen Methode bestimmt, 150 TAU an amylolytischer Aktivität pro Reinigungsgang.

Nach dem Spülen wurde der Abtrag der Anschmutzung A nach Anfärbung mit lod mittels der lod-Stärke-Reaktion visuell auf einer Skala von 0 (= unverändert, das heißt sehr starke Anschmutzung) bis 10 (= keinerlei Anschmutzung erkennbar) benotet. Der Abtrag der Anschmutzungen B und C wurde gravimetrisch in Prozent ermittelt. Dafür wurde die Differenz aus dem Gewicht des verunreinigten und anschließend gespülten Gefäßes und dem Anfangsgewicht des Gefäßes in Relation zu der Gewichtsdifferenz des nicht gespülten Gefäßes zum Anfangsgewicht gesetzt. Diese Relation kann als Prozent Abtrag angesehen werden.

Die erhaltenen Ergebnisse werden in folgender Tabelle 6 zusammengestellt. Angegeben sind dort die Mittelwerte aus jeweils neun Messungen für A und B, beziehungsweise 18 Messungen für C. Sie erlauben einen unmittelbaren Rückschluß auf den Beitrag des enthaltenen Enzyms auf die Reinigungsleistung des verwendeten Mittels.

**Tabelle 6:**

| **Basis-Waschmittel mit** | **A** | **B** | **C** |
|---|---|---|---|
| | | % Abtrag | % Abtrag |
| **erfindungswesentl. Amylase** | 6,2 | 91,2 | 95,8 |
| **Termamyl®** | 3,7 | 53,9 | 15,7 |
| **Duramyl®** | 2,2 | 78,8 | 35,5 |
| **BAN®** | 3,7 | 69,1 | 34,2 |
| **Kontrolle ohne Amylase** | 4,3 | 31,7 | 0,6 |

Diese Ergebnisse zeigen, daß die α-Amylase aus *Bacillus sp.* A 7-7 (DSM 12368) hinsichtlich ihrer Reinigungsleistung in maschinellen Geschirrspülmitteln an allen drei Anschmutzungen bei 45°C Spültemperatur allen anderen getesteten Amylasen überlegen ist.

### Beispiel 10

Gefäße mit harten, glatten Oberflächen wurden mit denselben Anschmutzungen wie im vorangegangenen Beispiel versehen und bei 55°C gespült. Die Reinigungsbedingungen und die Rezeptur des verwendeten Mittel entsprachen ebenfalls denen des vorangegangenen Beispiels.
Nach dem Spülen wurde der Abtrag der Anschmutzung A wie in Beispiel 4 visuell über die lod-Stärke-Reaktion ermittelt und auf einer Skala von 0 bis 10 bewertet; und der Abtrag der Anschmutzungen B und C wurde ebenfalls wie in Beispiel 4 gravimetrisch in Prozent ermittelt. Die erhaltenen Ergebnisse werden in folgender Tabelle 6 zusammengestellt. Angegeben sind dort die Mittelwerte aus jeweils 9 Messungen für A, 10 für B, und 18 Messungen für C. Sie erlauben einen unmittelbaren Rückschluß auf den Beitrag des enthaltenen Enzyms auf die Reinigungsleistung des verwendeten Mittels.

**Tabelle 7:**

| **Basis-Waschmittel mit** | **A** | **B** | **C** |
|---|---|---|---|
| | | % Abtrag | % Abtrag |
| **erfindungswesentl. Amylase** | 8,3 | 97 | 99 |
| **Termamyl®** | 6,7 | 95 | 53 |
| **Duramyl®** | 6,9 | 95 | 89 |
| **BAN®** | 6,2 | 92 | 77 |
| **Kontrolle ohne Amylase** | 5,3 | 71 | 27 |

Diese Ergebnisse zeigen, daß die α-Amylase aus *Bacillus sp.* A 7-7 (DSM 12368) hinsichtlich ihrer Reinigungsleistung in maschinellen Geschirrspülmitteln an allen drei Anschmutzungen auch bei 55°C Spültemperatur allen anderen getesteten Amylasen überlegen ist.

### Beschreibung der Figuren

**Figur 1:** Alignment des erfindungsgemäßen amylolytischen Enzyms aus *Bacillus sp.*
A 7-7 (DSM 12368) mit den drei nächstähnlichen Amylasen.
Darin bedeuten:
1. Amylase aus *Bacillus sp.* A7-7 (DSM 12368)
2. Reife Amylase aus *Bacillus sp.* # 707 gemäß: Tsukamoto, Kimura, Ishii, Takano und Yamane, *Biochem. Biophys. Res. Commun*. 151 (1988), S. 25-31
3. Amylase der SEQ ID NO. 1 aus der Anmeldung WO 96/23873
4. Amylase der SEQ ID NO. 2 aus der Anmeldung WO 96/23873

## Patentansprüche

1. Amylolytisches Protein mit einer Aminosäuresequenz, die zu der in SEQ ID NO. 2 angegebenen Aminosäuresequenz über die ganze Länge mindestens zu 96 % identisch ist.

2. Amylolytisches Protein mit einer Aminosäuresequenz, die zu der in SEQ ID NO. 2 angegebenen Aminosäuresequenz über die ganze Länge mindestens zu 98 % identisch ist.

3. Amylolytisches Protein mit einer Aminosäuresequenz, die zu der in SEQ ID NO. 2 angegebenen Aminosäuresequenz in den Positionen 32 bis 516 über die ganze Länge mindestens zu 96 % identisch ist.

4. Amylolytisches Protein mit einer Aminosäuresequenz, die zu der in SEQ ID NO. 2 angegebenen Aminosäuresequenz in den Positionen 32 bis 516 über die ganze Länge mindestens zu 98 % identisch ist.

5. Amylolytisches Protein, das sich von einer Nukleotidsequenz ableitet, die zu der in SEQ ID NO. 1 angegebenen Nukleotidsequenz über die ganze Länge mindestens zu 87,5 % identisch ist, insbesondere über den Teilbereich, der den Aminosäuren 32 bis 516 gemäß der SEQ ID NO. 2 entspricht.

6. Amylolytisches Protein, das sich von einer Nukleotidsequenz ableitet, die zu der in SEQ ID NO. 1 angegebenen Nukleotidsequenz über die ganze Länge mindestens zu 90 % identisch ist, insbesondere über den Teilbereich, der den Aminosäuren 32 bis 516 gemäß der SEQ ID NO. 2 entspricht.

7. Amylolytisches Protein, das mit der in SEQ ID NO.2 angegebenen Aminosäuresequenz insgesamt und/oder in den Positionen 32 bis 516 identisch ist und/oder das mit einer von der in SEQ ID NO. 1 angegebenen Nukleotidsequenz abgeleiteten Aminosäuresequenz insgesamt und/oder in den Positionen 32 bis 516 identisch ist.

8. Amylolytisch aktives Derivat eines Proteins gemäß einem der Ansprüche 1 bis 7, erhalten durch Umwandlung einer Seitenkette einer Aminosäure oder durch kovalente Bindung einer anderen Verbindung an das Protein.

9. Amylolytisches Protein oder Derivat gemäß Anspruch 8, das wenigstens eine antigene Determinante mit einem der in den Ansprüchen 1 bis 8 genannten Proteine oder Derivate gemeinsam hat.

10. Amylolytisches Protein oder Derivat gemäß einem der Ansprüche 1 bis 9, **dadurch gekennzeichnet, daß** es natürlicherweise aus einem Mikroorganismus erhältlich ist.

11. Amylolytisches Protein oder Derivat gemäß Anspruch 10, **dadurch gekennzeichnet, daß** es sich bei dem Mikroorganismus um ein gram-positives Bakterium handelt.

12. Amylolytisches Protein oder Derivat gemäß Anspruch 11, **dadurch gekennzeichnet, daß** es sich bei dem gram-positiven Bakterium um eines der Gattung Bacillus handelt.

13. Amylolytisches Protein oder Derivat gemäß Anspruch 12, **dadurch gekennzeichnet, daß** es sich bei der Bacillus-Spezies um *Bacillus sp. A 7-7*, insbesondere um *Bacillus sp. A 7-7* (DSM 12368) handelt.

14. Für ein amylolytisches Protein codierende Nukleinsäure, deren Nukleotidsequenz zu der in SEQ ID NO. 1 angegebenen Nukleotidsequenz über die ganze Länge mindestens zu 87,5 % identisch ist, insbesondere über den Teilbereich, der den Aminosäuren 32 bis 516 gemäß der SEQ ID NO. 2 entspricht.

15. Für ein amylolytisches Protein codierende Nukleinsäure, deren Nukleotidsequenz zu der in SEQ ID NO. 1 angegebenen Nukleotidsequenz über die ganze Länge mindestens zu 90 % identisch ist, insbesondere über den Teilbereich, der den Aminosäuren 32 bis 516 gemäß der SEQ ID NO. 2 entspricht.

16. Für ein amylolytisches Protein codierende Nukleinsäure, die zu der in SEQ ID NO. 1 angegebenen Nukleotidsequenz zu 100 % identisch ist, insbesondere über den Teilbereich, der den Aminosäuren 32 bis 516 gemäß der SEQ ID NO. 2 entspricht.

17. Nukleinsäure, die für eines der in den Ansprüchen 1 bis 13 bezeichneten Proteine oder Derivate codiert.

18. Natürlicher Mikroorganismus, der eines der in den Ansprüchen 1 bis 13 bezeichneten Proteine oder Derivate bildet oder für diese codierende Nukleinsäuren enthält.

19. Mikroorganismus nach Anspruch 18, **dadurch gekennzeichnet, daß** er ein Bakterium ist.

20. Mikroorganismus nach Anspruch 19, **dadurch gekennzeichnet, daß** es sich bei dem Bakterium um ein gram-positives Bakterium handelt.

21. Mikroorganismus nach Anspruch 20, **dadurch gekennzeichnet, daß** es sich bei dem gram-positiven Bakterium um eines der Gattung Bacillus, insbesondere *Bacillus sp.* A 7-7, ganz besonders um *Bacillus sp.* A 7-7 DSM (12368) handelt.

22. Vektor, der einen in den Ansprüchen 14 bis 17 bezeichneten Nukleinsäurebereich enthält oder einen Nukleinsäurebereich enthält, das für eines der in den Ansprüchen 1 bis 13 bezeichneten Proteine oder Derivate codiert.

23. Klonierungsvektor gemäß Anspruch 22.

24. Expressionsvektor gemäß Anspruch 22.

25. Zelle, die einen Vektor nach einem der Ansprüche 22 bis 24 enthält.

26. Wirtszelle, die eines der in Ansprüchen 1 bis 13 bezeichneten Proteine oder Derivate exprimiert oder zu dessen Expression angeregt werden kann, vorzugsweise unter Einsatz eines Expressionsvektors gemäß Anspruch 24.

27. Wirtszelle gemäß Anspruch 26, **dadurch gekennzeichnet, daß** sie ein Bakterium ist, insbesondere eines, das das gebildete Protein oder Derivat ins umgebende Medium sekretiert.

28. Wirtszelle gemäß Anspruch 26 oder 27, **dadurch gekennzeichnet, daß** sie ein Bakterium der Gattung Bacillus, insbesondere der Species *Bacillus licheniformis, Bacillus amyloliquefaciens, Bacillus subtilis* oder *Bacillus alcalophilus* ist.

29. Wirtszelle gemäß Anspruch 26 oder 27, **dadurch gekennzeichnet, daß** sie ein gram-negatives Bakterium ist.

30. Wirtszelle gemäß Anspruch 29, **dadurch gekennzeichnet, daß** sie der Gattung Escherichia, bevorzugt der Spezies *Escherichia coli,* besonders bevorzugt einem der Stämme *E. coli* JM 109, *E. coli* DH 100B oder *E. coli* DH 12S angehört.

31. Wirtszelle gemäß Anspruch 26, **dadurch gekennzeichnet, daß** sie eine eukaryontische Zelle ist, insbesondere eine, die das gebildete Protein posttranslational modifiziert.

32. Verfahren zur Herstellung eines Proteins oder Derivats gemäß einem der Ansprüche 1 bis 13 unter Verwendung einer Nukleinsäure gemäß einem der Ansprüche 14 bis 17 und/oder unter Verwendung eines Vektors gemäß einem der Ansprüche 22 bis 24 und/oder unter Verwendung einer Wirtszelle gemäß einem der Ansprüche 26 bis 31 oder unter Verwendung einer Zelle, die dieses natürlicherweise bildet, insbesondere einer nach einem der Ansprüche 18 bis 21.

33. Wasch- oder Reinigungsmittel, **dadurch gekennzeichnet, daß** es ein Protein oder Derivat gemäß einem der Ansprüche 1 bis 13 enthält.

34. Mittel gemäß Anspruch 33, **dadurch gekennzeichnet, daß** es 0,000001 Gewichts-Prozent bis 5 Gew.-%, insbesondere 0,00001 bis 3 Gew.-% des amylolytischen Proteins oder Derivats enthält.

35. Mittel gemäß einem der Ansprüche 33 oder 34, **dadurch gekennzeichnet, daß** es zusätzlich eine oder mehrere andere amylolytische Proteine, insbesondere α-Amylasen enthält.

36. Mittel gemäß einem der Ansprüche 33 bis 35, **dadurch gekennzeichnet, daß** es zusätzlich andere Enzyme, insbesondere eine oder mehrere Proteasen, Lipasen, β-Glucanasen und/oder Cellulasen enthält.

37. Mittel gemäß einem der Ansprüche 33 bis 36, **dadurch gekennzeichnet, daß** es aus mehr als einer Phase besteht.

38. Mittel gemäß einem der Ansprüche 33 bis 37, **dadurch gekennzeichnet, daß** es fest ist und mindestens zwei verschiedene Pulver oder Granulate in insgesamt loser Form miteinander vermischt vorliegen.

39. Mittel gemäß Anspruch 37, **dadurch gekennzeichnet, daß** mindestens zwei feste Phasen miteinander verbunden vorliegen, insbesondere nach einem gemeinsamen Kompaktierungsschritt.

40. Festes Mittel gemäß einem der Ansprüche 38 bis 39, **dadurch gekennzeichnet, daß** wenigstens eine der Phasen ein Amylase-sensitives Material, insbesondere Stärke enthält oder von diesem zumindest teilweise umgeben oder beschichtet ist.

41. Mittel gemäß einem der Ansprüche 33 bis 37, **dadurch gekennzeichnet, daß** es flüssig, gelförmig oder pastös ist und das enthaltene Protein und/oder wenigstens eines der enthaltenen Enzyme und/oder wenigstens eine der sonstigen enthaltenen Komponenten einzeln oder zusammen mit anderen Bestandteilen verkapselt vorliegt, bevorzugt in Mikrokapseln, besonders bevorzugt in solchen aus einem Amylase-sensitiven Material.

42. Mittel gemäß einem der Ansprüche 33 bis 41, **dadurch gekennzeichnet, daß** die amylolytische Aktivität durch einen der sonstigen Bestandteile des Mittels modifiziert, insbesondere stabilisiert und/oder in ihrem Beitrag zur Wasch-, beziehungsweise Reinigungsleistung des Mittels gesteigert wird.

43. Verfahren zur Reinigung von Textilien oder von harten Oberflächen, **dadurch gekennzeichnet, daß** in wenigstens einem der Verfahrensschritte ein amylolytisches Protein oder Derivat gemäß einem der Ansprüche 1 bis 13 aktiv wird.

44. Verfahren zur Reinigung von Textilien oder von harten Oberflächen, **dadurch gekennzeichnet, daß** in wenigstens einem der Verfahrensschritte ein Mittel gemäß einem der Ansprüche 33 bis 42 eingesetzt wird.

45. Verfahren nach Anspruch 43 oder 44, **dadurch gekennzeichnet, daß** das amylolytische Protein oder Derivat in dem betreffenden Verfahrensschritt in einer Menge von 0,01 mg bis 200 mg pro Anwendung, vorzugsweise von 0,02 mg bis 100 mg pro Anwendung eingesetzt wird.

46. Verwendung eines amylolytischen Proteins oder Derivats gemäß einem der Ansprüche 1 bis 13 allein oder zusammen mit mindestens einem anderen reinigungsaktiven oder die Reinigungswirkung unterstützenden Wirkstoff zur Reinigung von Textilien oder von harten Oberflächen.

47. Verwendung eines Mittels gemäß einem der Ansprüche 33 bis 42 zur Reinigung von Textilien oder von harten Oberflächen.

48. Verwendung nach Anspruch 46 oder 47, **dadurch gekennzeichnet, daß** pro Anwendung, vorzugsweise pro Anwendung in einer Geschirrspülmaschine oder einer Waschmaschine, 0,01 mg bis 200 mg des amylolytischen Proteins oder Derivats, bevorzugt 0,02 mg bis 100 mg des amylolytischen Proteins oder Derivats eingesetzt werden.

49. Verwendung eines amylolytischen Proteins oder Derivats gemäß einem der Ansprüche 1 bis 13 allein oder zusammen mit mindestens einem anderen reinigungsaktiven oder die Reinigungswirkung unterstützenden Wirkstoff in einem aus mehr als einer Phase bestehenden Wasch- oder Reinigungsmittel zur Aktivierung der eigenen oder anderer Phasen.

50. Verwendung eines amylolytischen Proteins oder Derivats gemäß einem der Ansprüche 1 bis 13 allein oder zusammen mit mindestens einem anderen reinigungsaktiven oder die Reinigungswirkung unterstützenden Wirkstoff in einem Wasch- oder Reinigungsmittel mit verkapselten Inhaltsstoffen zur Freisetzung der Inhaltsstoffe aus den Kapseln.

51. Verwendung eines Proteins oder Derivats gemäß einem der Ansprüche 1 bis 13 zur Behandlung von Rohmaterialien oder Zwischenprodukten in der Textilherstellung, insbesondere zum Entschlichten von Baumwolle.

52. Verfahren zur Stärkeverflüssigung, insbesondere zur Ethanolproduktion, **dadurch gekennzeichnet, daß** darin ein Protein oder Derivat gemäß einem der Ansprüche 1 bis 13 eingesetzt wird.

53. Verwendung eines Proteins oder Derivats gemäß einem der Ansprüche 1 bis 13 zur Herstellung von linearen und/oder kurzkettigen Oligosacchariden.

54. Verwendung eines Proteins oder Derivats gemäß einem der Ansprüche 1 bis 13 zur Hydrolyse von Cyclodextrinen.

55. Verwendung eines Proteins oder Derivats gemäß einem der Ansprüche 1 bis 13 zur Freisetzung von niedermolekularen Verbindungen aus Polysaccharidträgern oder Cyclodextrinen.

56. Verwendung eines Proteins oder Derivats gemäß einem der Ansprüche 1 bis 13 zur Herstellung von Lebensmitteln und/oder Lebensmittelbestandteilen.

57. Verwendung eines Proteins oder Derivats gemäß einem der Ansprüche 1 bis 13 zur Herstellung von Tierfutter und/oder Tierfutterbestandteilen.

58. Verwendung eines Proteins oder Derivats gemäß einem der Ansprüche 1 bis 13 zur Auflösung stärkehaltiger Klebeverbindungen.

59. Temporäres Klebeverfahren, **dadurch gekennzeichnet, daß** darin ein Protein oder Derivat gemäß einem der Ansprüche 1 bis 13 eingesetzt wird.

## Claims

1. Amylolytic protein having an amino acid sequence which, over its entire length, is at least 96% identical to the amino acid sequence indicated in SEQ ID NO. 2.

2. Amylolytic protein having an amino acid sequence which, over its entire length, is at least 98% identical to the amino acid sequence indicated in SEQ ID NO. 2.

3. Amylolytic protein having an amino acid sequence which, over the entire length of from positions 32 to 516, is at least 96% identical to the amino acid sequence indicated in SEQ ID NO. 2.

4. Amylolytic protein having an amino acid sequence which, over the entire length of from positions 32 to 516, is at least 98% identical to the amino acid sequence indicated in SEQ ID NO. 2.

5. Amylolytic protein derived from a nucleotide sequence which, over its entire length, in particular over the part corresponding to the amino acids 32 to 516 of SEQ ID NO. 2, is at least 87.5% identical to the nucleotide sequence indicated in SEQ ID NO. 1.

6. Amylolytic protein derived from a nucleotide sequence which, over its entire length, in particular over the part corresponding to the amino acids 32 to 516 of SEQ ID NO. 2, is at least 90% identical to the nucleotide sequence indicated in SEQ ID NO. 1.

7. Amylolytic protein which is identical to the entire amino acid sequence indicated in SEQ ID NO. 2 and/or to positions 32 to 516 thereof and/or which is identical to an entire amino acid sequence derived from the nucleotide sequence indicated in SEQ ID NO. 1 and/or to positions 32 to 516 thereof.

8. Amylolytically active derivative of a protein according to any of Claims 1 to 7, obtained by conversion of a side chain of an amino acid or by covalent binding of another compound to the protein.

9. Amylolytic protein or derivative according to Claim 8, which has at least one antigenic determinant in common with any of the proteins or derivatives set forth in Claims 1 to 8.

10. Amylolytic protein or derivative according to any of Claims 1 to 9, **characterized in that** it is naturally obtainable from a microorganism.

11. Amylolytic protein or derivative according to Claim 10, **characterized in that** the microorganism is a Gram-positive bacterium.

12. Amylolytic protein or derivative according to Claim 11, **characterized in that** the Gram-positive bacterium is one of the genus Bacillus.

13. Amylolytic protein or derivative according to Claim 12, **characterized in that** the Bacillus species is *Bacillus sp. A 7-7,* in particular *Bacillus sp. A 7-7* (DSM 12368).

14. Nucleic acid coding for an amylolytic protein, whose nucleotide sequence, over its entire length, in particular over the part corresponding to the amino acids 32 to 516 of SEQ ID NO. 2, is at least 87.5% identical to the nucleotide sequence indicated in SEQ ID NO. 1.

15. Nucleic acid coding for an amylolytic protein, whose nucleotide sequence, over its entire length, in particular over the part corresponding to the amino acids 32 to 516 of SEQ ID NO. 2, is at least 90% identical to the nucleotide sequence indicated in SEQ ID NO. 1.

16. Nucleic acid coding for an amylolytic protein, which is 100% identical to the nucleotide sequence indicated in SEQ ID NO. 1, in particular over the part corresponding to the amino acids 32 to 516 of SEQ ID NO. 2.

17. Nucleic acid coding for any of the proteins or derivatives as defined in Claims 1 to 13.

18. Natural microorganism producing any of the proteins or derivatives as defined in Claims 1 to 13 or harbouring nucleic acids coding therefor.

19. Microorganism according to Claim 18, **characterized in that** it is a bacterium.

20. Microorganism according to Claim 19, **characterized in that** the bacterium is a Gram-positive bacterium.

21. Microorganism according to Claim 20, **characterized in that** the Gram-positive bacterium is one of the genus Bacillus, in particular *Bacillus sp. A 7-7,* especially *Bacillus sp. A 7-7* (DSM 12368).

22. Vector comprising a nucleic acid region as defined in Claims 14 to 17 or comprising a nucleic acid region coding for any of the proteins or derivatives as defined in Claims 1 to 13.

23. Cloning vector according to Claim 22.

24. Expression vector according to Claim 22.

25. Cell harbouring a vector according to any of Claims 22 to 24.

26. Host cell which expresses or can be induced to express any of the proteins or derivatives as defined in Claims 1 to 13, preferably by employing an expression vector according to Claim 24.

27. Host cell according to Claim 26, **characterized in that** it is a bacterium, in particular one which secretes the protein or derivative produced into the surrounding medium.

28. Host cell according to Claim 26 or 27, **characterized in that** it is a bacterium of the genus Bacillus, in particular of the species *Bacillus licheniformis, Bacillus amyloliquefaciens, Bacillus subtilis* or *Bacillus alcalophilus.*

29. Host cell according to Claim 26 or 27, **characterized in that** it is a Gram-negative bacterium.

30. Host cell according to Claim 29, **characterized in that** it belongs to the genus Escherichia, preferably to the species *Escherichia coli,* particularly preferably to any of the strains *E. coli* JM 109, *E.* *coli* DH 100B and *E. coli* DH 12S.

31. Host cell according to Claim 26, **characterized in that** it is a eukaryotic cell, in particular one which posttranslationally modifies the protein produced.

32. Process for producing a protein or derivative according to any of Claims 1 to 13 by using a nucleic acid according to any of Claims 14 to 17 and/or by using a vector according to any of Claims 22 to 24 and/or by using a host cell according to any of Claims 26 to 31 or by using a cell which naturally produces the said protein or derivative, in particular a cell according to any of Claims 18 to 21.

33. Washing or cleaning agent, **characterized in that** it comprises a protein or derivative according to any of Claims 1 to 13.

34. Agent according to Claim 33, **characterized in that** it comprises from 0.000001 per cent by weight to 5% by weight, in particular 0.00001 to 3% by weight, of the amylolytic protein or derivative.

35. Agent according to either of Claims 33 and 34, **characterized in that** it additionally comprises one or more other amylolytic proteins, in particular α-amylases.

36. Agent according to any of Claims 33 to 35, **characterized in that** it additionally comprises other enzymes, in particular one or more proteases, lipases, β-glucanases and/or cellulases.

37. Agent according to any of Claims 33 to 36, **characterized in that** it consists of more than one phase.

38. Agent according to any of Claims 33 to 37, **characterized in that** it is solid and that at least two different powders or granules are present in a by and large loose mixture.

39. Agent according to Claim 37, **characterized in that** at least two solid phases are in combination with one another, in particular after a joint compacting step.

40. Solid agent according to either of Claims 38 and 39, **characterized in that** at least one of the phases comprises an amylase-sensitive material, in particular starch, or is, at least partially, surrounded thereby or coated therewith.

41. Agent according to any of Claims 33 to 37, **characterized in that** it is in liquid, gel or paste form and that the protein comprised and/or at least one of the enzymes comprised and/or at least one of the other components comprised are present, individually or together with other components, in encapsulated form, preferably in microcapsules, particularly preferably in those made of an amylase-sensitive material.

42. Agent according to any of Claims 33 to 41, **characterized in that** any of the other components of the agent modifies, in particular stabilizes, the amylolytic activity and/or increases the contribution thereof to the washing or cleaning performance of the agent.

43. Process for cleaning textiles or hard surfaces, **characterized in that** an amylolytic protein or derivative according to any of Claims 1 to 13 becomes active in at least one of the process steps.

44. Process for cleaning textiles or hard surfaces, **characterized in that** an agent according to any of Claims 33 to 42 is used in at least one of the process steps.

45. Process according to Claim 43 or 44, **characterized in that** the amylolytic protein or derivative is used in the process step in question in an amount of from 0.01 mg to 200 mg per application, preferably from 0.02 mg to 100 mg per application.

46. Use of an amylolytic protein or derivative according to any of Claims 1 to 13 alone or together with at least one other cleaning-active active ingredient or active ingredient supporting the cleaning action for cleaning textiles or hard surfaces.

47. Use of an agent according to any of Claims 33 to 42 for cleaning textiles or hard surfaces.

48. Use according to Claim 46 or 47, **characterized in that** from 0.01 mg to 200 mg, preferably from 0.02 mg to 100 mg, of the amylolytic protein or derivative are used per application, preferably per application in a dishwasher or a washing machine.

49. Use of an amylolytic protein or derivative according to any of Claims 1 to 13 alone or together with at least one other cleaning-active active ingredient or active ingredient supporting the cleaning action in a washing or cleaning agent consisting of more than one phase for activating its own or other phases.

50. Use of an amylolytic protein or derivative according to any of Claims 1 to 13 alone or together with at least one other cleaning-active active ingredient or active ingredient supporting the cleaning action in a washing or cleaning agent containing encapsulated ingredients for releasing the ingredients from the capsules.

51. Use of a protein or derivative according to any of Claims 1 to 13 for the treatment of raw materials or intermediates in the manufacture of textiles, in particular for desizing cotton.

52. Process for starch liquefaction, in particular for producing ethanol, **characterized in that** a protein or derivative according to any of Claims 1 to 13 is used therein.

53. Use of a protein or derivative according to any of Claims 1 to 13 for preparing linear and/or short-chain oligosaccharides.

54. Use of a protein or derivative according to any of Claims 1 to 13 for hydrolysing cyclodextrins.

55. Use of a protein or derivative according to any of Claims 1 to 13 for releasing low-molecular weight compounds from polysaccharide supports or from cyclodextrins.

56. Use of a protein or derivative according to any of Claims 1 to 13 for preparing food and/or food ingredients.

57. Use of a protein or derivative according to any of Claims 1 to 13 for preparing animal feed and/or animal feed ingredients.

58. Use of a protein or derivative according to any of Claims 1 to 13 for dissolving adhesive bonds containing starch.

59. Temporary bonding process, **characterized in that** a protein or derivative according to any of Claims 1 to 13 is used therein.

## Revendications

1. Protéine amylolytique ayant une séquence d'acides aminés, qui est au moins identique à 96% à la séquence d'acides aminés indiquée dans SEQ ID NO.2 sur toute la longueur.

2. Protéine amylolytique ayant une séquence d'acides aminés, qui est au moins identique à 98% à la séquence d'acides aminés indiquée dans SEQ ID NO.2 sur toute la longueur.

3. Protéine amylolytique ayant une séquence d'acides aminés, qui est au moins identique à 96% à la séquence d'acides aminés aux positions 32 à 516, indiquée dans SEQ ID NO.2 sur toute la longueur.

4. Protéine amylolytique ayant une séquence d'acides aminés qui est identique au moins à 98% à la séquence d'acides aminés aux positions 32 à 516, indiquée dans SEQ ID NO.2 sur toute la longueur.

5. Protéine amylolytique, qui est dérivée d'une séquence de nucléotides qui est identique au moins à 87,5% à la séquence de nucléotides indiquée dans SEQ ID NO.1 sur toute la longueur, en particulier sur la zone partielle qui correspond aux acides aminés 32 à 516 selon la SEQ ID NO.2.

6. Protéine amylolytique, qui est dérivée d'une séquence de nucléotides qui est identique au moins à 90% à la séquence de nucléotides indiquée dans SEQ ID NO.1 sur toute la longueur, en particulier sur la zone partielle qui correspond aux acides aminés 32 à 516 selon SEQ ID NO.2.

7. Protéine amylolytique, qui est identique à la séquence d'acides aminés indiquée dans SEQ ID NO.2 en totalité et/ou aux positions 32 à 516 et/ou qui est identique à la séquence d'acides aminés dérivée de la séquence de nucléotides indiquée dans SEQ ID NO.1 en totalité et/ou aux positions 32 à 516.

8. Dérivé à action amylolytique d'une protéine selon l'une quelconque des revendications 1 à 7, obtenu par transformation d'une chaîne latérale d'un acide aminé, ou par liaison covalente d'un autre composé, à la protéine.

9. Protéine ou dérivé amylolytique selon la revendication 8, qui présente conjointement avec l'une des protéines ou dérivés cités selon les revendications 1 à 8, au moins un déterminant antigénique.

10. Protéine ou dérivé amylolytique selon l'un quelconque des revendications 1 à 9, **caractérisé en qu'**il peut être obtenu de façon naturelle à partir d'un microorganisme.

11. Protéine ou dérivé amylolytique selon la revendication 10, **caractérisé en ce que**, quant au microorganisme, il s'agit d'une bactérie Gram positive.

12. Protéine ou dérivé amylolytique selon la revendication 11, **caractérisé en ce que**, quant à la bactérie Gram positive, il s'agit d'une du genre Bacillus.

13. Protéine ou dérivé amylolytique selon la revendication 12, **caractérisé en ce que**, quant à l'espèce Bacillus, il s'agit de *Bacillus sp. A 7-7,* en particulier de *Bacillus sp. A 7-7 (DSM 12368).*

14. Acide nucléique codant pour une protéine amylolytique, dont la séquence de nucléotides est identique au moins à 87,5% à la séquence de nucléotides indiquée dans SEQ ID NO.1 sur toute la longueur, en particulier sur la zone partielle qui correspond aux acides aminés 32 à 516 selon SEQ ID NO.2.

15. Acide nucléique codant pour une protéine amylolytique, dont la séquence de nucléotides est identique au moins à 90% à la séquence de nucléotides indiquée dans SEQ ID NO.1 sur toute la longueur, en particulier sur la zone partielle qui correspond aux acides aminés 32 à 516 selon SEQ ID NO.2.

16. Acide nucléique codant pour une protéine amylolytique, dont la séquence de nucléotides est identique au moins à 100% à la séquence de nucléotides indiquée dans SEQ ID NO.1, en particulier sur la zone partielle qui correspond aux acides aminés 32 à 516 selon SEQ ID NO.2.

17. Acide nucléique, qui code pour l'une des protéines ou dérivés désignés dans les revendications 1 à 13.

18. Microorganisme naturel qui forme l'une des protéines ou dérivés désignés dans les revendications 1 à 13, ou qui contient des acides nucléiques codant pour ceux-ci.

19. Microorganisme selon la revendication 18, **caractérisé en ce qu'**il est une bactérie.

20. Microorganisme selon la revendication 19, **caractérisé en ce que**, quant à la bactérie, il s'agit d'une bactérie Gram positive.

21. Microorganisme selon la revendication 20, **caractérisé en ce que**, quant à la bactérie Gram positive, il s'agit d'une bactérie du genre Bacillus en particulier *Bacillus sp. A* 7-7, tout particulièrement *Bacillus sp. A 7-7 (DSM 12368)*.

22. Vecteur, qui contient une zone d'acide nucléique désignée dans les revendications 14 à 17, ou qui contient une zone d'acide nucléique, qui code pour l'une des protéines ou dérivés désignés dans les revendications 1 à 13.

23. Vecteur de clonage selon la revendication 22.

24. Vecteur d'expression selon la revendication 22.

25. Cellule, qui contient un vecteur selon l'une quelconque des revendications 22 à 24.

26. Cellule hôte, qui peut exprimer l'une des protéines ou dérivés désignés dans les revendications 1 à 13 ou peut être stimulée pour exprimer celui-ci, de préférence en mettant en oeuvre un vecteur d'expression selon la revendication 24.

27. Cellule hôte selon la revendication 26, **caractérisée en ce qu'**elle est une bactérie, en particulier une bactérie qui sécrète la protéine ou dérivé formé dans le milieu environnant.

28. Cellule hôte selon la revendication 26 ou 27, **caractérisée en ce qu'**elle est une bactérie du genre Bacillus, en particulier de l'espèce *Bacillus licheniformis, Bacillus amyloliquefaciens, Bacillus subtilis* ou *Bacillus alcalophilus*.

29. Cellule hôte selon la revendication 26 ou 27, **caractérisée en ce qu'**elle est une bactérie Gram négative.

30. Cellule hôte selon la revendication 29, **caractérisée en ce qu'**elle appartient au genre Escherichia, de préférence à l'espèce *Escherichia coli,* de manière particulièrement préférée l'une des souches *E. coli* JM 109, *E. coli* DH 100B ou *E. coli* DH 12S.

31. Cellule hôte selon la revendication 26, **caractérisée en ce qu'**elle est une cellule eucaryote, en particulier une cellule qui modifie de façon post-traductionnelle la protéine formée.

32. Procédé de préparation d'une protéine ou d'un dérivé selon l'une quelconque des revendications 1 à 13, en employant un acide nucléique, selon l'une quelconque des revendications 14 à 17 et/ou en employant un vecteur selon l'une quelconque des revendications 22 à 24 et/ou en employant une cellule hôte selon l'une quelconque des revendications 26 à 31, ou en employant une cellule qui forme celui-ci naturellement, en particulier une cellule selon l'une quelconque des revendications 18 à 21.

33. Agent de lavage ou de nettoyage, **caractérisé en ce qu'**il contient une protéine ou un dérivé selon l'une quelconque des revendications 1 à 13.

34. Agent selon la revendication 33, **caractérisé en ce qu'**il contient 0,000001% en poids jusqu'à 5% en poids, en particulier de 0,00001% à 3% en poids, de la protéine ou du dérivé, amylolytique.

35. Agent selon l'une quelconque des revendications 33 ou 34, **caractérisé en ce qu'**il contient en outre une ou plusieurs autres protéines amylolytiques en particulier des α-amylases.

36. Agent selon l'une quelconque des revendications 33 à 35, **caractérisé en ce qu'**il contient en outre d'autres enzymes, en particulier une ou plusieurs protéases, lipases, β-glucanases, et/ou cellulases.

37. Agent selon l'une quelconque des revendications 33 à 36, **caractérisé en ce qu'**il est constitué de plus d'une phase.

38. Agent selon l'une quelconque des revendications 33 à 37, **caractérisé en ce qu'**il est solide, et qu'au moins deux poudres ou granulés différents existent à l'état mélangé mutuellement, sous une forme au total en vrac.

39. Agent selon la revendication 37, **caractérisé en ce qu'**au moins deux phases solides existent liées l'une à l'autre, en particulier après une étape de compaction commune.

40. Agent solide selon l'une quelconque des revendications 38 à 39, **caractérisé en ce qu'**au moins l'une des phases contient une matière sensible aux amylases, en particulier l'amidon, ou bien elle est au moins partiellement entourée par celle-ci, ou revêtue de celle-ci.

41. Agent selon l'une quelconque des revendications 33 à 37, **caractérisé en ce qu'**il est liquide, en forme de gel ou pâteux, et la protéine contenue et/ou au moins l'une des enzymes contenues, et/ou au moins l'un des autres composants contenus, existent individuellement ou conjointement, encapsulés avec d'autres composants, de préférence dans des microcapsules, de manière particulièrement préférée dans celles constituées d'une matière sensible aux amylases.

42. Agent selon l'une quelconque des revendications 33 à 41, **caractérisé en ce que** l'activité amylolytique est modifiée par l'un des autres composants de l'agent, en particulier stabilisée et/ou augmentée dans sa contribution à la performance de lavage, respectivement de nettoyage, de l'agent.

43. Procédé de nettoyage de textiles ou de surfaces dures, **caractérisé en ce que**, dans au moins l'une des étapes de procédé, une protéine ou un dérivé, amylolytique, selon l'une quelconque des revendications 1 à 13 devient actif.

44. Procédé de nettoyage de textiles ou de surfaces dures, **caractérisé en ce que**, dans au moins l'une des étapes de procédé, un agent selon l'une quelconque des revendications 33 à 42, est mis en oeuvre.

45. Procédé selon la revendication 43 ou 44, **caractérisé en ce que**, la protéine ou le dérivé, amylolytique, est mis en oeuvre dans l'étape de procédé en question en une quantité de 0,01 mg à 200 mg par application, de préférence de 0,02 mg à 100 mg par application.

46. Utilisation d'une protéine ou dérivé amylolytique selon l'une quelconque des revendications 1 à 13, seule ou conjointement avec au moins une autre substance active à action nettoyante, ou soutenant l'action de nettoyage pour nettoyer les textiles et les surfaces dures.

47. Utilisation d'un agent selon l'une quelconque des revendications 33 à 42, pour nettoyer les textiles ou les surfaces dures.

48. Utilisation selon la revendication 46 ou 47, **caractérisée en ce que**, par application, de préférence par application dans un lave-vaisselle ou un lave-linge, 0,01 mg à 200 mg de la protéine ou dérivé, amylolytique, de préférence 0,02 mg à 100 mg de la protéine ou dérivé, amylolytique, sont mis en oeuvre.

49. Utilisation d'une protéine ou dérivé amylolytique selon l'une quelconque des revendications 1 à 13, seule ou conjointement avec au moins une autre substance active à action nettoyante ou soutenant l'action de lavage, dans un agent de lavage ou de nettoyage constitué de plus d'une phase, pour activer la même phase ou d'autres phases.

50. Utilisation d'une protéine ou dérivé amylolytique selon l'une quelconque des revendications 1 à 13, seul ou conjointement avec au moins une autre substance active à action nettoyante ou soutenant l'action de nettoyage, dans un agent de lavage ou de nettoyage avec des ingrédients encapsulés destinés à libérer les ingrédients à partir des capsules.

51. Utilisation d'une protéine ou dérivé selon l'une quelconque des revendications 1 à 13, pour traiter des matières premières ou des produits intermédiaires dans la fabrication de textiles, en particulier pour le désensimage du coton.

52. Procédé de liquéfaction de l'amidon, en particulier pour la production d'éthanol, **caractérisé en ce qu'**une protéine ou dérivé selon l'une quelconque des revendications 1 à 13, y est mis en oeuvre.

53. Utilisation d'une protéine ou dérivé selon l'une quelconque des revendications 1 à 13, pour préparer des oligosaccharides linéaires et/ou à chaîne courte.

54. Utilisation d'une protéine ou dérivé selon l'une quelconque des revendications 1 à 13, pour l'hydrolyse de cyclodextrines.

55. Utilisation d'une protéine ou dérivé selon l'une quelconque des revendications 1 à 13, pour libérer des composés de faible masse moléculaire, à partir de supports en polysaccharides ou à partir de cyclodextrines.

56. Utilisation d'une protéine ou dérivé selon l'une quelconque des revendications 1 à 13, pour préparer des aliments et/ou des composants alimentaires.

57. Utilisation d'une protéine ou dérivé selon l'une quelconque des revendications 1 à 13, pour préparer des aliments pour animaux et/ou des composants d'aliments pour animaux.

58. Utilisation d'une protéine ou dérivé selon l'une quelconque des revendications 1 à 13, pour dissoudre des assemblages collés contenant de l'amidon.

59. Procédé de collage temporaire, **caractérisé en ce qu'**une protéine ou dérivé selon l'une quelconque des revendications 1 à 13, y est mis en oeuvre.


### SEQUENZPROTOKOLL

<110> Henkel Kommanditgesellschaft auf Aktien
<120> Neues amylolytisches Enzym aus Bacillus sp. A 7-7 (DSM 12368) sowie Wasch- und Reinigungsmittel mit diesem neuen amylolytischen Enzym
<130> H 4713 / H 4846
<140>
   <141>
<150> DE10036752
   <151> 2000-07-28
<150> DE10036753
   <151> 2000-07-28
<160> 2
<170> PatentIn Ver. 2.1
<210> 1
   <211> 1551
   <212> DNA
   <213> Bacillus sp. A 7-7 (DSM 12368)
<220>
   <221> CDS
   <222> (1)..(1551)
<220>
   <221> mat_peptide
   <222> (94)
<400> 1
   atg aga aaa cgt aaa aat gga tta atc agt att cta ttg gca ttt ttg     48
   Met Arg Lys Arg Lys Asn Gly Leu Ile Ser Ile Leu Leu Ala Phe Leu
       -30                 -25                 -20
   ttg gta ctt aca tca ata cct ttt act tca gca aac gta gaa gca cac     96
   Leu Val Leu Thr Ser Ile Pro Phe Thr Ser Ala Asn Val Glu Ala His
   -15                 -10                  -5                  -1   1
   cat aat ggc aca aat gga aca atg atg caa tat ttt gaa tgg tat ttg    144
   His Asn Gly Thr Asn Gly Thr Met Met Gln Tyr Phe Glu Trp Tyr Leu
                 5                  10                  15
   cca aat gac ggt aat cat tgg aat aga tta aga tca gat gca agt aat    192
   Pro Asn Asp Gly Asn His Trp Asn Arg Leu Arg Ser Asp Ala Ser Asn
            20                  25                  30
   ctt aaa gat aaa ggg att aca gcg gtt tgg ata cca cct gct tgg aaa    240
   Leu Lys Asp Lys Gly Ile Thr Ala Val Trp Ile Pro Pro Ala Trp Lys
       35                  40                  45
   ggg gct tct caa aat gat gta ggg tat gga gcc tat gat ctg tat gat    288
   Gly Ala Ser Gln Asn Asp Val Gly Tyr Gly Ala Tyr Asp Leu Tyr Asp
    50                  55                  60                  65
   tta gga gaa ttc aat caa aaa gga acc gta cgt aca aag tac gga acc    336
   Leu Gly Glu Phe Asn Gln Lys Gly Thr Val Arg Thr Lys Tyr Gly Thr
                   70                  75                  80
   cgt aat caa tta caa gct gca gta acc gcc tta aaa agt aat ggt att    384
   Arg Asn Gln Leu Gln Ala Ala Val Thr Ala Leu Lys Ser Asn Gly Ile
               85                  90                  95
   caa gta tac gga gat gtc gta atg aat cat aag ggt gga gcg gat gcc    432
   Gln Val Tyr Gly Asp Val Val Met Asn His Lys Gly Gly Ala Asp Ala
           100                 105                 110
   act gag tgg gtt cga gcg gtt gaa gtg aac cca agt aat cgt aat caa    480
   Thr Glu Trp Val Arg Ala Val Glu Val Asn Pro Ser Asn Arg Asn Gln
       115                 120                 125
   gaa gtc tct ggt gat tat acg att gag gct tgg act aag ttt gat ttt    528
   Glu Val Ser Gly Asp Tyr Thr Ile Glu Ala Trp Thr Lys Phe Asp Phe
   130                 135                 140                 145
   cct ggt cga ggt aat acc cac tct aac ttt aaa tgg aga tgg tat cat    576
   Pro Gly Arg Gly Asn Thr His Ser Asn Phe Lys Trp Arg Trp Tyr His
                   150                 155                 160
   ttc gat ggt gta gat tgg gat cag tca cgt caa ttg cag aat cga atc    624
   Phe Asp Gly Val Asp Trp Asp Gln Ser Arg Gln Leu Gln Asn Arg Ile
               165                 170                 175
   tat aaa ttc aga gga gat gga aaa ggt tgg gac tgg gaa gtt gat aca    672
   Tyr Lys Phe Arg Gly Asp Gly Lys Gly Trp Asp Trp Glu Val Asp Thr
           180                 185                 190
   gag aac gga aac tat gac tat cta atg tac gcg gat att gat atg gat    720
   Glu Asn Gly Asn Tyr Asp Tyr Leu Met Tyr Ala Asp Ile Asp Met Asp
       195                 200                 205
   cac ccc gaa gta gtg aat gaa ctc aga aac tgg ggt gta tgg tat acc    768
   His Pro Glu Val Val Asn Glu Leu Arg Asn Trp Gly Val Trp Tyr Thr
   210                 215                 220                 225
   aat aca ctg ggg cta gac ggg ttc aga ata ggt gcg gta aaa cat ata    816
   Asn Thr Leu Gly Leu Asp Gly Phe Arg Ile Gly Ala Val Lys His Ile
                   230                 235                 240
   aaa tat agc ttt act cgt gat tgg ctt act cac gtt aga aat acg aca    864
   Lys Tyr Ser Phe Thr Arg Asp Trp Leu Thr His Val Arg Asn Thr Thr
               245                 250                 255
   ggt aaa aat atg ttt gca gtt gca gag ttc tgg aag aat gac ata ggt    912
   Gly Lys Asn Met Phe Ala Val Ala Glu Phe Trp Lys Asn Asp Ile Gly
           260                 265                 270
   gca att gaa aat tac tta agt aaa aca aat tgg aat cat tca gtt ttt    960
   Ala Ile Glu Asn Tyr Leu Ser Lys Thr Asn Trp Asn His Ser Val Phe
       275                 280                 285
   gat gtg ccc ctg cat tat aac ctt tat aat gca tcg aga agt ggt ggc    1008
   Asp Val Pro Leu His Tyr Asn Leu Tyr Asn Ala Ser Arg Ser Gly Gly
   290                 295                 300                 305
   aat tat gat atg agg caa ata ttt aat gga aca gtt gtt cag aga cat    1056
   Asn Tyr Asp Met Arg Gln Ile Phe Asn Gly Thr Val Val Gln Arg His
                   310                 315                 320
   cct aca cat gct gta aca ttt gtt gat aac cat gat tca cag ccg gaa     1104
   Pro Thr His Ala Val Thr Phe Val Asp Asn His Asp Ser Gln Pro Glu
               325                 330                 335
   gaa gcc cta gag tca ttt gtt gaa gag tgg ttc aaa ccg tta gcg tgt     1152
   Glu Ala Leu Glu Ser Phe Val Glu Glu Trp Phe Lys Pro Leu Ala Cys
           340                 345                 350
   gct ctc aca cta aca cgt gat caa gga tat cct tcc gtt ttt tat gga     1200
   Ala Leu Thr Leu Thr Arg Asp Gln Gly Tyr Pro Ser Val Phe Tyr Gly
       355                 360                 365
   gat tat tat ggg att ccg acg cat ggt gta cca gca atg aaa tct aag     1248
   Asp Tyr Tyr Gly Ile Pro Thr His Gly Val Pro Ala Met Lys Ser Lys
   370                 375                 380                 385
   att gat ccg att tta gaa gca cgt caa aag tat gcg tac gga aaa caa     1296
   Ile Asp Pro Ile Leu Glu Ala Arg Gln Lys Tyr Ala Tyr Gly Lys Gln
                   390                 395                 400
   aat gat tat ttg gat cac cat aat atg att ggc tgg acg cgt gaa ggt     1344
   Asn Asp Tyr Leu Asp His His Asn Met Ile Gly Trp Thr Arg Glu Gly
               405                 410                 415
   aat aca gca cat ccc aac tca gga cta gca act att atg tcg gat ggc     1392
   Asn Thr Ala His Pro Asn Ser Gly Leu Ala Thr Ile Met Ser Asp Gly
           420                 425                 430
   cca gga gga aat aaa tgg atg tat gtt ggg cgt aat aag gct gga caa     1440
   Pro Gly Gly Asn Lys Trp Met Tyr Val Gly Arg Asn Lys Ala Gly Gln
       435                 440                 445
   gtt tgg aga gat att aca gga aat cgc tca ggt acg gtg acg att aac     1488
   Val Trp Arg Asp Ile Thr Gly Asn Arg Ser Gly Thr Val Thr Ile Asn
   450                 455                 460                 465
   gca gat ggg tgg ggt aat ttt tct gta aat ggt ggg tct gta tct ata     1536
   Ala Asp Gly Trp Gly Asn Phe Ser Val Asn Gly Gly Ser Val Ser Ile
                   470                 475                 480
   tgg gta aat aat taa                                                 1551
   Trp Val Asn Asn
               485
<210> 2
   <211> 516
   <212> PRT
   <213> Bacillus sp. A 7-7 (DSM 12368)
<400> 2
   Met Arg Lys Arg Lys Asn Gly Leu Ile Ser Ile Leu Leu Ala Phe Leu
    1               5                   10                  15
   Leu Val Leu Thr Ser Ile Pro Phe Thr Ser Ala Asn Val Glu Ala His
               20                  25                  30
   His Asn Gly Thr Asn Gly Thr Met Met Gln Tyr Phe Glu Trp Tyr Leu
           35                  40                  45
   Pro Asn Asp Gly Asn His Trp Asn Arg Leu Arg Ser Asp Ala Ser Asn
           50                  55                  60
   Leu Lys Asp Lys Gly Ile Thr Ala Val Trp Ile Pro Pro Ala Trp Lys
   65                  70                  75                  80
   Gly Ala Ser Gln Asn Asp Val Gly Tyr Gly Ala Tyr Asp Leu Tyr Asp
                   85                  90                  95
   Leu Gly Glu Phe Asn Gln Lys Gly Thr Val Arg Thr Lys Tyr Gly Thr
               100                 105                 110
   Arg Asn Gln Leu Gln Ala Ala Val Thr Ala Leu Lys Ser Asn Gly Ile
           115                 120                 125
   Gln Val Tyr Gly Asp Val Val Met Asn His Lys Gly Gly Ala Asp Ala
       130                 135                 140
   Thr Glu Trp Val Arg Ala Val Glu Val Asn Pro Ser Asn Arg Asn Gln
   145                 150                 155                 160
   Glu Val Ser Gly Asp Tyr Thr Ile Glu Ala Trp Thr Lys Phe Asp Phe
                   165                 170                 175
   Pro Gly Arg Gly Asn Thr His Ser Asn Phe Lys Trp Arg Trp Tyr His
               180                 185                 190
   Phe Asp Gly Val Asp Trp Asp Gln Ser Arg Gln Leu Gln Asn Arg Ile
           195                 200                 205
   Tyr Lys Phe Arg Gly Asp Gly Lys Gly Trp Asp Trp Glu Val Asp Thr
       210                 215                 220
   Glu Asn Gly Asn Tyr Asp Tyr Leu Met Tyr Ala Asp Ile Asp Met Asp
   225                 230                 235                 240
   His Pro Glu Val Val Asn Glu Leu Arg Asn Trp Gly Val Trp Tyr Thr
                   245                 250                 255
   Asn Thr Leu Gly Leu Asp Gly Phe Arg Ile Gly Ala Val Lys His Ile
               260                 265                 270
   Lys Tyr Ser Phe Thr Arg Asp Trp Leu Thr His Val Arg Asn Thr Thr
           275                 280                 285
   Gly Lys Asn Met Phe Ala Val Ala Glu Phe Trp Lys Asn Asp Ile Gly
       290                 295                 300
   Ala Ile Glu Asn Tyr Leu Ser Lys Thr Asn Trp Asn His Ser Val Phe
   305                 310                 315                 320
   Asp Val Pro Leu His Tyr Asn Leu Tyr Asn Ala Ser Arg Ser Gly Gly
                   325                 330                 335
   Asn Tyr Asp Met Arg Gln Ile Phe Asn Gly Thr Val Val Gln Arg His
               340                 345                 350
   Pro Thr His Ala Val Thr Phe Val Asp Asn His Asp Ser Gln Pro Glu
           355                 360                 365
   Glu Ala Leu Glu Ser Phe Val Glu Glu Trp Phe Lys Pro Leu Ala Cys
       370                 375                 380
   Ala Leu Thr Leu Thr Arg Asp Gln Gly Tyr Pro Ser Val Phe Tyr Gly
   385                 390                 395                 400
   Asp Tyr Tyr Gly Ile Pro Thr His Gly Val Pro Ala Met Lys Ser Lys
                   405                 410                 415
   Ile Asp Pro Ile Leu Glu Ala Arg Gln Lys Tyr Ala Tyr Gly Lys Gln
               420                 425                 430
   Asn Asp Tyr Leu Asp His His Asn Met Ile Gly Trp Thr Arg Glu Gly
           435                 440                 445
   Asn Thr Ala His Pro Asn Ser Gly Leu Ala Thr Ile Met Ser Asp Gly
       450                 455                 460
   Pro Gly Gly Asn Lys Trp Met Tyr Val Gly Arg Asn Lys Ala Gly Gln
   465                 470                 475                 480
   Val Trp Arg Asp Ile Thr Gly Asn Arg Ser Gly Thr Val Thr Ile Asn
                   485                 490                 495
   Ala Asp Gly Trp Gly Asn Phe Ser Val Asn Gly Gly Ser Val Ser Ile
               500                 505                 510
   Trp Val Asn Asn
           515